# EUROPEAN PATENT APPLICATION

(11) **EP 3 095 455 A1**
(43) Date of publication of application: **23.11.2016**
(21) Application number: 16164548.6
(22) Date of filing: 18.12.2007
(51) Int. Cl.: A61K 38/17, A61K 38/19, A61K 38/43, A61K 39/395, A61P 35/00

(54) **VEGF-SPECIFIC ANTAGONISTS FOR ADJUVANT AND NEOADJUVANT THERAPY AND THE TREATMENT OF EARLY STAGE TUMORS**

(30) Priority: 19.12.2006 US 870741 P; 19.12.2006 US 870745 P; 27.12.2006 US 877267 P; 22.03.2007 US 919638 P; 05.07.2007 US 958384 P; 20.11.2007 US 989397 P
(62) Divisional of application: 07869456.9
(71) Applicant: GENENTECH, INC., South San Francisco, CA 94080-4990 (US)
(72) Inventor: FERRARA, Napoleone, San Francisco, CA 94109 (US); KORSISAARI, Nina, Jamaica Plain, MA 02130 (US); MASS, Robert D., Mill Valley, CA 94941 (US)
(74) Representative: Denison, Christopher Marcus

(57) **Abstract**

Disclosed herein are methods of treating benign, pre-cancerous, or non-metastatic tumors using an anti-VEGF-specific antagonist. Also disclosed are methods of treating a subject at risk of developing benign, pre-cancerous, or non-metastatic tumors using an anti-VEGF-specific antagonist. Also disclosed are methods of treating or preventing recurrence of a tumor using an anti-VEGF-specific antagonist as well as use of VEGF-specific antagonists in neoadjuvant and adjuvant cancer therapy.

## Description

### Background

Cancer is one of the most deadly threats to human health. In the U.S. alone, cancer affects nearly 1.3 million new patients each year, and is the second leading cause of death after cardiovascular disease, accounting for approximately 1 in 4 deaths. Solid tumors are responsible for most of those deaths. Although there have been significant advances in the medical treatment of certain cancers, the overall 5-year survival rate for all cancers has improved only by about 10% in the past 20 years. Cancers, or malignant tumors, metastasize and grow rapidly in an uncontrolled manner, making timely detection and treatment extremely difficult.

Current methods of cancer treatment are relatively non-selective and generally target the tumor after the cancer has progressed to a more malignant state. Surgery removes the diseased tissue; radiotherapy shrinks solid tumors; and chemotherapy kills rapidly dividing cells. Chemotherapy, in particular, results in numerous side effects, in some cases so severe as to limit the dosage that can be given and thus preclude the use of potentially effective drugs. Moreover, cancers often develop resistance to chemotherapeutic drugs. The treatment of early stage or benign tumors would be desirable for preventing progression to a malignant or metastatic state, thereby reducing the morbidity and mortality associated with cancer.

For most patients newly diagnosed with operable cancer, the standard treatment is definitive surgery followed by chemotherapy. Such treatment aims at removing as much primary and metastatic disease as possible in order to prevent recurrence and improve survival. Indeed, most of these patients have no macroscopic evidence of residual tumor after surgery. However, many of them would later develop recurrence and may eventually die of their diseases. This occurs because a small number of viable tumor cells became metastasized prior to the surgery, escaped the surgery and went undetected after the surgery due to the limitation of current detection techniques.

Therefore, postoperative adjuvant treatments become important as auxiliary weapons to surgery in order to eliminate these residual micrometastatic cancer cells before they become repopulated and refractory. Over the past several decades, advances in adjuvant therapy have generally been incremental, centering on use of various chemotherapeutic agents. Many chemotherapy regimens have shown clinical benefits in adjuvantly treating patients with early stage major cancer indications such as lung, breast and colorectal cancers. Strauss et al. J Clin Oncol 22:7019 (2004); International Adjuvant Lung Cancer Trial Collaboration Group N Engl J Med 350:351-60 (2004). Moertel et al. Ann Intern Med 122:321-6 (1995); IMPACT Lancet 345:939-44 (1995); Citron et al. J Clin Oncol 21:1431-9 (2003).

Despite established benefits of chemo-based adjuvant therapy, one major limitation associated with chemotherapy of any kind is the significant toxicities. Generally, chemotherapeutic drugs are not targeted to the tumor site, and are unable to discriminate between normal and tumor cells. The issue of toxicities is especially challenging in adjuvant setting because of the lengthy treatment and its lasting impact on patients' quality of life. Moreover, benefits of adjuvant chemotherapy in patients with lower risk of recurrence remain unclear, making it questionable whether it is worthwhile for them to suffer the side effects of chemotherapy.

Neoadjuvant therapy, an adjunctive therapy given before the main definitive surgery, has emerged as another important part of cancer therapy. There are several advantages to give neoadjuvant treatment prior to a definitive surgery. First, it may help to improve patient's performance status prior to surgery, due to the reduction of tumor volume, ascites and pleural effusion. Second, the reduction of tumor volume may allow a less extensive surgery hence preserving patient's organ and function thereof. This is particularly valuable for, e.g., breast cancer patients. Also, reduction of tumor volume may enable surgery of otherwise inoperable tumors. Lastly, neoadjuvant therapy may improve the chance of completely removing tumor by surgery, thereby improving survival. Over the past decade, there have been many clinical trials on neoadjuvant therapy using various chemotherapeutic agents and or radiation to treat patients with such cancers as breast cancer, head and neck cancer, rectal cancer, bladder cancer, non-small cell lung cancer, cervical cancer, esophageal and gastric cancer and prostate cancer. For a review, see Tanvetyanon et al., Southern Med. J. 98:338-344 (2005).

As explained above, one major limitation associated with chemotherapy of any kind is the significant toxicities. Many neoadjuvant chemotherapy regimens are cumbersome, requiring frequent treatments over a long period of time. Moreover, benefits, especially survival benefits, of neoadjuvant chemotherapy in patients with lower risk of recurrence remain unclear, making it questionable whether it is worthwhile for them to wait instead of immediate surgery.

Angiogenesis is an important cellular event in which vascular endothelial cells proliferate, prune, and reorganize to form new vessels from preexisting vascular network. There is compelling evidence that the development of a vascular supply is essential for normal and pathological proliferative processes. Delivery of oxygen and nutrients, as well as the removal of catabolic products, represent rate-limiting steps in the majority of growth processes occurring in multicellular organisms.

While induction of new blood vessels is considered to be the predominant mode of tumor angiogenesis, recent data have indicated that some tumors may grow by co-opting existing host blood vessels. The co-opted vasculature then regresses, leading to tumor regression that is eventually reversed by hypoxia-induced angiogenesis at the tumor margin.

One of the key positive regulators of both normal and abnormal angiogenesis is vascular endothelial growth factor (VEGF)-A. VEGF-A is part of a gene family including VEGF-B, VEGF-C, VEGF-D, VEGF-E, VEGF-F, and P1GF. VEGF-A primarily binds to two high affinity receptor tyrosine kinases, VEGFR-1 (Flt-1) and VEGFR-2 (Flk-1/KDR), the latter being the major transmitter of vascular endothelial cell mitogenic signals of VEGF-A. Additionally, neuropilin-1 has been identified as a receptor for heparin-binding VEGF-A isoforms, and may play a role in vascular development.

In addition to being an angiogenic factor, VEGF, as a pleiotropic growth factor, exhibits multiple biological effects in other physiological processes, such as endothelial cell survival and proliferation, vessel permeability and vasodilation, monocyte chemotaxis, and calcium influx. Moreover, other studies have reported mitogenic effects of VEGF on a few non-endothelial cell types, such as retinal pigment epithelial cells, pancreatic duct cells, and Schwann cells.

The recognition of VEGF as a primary regulator of angiogenesis in pathological conditions has led to numerous attempts to block VEGF activities in conditions that involve pathological angiogenesis.

VEGF expression is upregulated in a majority of malignancies and the overexpression of VEGF correlates with a more advanced stage or with a poorer prognosis in many solid tumors. Therefore, molecules that inhibit VEGF signaling pathways have been used for the treatment of relatively advanced solid tumors in which pathological angiogenesis is noted.

Despite the evidence implicating the role of VEGF in the development of conditions or diseases that involve pathological angiogenesis, including late stage and metastatic or invasive tumors, less is known about the role of VEGF in early stage or benign cancers, the recurrence of tumors after dormancy, or in the development of secondary site tumors from dormant tumors, malignant tumors, or micrometastases. The invention addresses these and other needs, as will be apparent upon review of the following disclosure.

### Summary of the Invention

The use of VEGF-specific antagonists in combination with chemotherapy has been shown to be beneficial in patients with metastatic colorectal and non-small cell lung cancer, among others, but little is known about the impact of VEGF-specific antagonist therapy on benign or early stage tumors; the recurrence of tumors after dormancy, surgical, or other intervention; the development of secondary site tumors from dormant tumors, malignant tumors, or micrometastases; or in the adjuvant or neoadjuvant setting. We provide herein results that demonstrate that VEGF-specific antagonists can be used for the treatment of early stage tumors including benign, pre-cancerous, non-metastatic, and operable tumors. The results further demonstrate that VEGF-specific antagonists can be used for neoadjuvant therapy of cancer (e.g., benign or malignant cancers) or for preventing and/or reducing the likelihood of cancer recurrence (e.g., benign or malignant cancers), including methods of adjuvant therapy. The invention constitutes a significant medical breakthrough providing for the more effective, less toxic, care of patients with cancer, including benign, early stage, and operable cancers (both prior to and after surgery).

Accordingly, the invention features methods of treating a benign, pre-cancerous or non-metastatic cancer in a subject, which comprise administering to the subject an effective amount of a VEGF-specific antagonist. In certain embodiments, the administration of the VEGF-specific antagonist prevents the benign, pre-cancerous, or non-metastatic cancer from becoming an invasive or metastatic cancer. For example, the benign, pre-cancerous or non-metastatic cancer can be a stage 0, stage I, or stage II cancer, and in certain embodiments, the administration of the VEGF-specific antagonist prevents the benign, pre-cancerous or non-metastatic cancer from progressing to the next stage(s), e.g., a stage I, a stage II, a stage III or stage IV cancer. In certain embodiments, the VEGF-specific antagonist is administered for a time and in an amount sufficient to treat the benign, pre-cancerous, or non-metastatic tumor in the subject or to prevent the benign, pre-cancerous, or non-metastatic tumor from becoming an invasive or metastatic cancer. In certain embodiments, administering the VEGF-specific antagonist reduces tumor size, tumor burden, or the tumor number of the benign, pre-cancerous, or non-metastatic tumor. The VEGF-specific antagonist can also be administered in an amount and for a time to decrease the vascular density in the benign, pre-cancerous, or non-metastatic tumor.

As described herein, the methods of the invention can be used to treat, e.g., a stage 0 (e.g., a carcinoma in situ), stage I, or stage II cancer. The methods of neoadjuvant and adjuvant therapy can be used to treat any type of cancer, e.g., benign or malignant. In certain embodiments of the invention, the cancer is an epithelial cell solid tumor, including, but not limited to, gastrointestinal cancer, colon cancer, breast cancer, prostate cancer, renal cancer, lung cancer (e.g., non-small cell lung cancer), melanoma, ovarian cancer, pancreatic cancer, head and neck cancer, liver cancer and soft tissue cancers (e.g., B cell lymphomas such as NHL and multiple myeloma and leukemias such as chronic lymphocytic leukemia). In another embodiment, the benign, pre-cancerous, or non-metastatic tumor is a polyp, adenoma, fibroma, lipoma, gastrinoma, insulinoma, chondroma, osteoma, hemangioma, lymphangioma, meningioma, leiomyoma, rhabdomyoma, squamous cell papilloma, acoustic neuromas, neurofibroma, bile duct cystanoma, leiomyomas, mesotheliomas, teratomas, myxomas, trachomas, granulomas, hamartoma, transitional cell papilloma, pleiomorphic adenoma of the salivary gland, desmoid tumor, dermoid cystpapilloma, cystadenoma, focal nodular hyperplasia, or a nodular regenerative hyperplasia. In another embodiment, the method is desirably used to treat an adenoma. Non-limiting examples of adenomas include liver cell adenoma, renal adenoma, metanephric adenoma, bronchial adenoma, alveolar adenoma, adrenal adenoma, pituitary adenoma, parathyroid adenoma, pancreatic adenoma, salivary gland adenoma, hepatocellular adenoma, gastrointestinal adenoma, tubular adenoma, and bile duct adenoma.

The invention also features methods that comprise administering to a subject an effective amount of a VEGF-specific antagonist to prevent occurrence or recurrence of a benign, pre-cancerous, or non-metastatic cancer in the subject. In certain embodiments of the invention, the subject is at risk for cancer, polyps, or a cancer syndrome. In one example, the subject has a family history of cancer, polyps, or an inherited cancer syndrome (e.g., multiple endocrine neoplasia type 1 (MEN1)). In certain aspects of the invention, the subject is at risk of developing a benign, pre-cancerous, or non-metastatic gastrointestinal tumor, a desmoid tumor, or an adenoma (e.g., a gastrointestinal adenoma, a pituitary adenoma, or a pancreatic adenoma). In certain embodiments, the method prevents occurrence or recurrence of said benign, pre-cancerous or non-metastatic cancer in a subject who has never had a tumor, a subject who has never had a clinically detectable cancer, or a subject who has only had a benign tumor.

In another aspect, the invention features a method of treating a stage 0, stage I, or stage II gastrointestinal tumor in a subject that includes administering to the subject a VEGF-specific antagonist for a time and in an amount sufficient to treat the stage 0, stage I, or stage II gastrointestinal tumor in the subject. The gastrointestinal tumor can be any stage 0, stage I, or stage II cancer of the gastrointestinal system including, anal cancer, colorectal cancer, rectal cancer, esophageal cancer, gallbladder cancer, gastric cancer, liver cancer, pancreatic cancer, and cancer of the small intestine. In one embodiment, the gastrointestinal tumor is a stage 0 (e.g., a high grade adenoma) or stage I tumor. In one embodiment, the subject has not previously undergone a resection to treat the gastrointestinal tumor.

In another aspect, the invention features a method of treating a subject at risk of developing a gastrointestinal tumor that includes administering to the subject a VEGF-specific antagonist for a time and in an amount sufficient to prevent the occurrence or reoccurrence of the gastrointestinal tumor in the subject. The gastrointestinal tumor can be any gastrointestinal tumor including but not limited to an adenoma, one or more polyps, or a stage 0, I, or II cancer.

In certain embodiments of the above methods, the subject is a human over the age of 50, has an inherited cancer syndrome, or has a family history of colon cancer or polyps. Non-limiting examples of inherited gastrointestinal cancer syndromes include familial adenomatous polyposis (FAP), Gardner's syndrome, pancreatic cancer, and hereditary non-polyposis colorectal cancer (HNPCC). In certain embodiments, the subject may or may not have previously undergone a colonoscopy. In one embodiment, the VEGF-specific antagonist is administered in an amount and for a time to reduce the number of adenomatous colorectal polyps in a subject having FAP.

In another aspect, the invention features a method of preventing or reducing the likelihood of recurrence of a cancer in a subject that includes administering to the subject a VEGF-specific antagonist for a time and in an amount sufficient to prevent or reduce the likelihood of cancer recurrence in the subject. The invention includes a method of preventing the recurrence of a cancer in a subject having a tumor that includes the steps of removing the tumor (e.g., using definitive surgery) and thereafter administering to the subject a VEGF-specific antagonist. The invention includes methods of preventing the regrowth of a tumor in a subject that includes the steps of removing the tumor (e.g., using definitive surgery) and thereafter administering to the subject a VEGF-specific antagonist. In a related aspect, the invention includes a method of preventing recurrence of cancer in a subject or reducing the likelihood of cancer recurrence in a subject that optionally includes administering to the subject an effective amount of a VEGF-specific antagonist prior to surgery, performing definitive surgery, and administering an effective amount of a VEGF-specific antagonist following the surgery wherein the adminstration of the VEGF-specific antagonist after the surgery prevents recurrence of the cancer or reduces the likelihood of cancer recurrence. In another related aspect, the invention includes a method of preventing recurrence of cancer in a subject or reducing the likelihood of cancer recurrence in a subject that includes administering to the subject an effective amount of a VEGF-specific antagonist in the absence of any additional anti-cancer therapeutic agent, wherein the administering prevents recurrence of cancer in a subject or reduces the likelihood of cancer recurrence in a subject.

For each of the above aspects, the tumor can be any type of tumor including but not limited to the solid tumors, and particularly the tumors and adenomas, described herein. The subject can have a dormant tumor or micrometastases, which may or may not be clinically detectable. In one embodiment of this aspect, the VEGF-specific antagonist is administered for a time and in an amount sufficient to reduce neovascularization of a dormant tumor or micrometastases. In another embodiment, the VEGF-specific antagonist is administered for a time and in an amount sufficient to prevent occurrence of a clinically detectable tumor, or metastasis thereof, or to increase the duration of survival of the subject.

In one embodiment, the VEGF-specific antagonist is a monotherapy. In another embodiment, the subject has been previously treated for the tumor, for example, using an anti-cancer therapy. In one example, the anti-cancer therapy is surgery. In another embodiment, the subject can be further treated with an additional anti-cancer therapy before, during (e.g., simultaneously), or after administration of the VEGF-specific antagonist. Examples of anti-cancer therapies include, without limitation, surgery, radiation therapy (radiotherapy), biotherapy, immunotherapy, chemotherapy, or a combination of these therapies.

In embodiments where the subject has undergone definitive surgery, the VEGF-specific antagonist is generally administered after a period of time in which the subject has recovered from the surgery. This period of time can include the period required for wound healing or healing of the surgical incision, the time period required to reduce the risk of wound dehiscence, or the time period required for the subject to return to a level of health essentially similar to or better than the level of health prior to the surgery. The period between the completion of the definitive surgery and the first administration of the VEGF-specific antagonist can also include the period needed for a drug holiday, wherein the subject requires or requests a period of time between therapeutic regimes. Generally, the time period between completion of definitive surgery and the commencement of the VEGF-specific antagonist therapy can include less than one week, 1 week, 2 weeks, 3 weeks, 4 weeks (28 days), 5 weeks, 6 weeks, 7 weeks, 8 weeks, 3 months, 4 months, 5 months, 6 months, 7 months, 8 months, 9 months, 10 months, 11 months, 1 year, 2 years, 3 years, or more. In one embodiment, the period of time between definitive surgery and administering the VEGF-specific antagonist is greater than 2 weeks and less than 1 year.

Each of the above aspects can further include monitoring the subject for recurrence of the cancer.

The invention also provides methods of neoadjuvant therapy prior to the surgical removal of operable cancer in a subject, e.g., a human patient, comprising administering to the patient an effective amount of a VEGF-specific antagonist, e.g., bevacizumab, where the patient has been diagnosed with a tumor or cancer. The VEGF-specific antagonist can be administered alone or in combination with at least one chemotherapeutic agent.

The invention also includes a method of treating a subject with operable cancer that includes administering to the subject an effective amount of a VEGF-specific antagonist prior to surgery and thereafter performing surgery whereby the cancer is resected. In one embodiment, the method further includes the step of administering to the subject an effective amount of a VEGF-specific antagonist after surgery to prevent recurrence of the cancer.

In another aspect, the invention concerns a method of neoadjuvant therapy comprising administering to a subject with operable cancer an effective amount of a VEGF-specific antagonist, e.g., bevacizumab, and at least one chemotherapeutic agent prior to definitive surgery. The method can be used to extend disease free survival (DFS) or overall survival (OS) in the subject. In one embodiment, the DFS or the OS is evaluated about 2 to 5 years after initiation of treatment.

In another aspect, the invention includes a method of reducing tumor size in a subject having an unresectable tumor comprising administering to the subject an effective amount of a VEGF-specific antagonist wherein the administering reduces the tumor size thereby allowing complete resection of the tumor. In one embodiment, the method further includes administering to the subject an effective amount of a VEGF-specific antagonist after complete resection of the tumor.

In another aspect, the invention concerns a method of treating cancer in a subject comprising the following steps: a) a first stage comprising a plurality of treatment cycles wherein each cycle comprises administering to the subject an effective amount of a VEGF-specific antagonist, e.g., bevacizumab, and at least one chemotherapeutic agent at a predetermined interval; b) a definitive surgery whereby the cancer is removed; and c) a second stage comprising a plurality of maintenance cycles wherein each cycle comprises administering to the subject an effective amount of a VEGF-specific antagonist, e.g., bevacizumab, without any chemotherapeutic agent at a predetermined interval. In one embodiment, the first stage comprises a first plurality of treatment cycles wherein a VEGF-specific antagonist, e.g., bevacizumab, and a first chemotherapy regimen are administered followed by a second plurality of treatment cycles wherein a VEGF-specific antagonist, e.g., bevacizumab, and a second chemotherapy regimen are administered. In one embodiment, if the cancer to be treated is breast cancer, the first chemotherapy regimen comprises doxorubicin and cyclophosphamide and the second chemotherapy regimen comprises paclitaxel.

The invention provides methods comprising administering to a subject with metastatic or nonmetastatic cancer, following definitive surgery, an effective amount of a VEGF-specific antagonist, e.g., bevacizumab. In one embodiment the method further includes the use of at least one chemotherapeutic agent. The method can be used to extend DFS or OS in the subject. In one embodiment, the DFS or the OS is evaluated about 2 to 5 years after initiation of treatment. In one embodiment, the subject is disease free for at least 1 to 5 years after treatment.

In one aspect, the method comprises the following steps: a) a first stage comprising a plurality of treatment cycles wherein each cycle comprises administering to the subject an effective amount of a VEGF-specific antagonist, e.g., bevacizumab, and at least one chemotherapeutic agent at a predetermined interval; and b) a second stage comprising a plurality of maintenance cycles wherein each cycle comprises administering to the subject an effective amount of a VEGF-specific antagonist, e.g., bevacizumab, without any chemotherapeutic agent at a predetermined interval; wherein the combined first and second stages last for at least one year after the initial postoperative treatment. In one embodiment, the first stage comprises a first plurality of treatment cycles wherein a VEGF-specific antagonist, e.g., bevacizumab, and a first chemotherapy regimen are administered, followed by a second plurality of treatment cycles wherein a VEGF-specific antagonist, e.g., bevacizumab, and a second chemotherapy regimen are administered. If the cancer to be treated is breast cancer, for example, the first chemotherapy regimen comprises doxorubicin and cyclophosphamide and the second chemotherapy regimen comprises paclitaxel.

In certain embodiments of each of the above aspects, the VEGF-specific antagonist is a compound that binds to VEGF or reduces VEGF expression or biological activity. The VEGF-specific antagonist can be any one of the following exemplary compounds: a polypeptide that specifically binds to VEGF, a VEGF-specific ribozyme, a VEGF-specific peptibody, an antisense nucleobase oligomer complementary to at least a portion of a nucleic acid molecule encoding a VEGF polypeptide, a small RNA molecule complementary to at least a portion of a nucleic acid molecule encoding a VEGF polypeptide, or an aptamer. The polypeptide that specifically binds to VEGF can be a soluble VEGF receptor protein, or VEGF binding fragment thereof, or a chimeric VEGF receptor protein such as Flt-1/Fc, KDR/Fc, or Flt/KDR/Fc. The polypeptide that specifically binds to VEGF can also be an anti-VEGF antibody or antigen-binding fragment thereof. The anti-VEGF antibody, or antigen-binding fragment thereof, can be a monoclonal antibody, a chimeric antibody, a fully human antibody, or a humanized antibody. Exemplary antibodies useful in the methods of the invention include bevacizumab (AVASTIN®), G6-31, B20-4.1, and fragments thereof. The antibody, or antigen-binding fragment thereof, can also be an antibody that lacks an Fc portion, an F(ab')₂, an Fab, or an Fv structure.

Depending on the type and severity of the disease, preferred dosages for the VEGF-specific antagonist, e.g., bevacizumab, are described herein and can range from about 1µg/kg to about 50 mg/kg, most preferably from about 5mg/kg to about 15mg/kg, including but not limited to 7.5 mg/kg or 10 mg/kg. The frequency of administration will vary depending on the type and severity of the disease. For repeated administrations over several days or longer, depending on the condition, the treatment is sustained until the cancer is treated or the desired therapeutic effect is achieved, as measured by the methods described herein or known in the art. In one example, the VEGF-specific antagonist (e.g., an antibody) of the invention is administered once every week, every two weeks, or every three weeks, at a dose range from about 5 mg/kg to about 15 mg/kg, including but not limited to 7.5 mg/kg or 10 mg/kg. However, other dosage regimens may be useful. The progress of the therapy of the invention is easily monitored by conventional techniques and assays.

In additional embodiments of each of the above aspects, the VEGF-specific antagonist is administered locally or systemically (e.g., orally or intravenously). In one embodiment, the treatment with a VEGF-specific antagonist is prolonged until the patient has been cancer free for a time period selected from the group consisting of, 1 year, 2 years, 3 years, 4 years 5 years, 6 years, 7 years, 8 years, 9 years, 10 years, 11 years, and 12 years.

Although the subject can be treated in a number of different ways prior to, during, or after the administration of the VEGF-specific antagonist, in one embodiment of each of the aspects of the invention, the subject is treated without surgery or chemotherapy. In other embodiments, treatment with the VEGF-specific antagonist is a monotherapy or a monotherapy for the duration of the VEGF-specific antagonist treatment period, as assessed by the clinician or described herein.

In other embodiments, treatment with the VEGF-specific antagonist is in combination with an additional anti-cancer therapy, including but not limited to, surgery, radiation therapy, chemotherapy, differentiating therapy, biotherapy, immune therapy, an angiogenesis inhibitor, and an anti-proliferative compound. Treatment with the VEGF-specific antagonist can also include any combination of the above types of therapeutic regimens. In addition, cytotoxic agents, anti-angiogenic and anti-proliferative agents can be used in combination with the VEGF-specific antagonist. In one embodiment, the anti-cancer therapy is chemotherapy. For example, the chemotherapeutic agent is selected from, e.g., alkylating agents, antimetabolites, folic acid analogs, pyrimidine analogs, purine analogs and related inhibitors, vinca alkaloids, epipodopyyllotoxins, antibiotics, L-Asparaginase, topoisomerase inhibitor, interferons, platinum cooridnation complexes, anthracenedione substituted urea, methyl hydrazine derivatives, adrenocortical suppressant, adrenocorticosteroides, progestins, estrogens, antiestrogen, androgens, antiandrogen, gonadotropin-releasing hormone analog, etc. In some aspects, the chemotherapeutic agent and the VEGF-specific antagonist are administered concurrently.

In the embodiments which include an additional anti-cancer therapy, the subject can be further treated with the additional anti-cancer therapy before, during (e.g., simultaneously), or after administration of the VEGF-specific antagonist. In one embodiment, the anti-cancer therapy is chemotherapy which includes the administration of irinotecan, fluorouracil, leucovorin, gemcitabine or a combination thereof. In one embodiment, the VEGF-specific antagonist, administered either alone or with an anti-cancer therapy, can be administered as maintenance therapy. In the one aspect, the anti-cancer therapy for the prostate cancer, ovarian cancer and breast cancer can be hormone therapy. In one exemplary embodiment, the VEGF-specific antagonist is administered in combination with an anti-cancer therapy that does not include an anti-Her2 antibody, or fragment or derivative thereof (e.g., the Herceptin® antibody).

The methods of the invention are particularly advantageous in treating and preventing early stage tumors, thereby preventing progression to the more advanced stages resulting in a reduction in the morbidity and mortality associated with advanced cancer. The method of the invention are also advantageous in preventing the recurrence of a tumor or the regrowth of a tumor, for example, a dormant tumor that persists after removal of the primary tumor, or in reducing or preventing the occurrence or proliferation of micrometastases.

For the methods of the invention, the cancer may be a solid tumor, e.g., such as, breast cancer, colorectal cancer, rectal cancer, lung cancer, renal cell cancer, a glioma (e.g., anaplastic astrocytoma, anaplastic oligoastrocytoma, anaplastic oligodendroglioma, glioblastoma multiforme), kidney cancer, prostate cancer, liver cancer, pancreatic cancer, soft-tissue sarcoma, carcinoid carcinoma, head and neck cancer, melanoma, and ovarian cancer. In one embodiment, the cancer is a gastrointestinal cancer.

In additional embodiments of each of the above aspects of the invention, the VEGF-specific antagonist is administered in an amount or for a time (e.g., for a particular therapeutic regimen over time) to reduce (e.g., by 20%, 30%, 40%, 50%, 60%, 70%, 80%, 90%, 100% or more) the number of cancer cells in the tumor or cancer, including but not limited to benign, pre-cancerous, or non-metastatic cancers; to reduce the size of the tumor, polyp, or adenoma; to reduce the tumor burden; to inhibit (i.e., to decrease to some extent and/or stop) cancer cell infiltration into peripheral organs; to reduce hormonal secretion; to reduce the number of polyps; to reduce vessel density in the tumor or cancer, including but not limited to benign, pre-cancerous, or non-metastatic cancers; to inhibit tumor metastasis; to reduce or inhibit tumor growth or tumor cell proliferation; to reduce or prevent the growth of a dormant tumor; to reduce or prevent the growth or proliferation of a micrometastases; to reduce or prevent the re-growth of a tumor after treatment or removal; to increase or extend the DFS or OS of a subject susceptible to or diagnosed with a benign, precancerous, or non-metastatic tumor; and/or to relieve to some extent one or more of the symptoms associated with the cancer. In one example, the survival is measured as DFS or OS in the subject, wherein the DFS or the OS is evaluated about 2 to 5 years after initiation of treatment. In some additional embodiments, the VEGF-specific antagonist is used to prevent the occurrence or reoccurrence of cancer in the subject. In one example, prevention of cancer recurrence is evaluated in a population of subjects after about four years to confirm no disease recurrence has occurred in at least about 80% of the population. In another example, the VEGF-specific antagonist used to reduce the likelihood of recurrence of a tumor or cancer in a subject. In one example, cancer recurrence is evaluated at about 3 years, wherein cancer recurrence is decreased by at least about 50% compared to subjects treated with chemotherapy alone.

The methods of the invention can also include monitoring the subject for recurrence of the cancer or tumor.

Other features and advantages of the invention will be apparent from the following Detailed Description, the drawings, and the claims.

### Brief Description of the Drawings

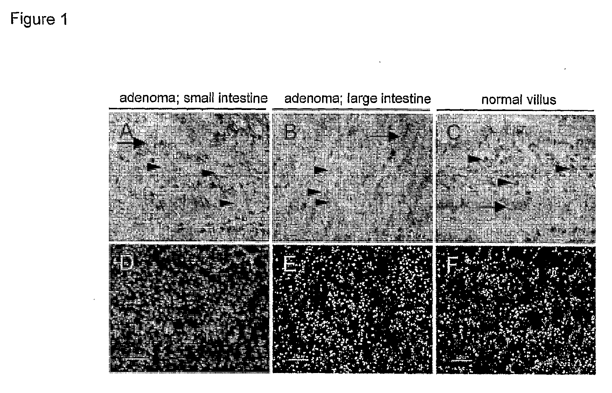

FIGURES 1A-1F are a series of photomicrographs showing VEGF-A expression in Apc^{min/+} adenomas and normal villus. *In situ* hybridization with VEGF-A probe on an intestinal adenoma from small (FIGURES 1A, 1D) and large (FIGURES 1B, 1E) bowel, as well as normal villi (FIGURES 1C, 1F) of a 14-week old Apc^{min/+} mouse demonstrates VEGF-A expression in the epithelial (arrows) and stromal cells (arrow heads). Brightfield; FIGURES 1A-1C, darkfield; FIGURES 1D-1F.

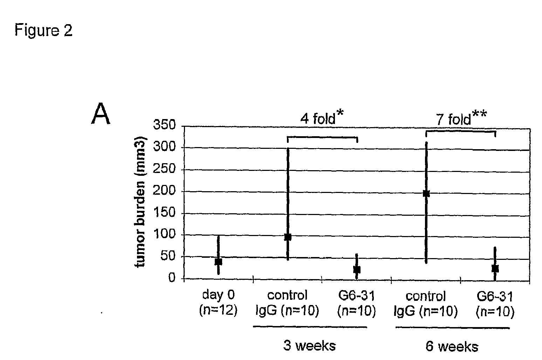

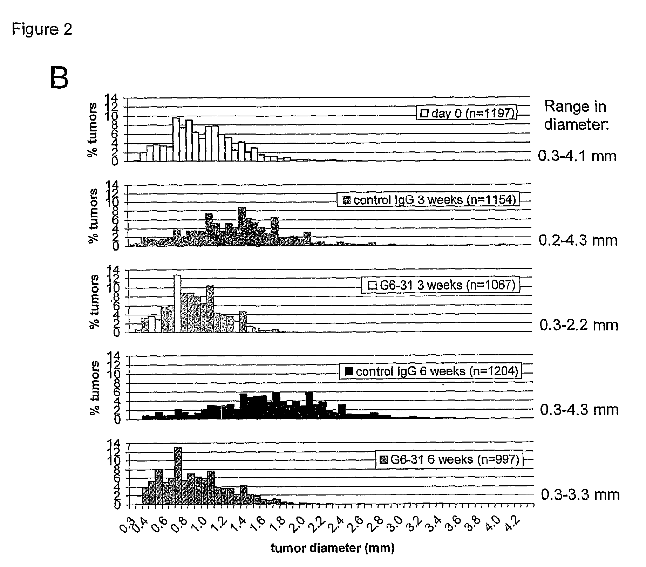

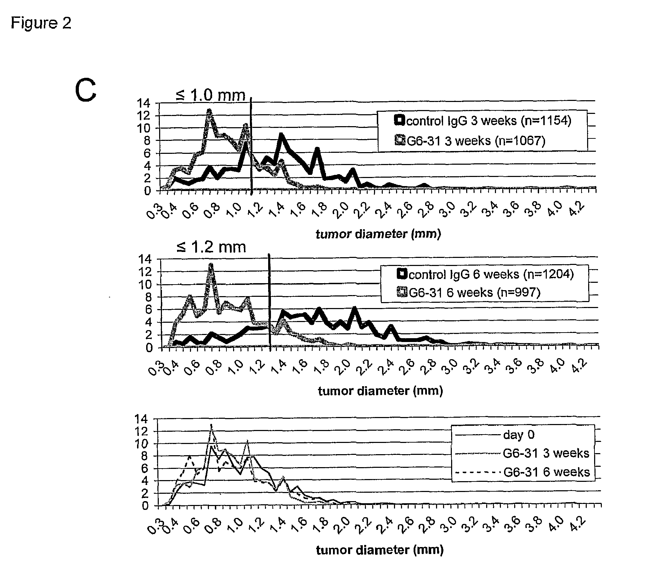

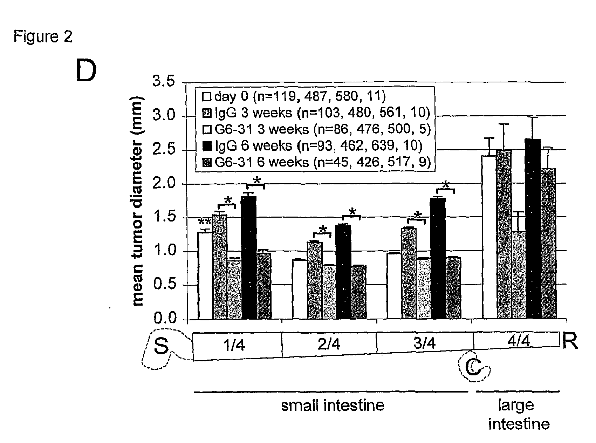

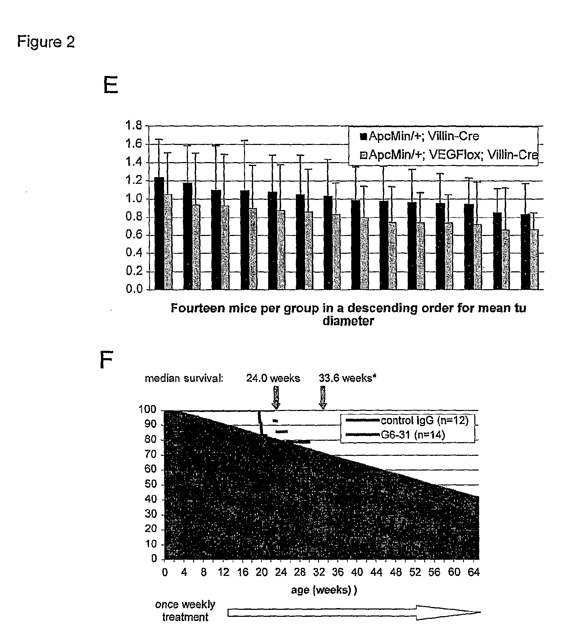

FIGURES 2A-2F are a series of graphs showing that inhibition of VEGF-A lowers tumor burden and extends survival. FIGURE 2A is a graph showing tumor burden of individual mice in the group. Tumor burden is indicated by bars from the largest to the smallest value of tumor burden. White crosses indicate group averages. *P<0.008, **p<5.3 x 10⁻⁵. N designates the number of animals. FIGURE 2B is a series of graphs showing the distribution of tumors by diameter and as percent of the total number of tumors. N designates the number of tumors in a group. FIGURE 2C is a series of graphs showing the overlay of tumor size frequencies after 3 weeks of treatment (top) and after 6 weeks of treatment (middle). The bottom graph shows an overlay of tumor size frequency in comparison to day 0 (bottom). Vertical bars illustrate the size smaller or equal of which tumor frequency is greater in mAb G6-31 treated animals; 1 mm in 3-week-treatment and 1.2 mm in 6-week-treatment group. FIGURE 2D is a graph showing the mean tumor diameter plotted against the intestinal location. N designates the number of tumors per group in the first, second, third, and fourth intestinal quarter, respectively. Day 0 group contained twelve animals, other groups ten. S; stomach, C; caecum, R; rectum. Bars represent SEM. *P<1.0 x 10⁻¹⁰, **p<0.002 compared to mAb G6-31 3 or 6 weeks. FIGURE 2E is a graph showing the mean tumor diameter of fourteen Apc^{min/+};Villin-Cre (black columns) and Apc^{min/+};VEGF^{lox};Villin-Cre (gray columns) mice presented in a descending order. Bars represent standard deviation (SD). FIGURE 2F is a graph showing the Kaplan-Meier of mAb G6-31 (gray line) - or control IgG (black line) -treated mice. Open arrow designates the duration of the treatments. Median survival is indicated with gray arrows. *P<2.4 x 10⁻³. N designates the number of mice in a group.

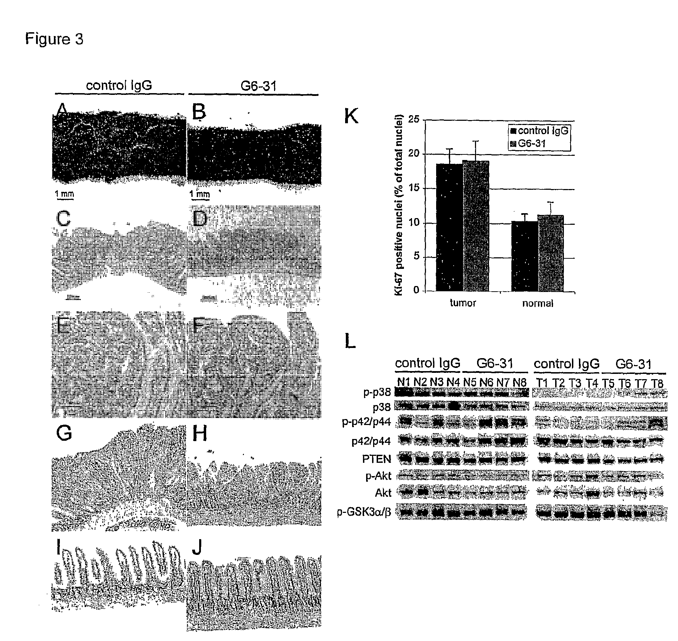

FIGURES 3A-3L show the effects of anti-VEGF-A treatment in altering the tumor morphology but not the proliferative index. FIGURES 3A-3B are photomicrographs of a jejunal segment of methylene blue-stained small intestine. FIGURES 3C-3D are photomicrographs showing low magnification images of an H&E stained section from jejunum. FIGURES 3E-3F are photomicrographs showing high magnification images of an H&E stained tumor section from jejunum. FIGURES 3G-3J are photomicrographs showing immunohistochemical staining with Ki-67 antibody of tumor tissue and normal mucosa. Counterstaining with H&E. FIGURE 3K is a graph showing the proliferative index expressed as percent of nuclei positive for Ki-67 relative to total number of nuclei. Bars represent SEM. FIGURE 3L is a western blot analysis of normal mucosal lysates from animals treated with control IgG (N1-N4) or mAb G6-31 (N5-N8). Tumor lysates from animals treated with control IgG (T1-T4) or mAb G6-31 (T5-T8).

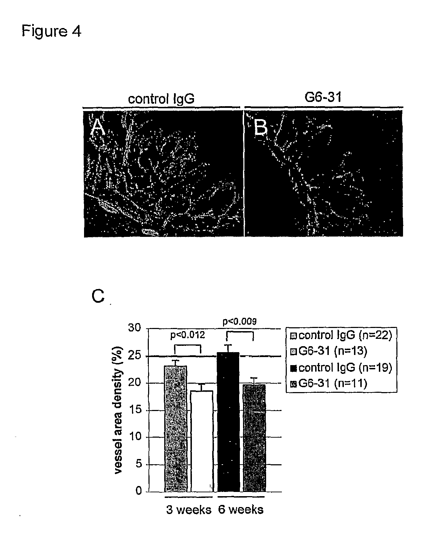

FIGURES 4A-4C show the reduced tumor vessel area density upon mAb G6-31 treatment. FIGURES 4A-4B are confocal images of 80 µm sections from immunohistochemical staining of tumors from jejunum. Green - CD31, vascular endothelial cells; blue - E-cadherin, epithelial cells; red - smooth muscle actin. FIGURE 4C is a graph showing the vascular density expressed as percent area positive for CD31 relative to total tumor area analyzed. Bars represent SEM; n designates the number of tumors analyzed.

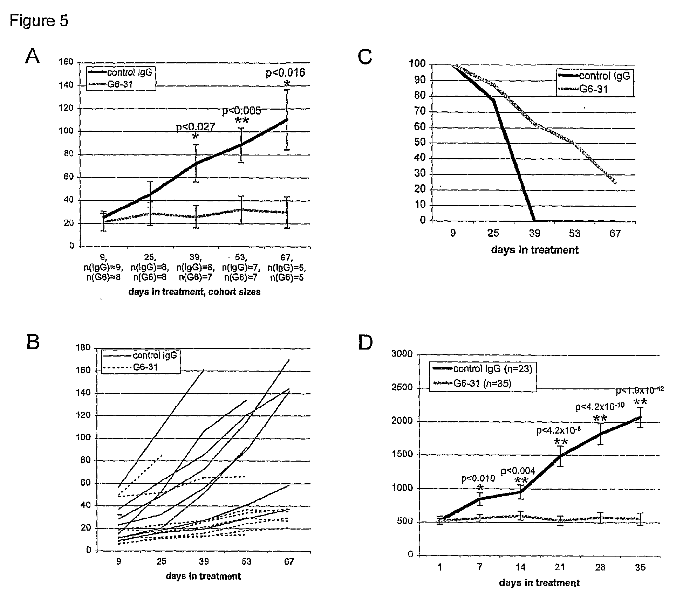

FIGURES 5A-5D are a series of graphs showing anti-VEGF-A treatment inhibits pituitary tumor growth. FIGURE 5A is a graph showing the mean tumor volume of control IgG (black line) and mAb G6-31 (gray line) treated groups at 9, 25, 39, 53, and 67 days of treatment. Bars represent SEM. N designates the number of mice in the group. FIGURE 5B is a graph showing the tumor volumes of individual mice treated with control IgG (solid lines) or mAb G6-31 (broken lines). Seven mice were euthanized before study's end-point due to ill health (lines ending before 67 days-time point). FIGURE 5C is a graph showing the tumor doubling free-survival of control IgG (black line) and mAb G6-31 (gray line) treated groups assessed at 9, 25, 39, 53, and 67 days after treatment onset. FIGURE 5D is a graph showing tumor volume measurements of control IgG (black line) and mAb G6-31 (gray line) treated subcutaneous pituitary tumor transplants at 1, 7, 14, 21, 28, and 35 days of treatment. Bars represent SEM. N designates the number of mice in the group.

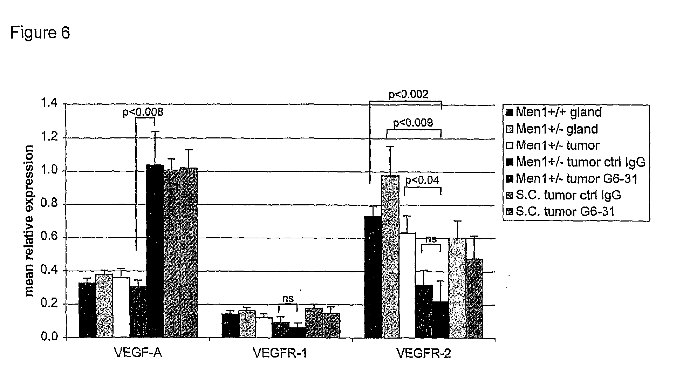

FIGURE 6 is a graph showing that pituitary gland and Men1^{+/-} pituitary adenomas express VEGF-A, VEGFR-1, and VEGFR-2. The relative expression of VEGF-A, VEGFR-1, and VEGFR-2 is shown for wild type pituitary gland (black column), non-tumorous pituitary gland tissue from Men1^{+/-} mice (gray), small non-treated pituitary adenomas, control IgG-treated (red), and mAb G6-31 treated (blue) pituitary tumors from Men1^{+/-} mice. Bars represent SEM. Ns; non-significant.

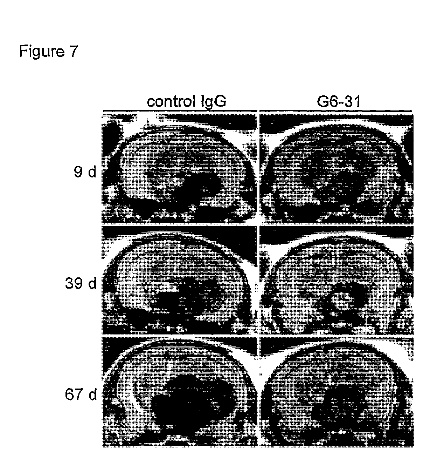

FIGURE 7 is a series of MRI images of representative pituitary tumors from Men1^{+/-} mice. Coronal sections with pituitary adenomas of a control IgG and a mAb G6-31 treated mouse at 9, 39, and 67 days of treatment are shown. For day nine, the edges of the pituitary adenomas have been highlighted with yellow asterisks. Volume of the control IgG treated tumor was 23.2, 55.9, and 142.0mm³, and that of the mAb G6-31 treated tumor was 18.9, 27.2, and 35.3mm³ at 9, 39, and 67 days after treatment onset, respectively.

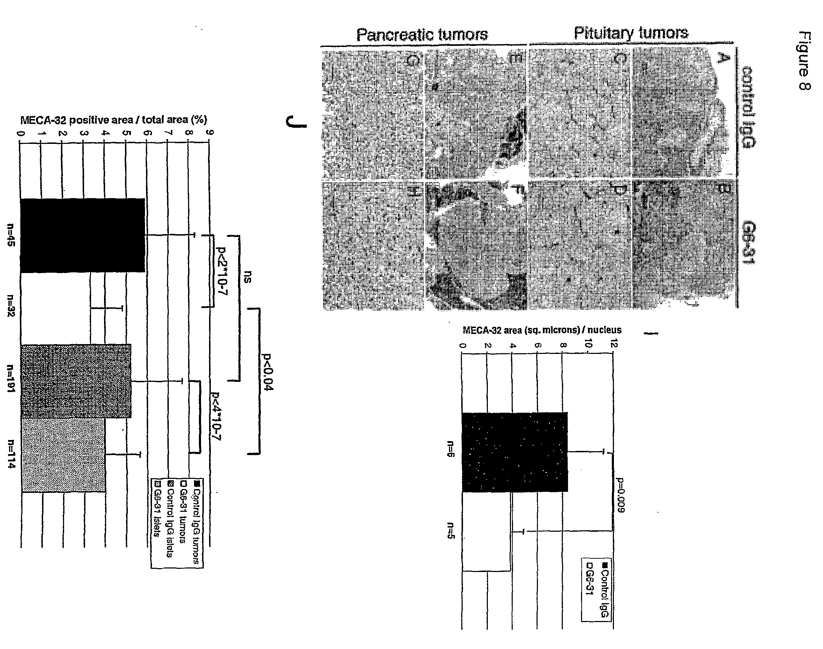

FIGURES 8A-8H are a series of images showing histologic examination of pituitary and pancreatic tumors of Men1^{+/-} mice. FIGURES 8A-8B show H&E stained pituitary tumors and FIGURES 8E-8F show H&E stained pancreatic tumors. FIGURES 8C-8D show immunohistochemistry staining of *in situ* pituitary tumors with panendothelial marker MECA-32 and FIGURES 8G-8H show immunohistochemistry staining of *in situ* pancreatic tumors with panendothelial marker MECA-32. FIGURE 8I is a graph showing the results of assaying vascular density in pituitary tumors and FIGURE 8J is a graph showing the results of assaying vascular density in pancreatic tumors in control IgG and anti-VEGF-treated animals. Bars represent SD. Ns=non-significant.

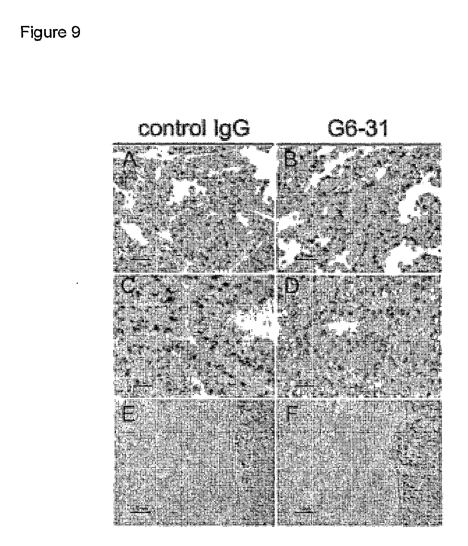

FIGURES 9A-9D are a series of images showing that pituitary tumors from Men1^{+/-} mice and pituitary tumor transplants stain positive for prolactin. Immunohistochemistry staining is shown for *in situ* pituitary tumors (FIGURES 9A-9B) and subcutaneous pituitary tumor transplants (FIGURES 9C-9D) with anti-prolactin antibody. FIGURE 9E and 9F are images showing that transplanted pituitary tumor adjacent to mammary gland show prolactin-induced secretory changes (left side of image).

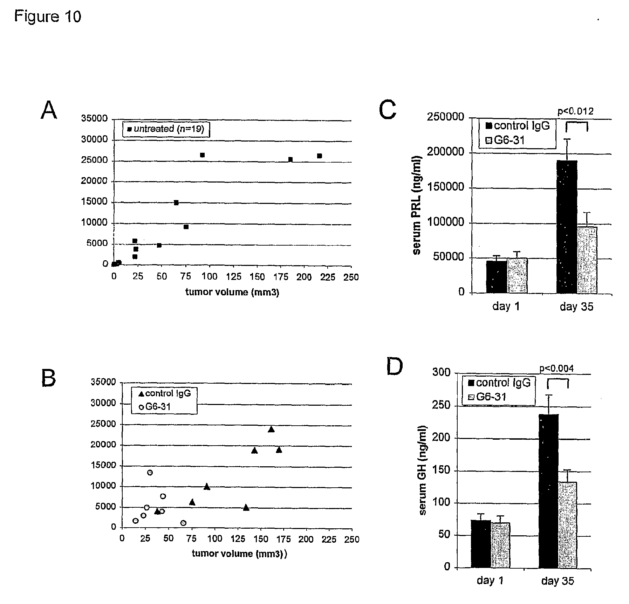

FIGURES 10A-10D show serum prolactin and growth hormone levels are elevated in mice with pituitary tumors and pituitary tumor transplants. FIGURE 10A is a graph showing serum PRL (ng/ml) level plotted against pituitary tumor volume (mm³) from 19 non-treated, tumor-bearing Men1^{+/-} mice, illustrating positive correlation. FIGURE 10B is a graph showing serum PRL level plotted against the pituitary tumor volume of control IgG (black triangles) and mAb G6-31 (gray spheres) treated Men1^{+/-} mice at study end-point. FIGURES 10C-10D are graphs showing serum prolactin (C) and growth hormone (D) levels from mice with pituitary adenoma transplants at day 1 and day 35 of treatment.

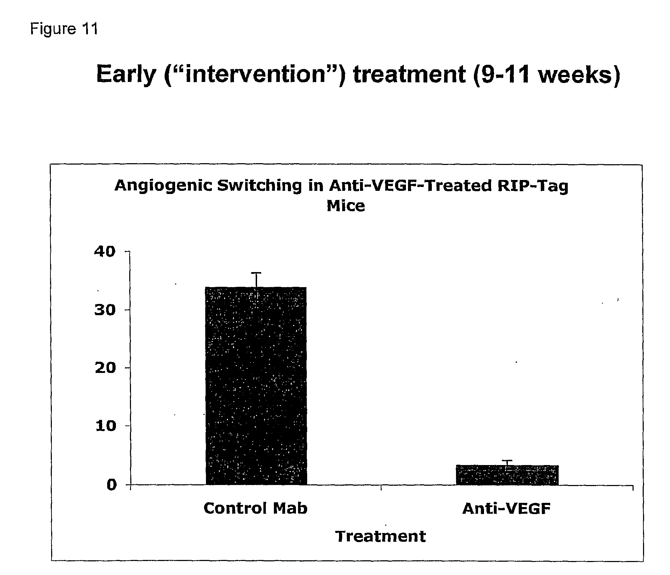

FIGURE 11 a graph showing the effects of anti-VEGF treatment ("intervention") during early stage tumor progression in the mouse Rip-TβAg model of pancreatic islet tumor development. The graph shows the decrease in tumor angiogenesis as measured by the mean number of angiogenic islets after treatment with an anti-VEGF antibody at 9 to 11 weeks as compared to treatment with an isotype matched control monoclonal antibody.

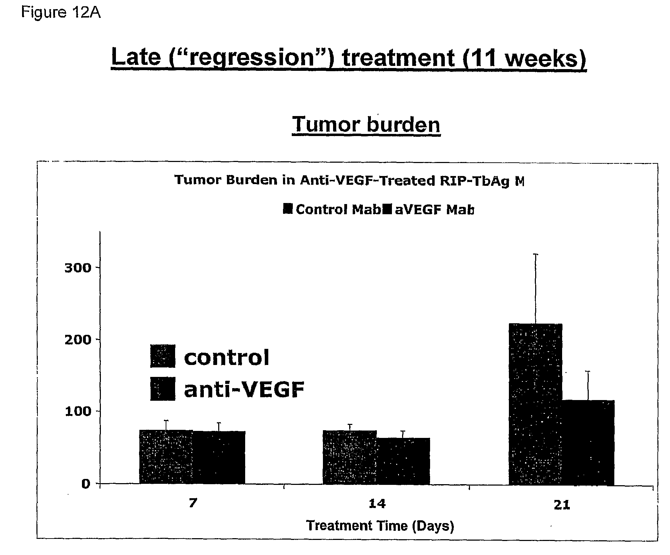

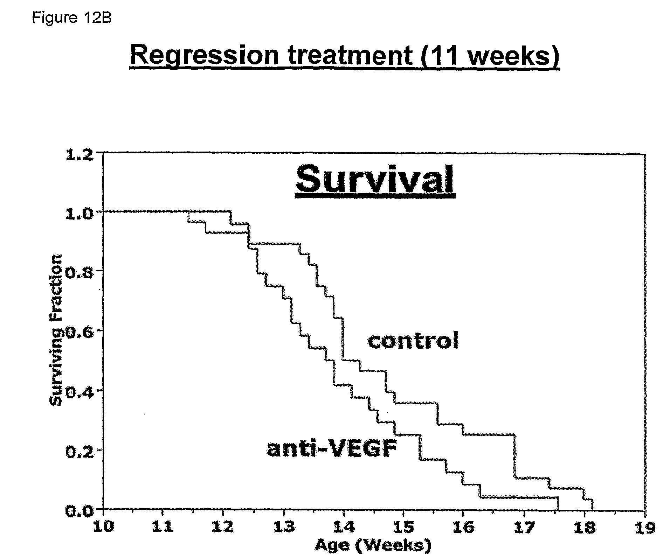

FIGURES 12A and 12B show the results of the regression trial where no significant differences in tumor burden or survival were detected between treatment with an anti-VEGF antibody and an isotype matched control monoclonal antibody in the mouse Rip-TβAg model of pancreatic islet tumor development. FIGURE 12A is a graph showing the tumor burden in mice treated with an anti-VEGF antibody as compared to those treated with an isotype matched control monoclonal antibody. FIGURE 12B is a graph showing the survival over time of mice treated with an anti-VEGF antibody as compared to those treated with an isotype matched control monoclonal antibody.

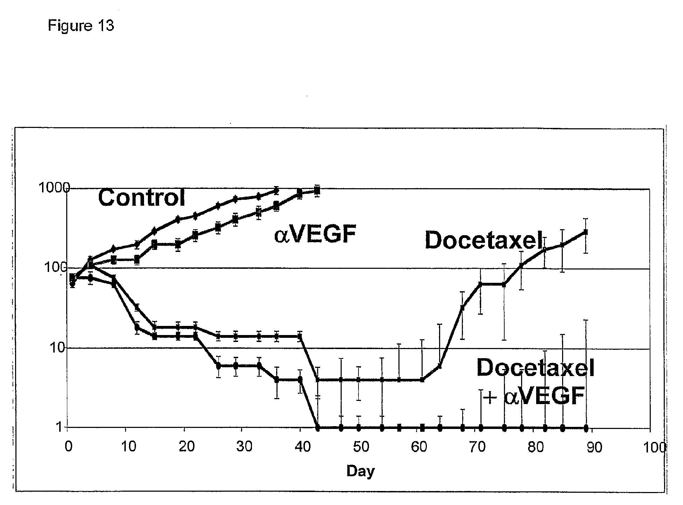

FIGURE 13 is a graph showing the effectiveness of prolonged anti-VEGF therapy to suppress the re-growth of tumors following cytoreduction with taxanes or gemcitabine.

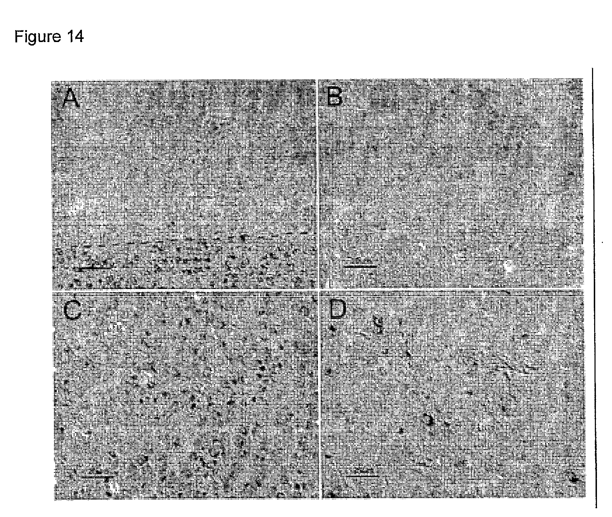

FIGURES 14A-14D are a series of images showing that primary non-treated pituitary adenoma (FIGURE 14A, above dotted line) is variably and weakly positive for growth hormone compared to adjacent normal anterior pituitary (below dotted line). One of four transplanted pituitary tumors (control IgG-treated) was weakly positive for growth hormone (FIGURE 14B). Primary pituitary tumors from mice treated with mab G6-31 (FIGURE 14C) or control IgG (FIGURE 14D) are focally positive for growth hormone.

### I. Definitions

The term "VEGF" or "VEGF-A" is used to refer to the 165-amino acid human vascular endothelial cell growth factor and related 121-, 145-, 189-, and 206- amino acid human vascular endothelial cell growth factors, as described by, e.g., Leung et al. Science, 246:1306 (1989), and Houck et al. Mol. Endocrin., 5:1806 (1991), together with the naturally occurring allelic and processed forms thereof. VEGF-A is part of a gene family including VEGF-B, VEGF-C, VEGF-D, VEGF-E, VEGF-F, and P1GF. VEGF-A primarily binds to two high affinity receptor tyrosine kinases, VEGFR-1 (Flt-1) and VEGFR-2 (Flk-1/KDR), the latter being the major transmitter of vascular endothelial cell mitogenic signals of VEGF-A. Additionally, neuropilin-1 has been identified as a receptor for heparin-binding VEGF-A isoforms, and may play a role in vascular development. The term "VEGF" or "VEGF-A" also refers to VEGFs from non-human species such as mouse, rat, or primate. Sometimes the VEGF from a specific species is indicated by terms such as hVEGF for human VEGF or mVEGF for murine VEGF. The term "VEGF" is also used to refer to truncated forms or fragments of the polypeptide comprising amino acids 8 to 109 or 1 to 109 of the 165-amino acid human vascular endothelial cell growth factor. Reference to any such forms of VEGF may be identified in the present application, e.g., by "VEGF (8-109)," "VEGF (1-109)" or "VEGF₁₆₅." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

The term "VEGF variant" as used herein refers to a VEGF polypeptide which includes one or more amino acid mutations in the native VEGF sequence. Optionally, the one or more amino acid mutations include amino acid substitution(s). For purposes of shorthand designation of VEGF variants described herein, it is noted that numbers refer to the amino acid residue position along the amino acid sequence of the putative native VEGF (provided in Leung et al., *supra* and Houck et al., *supra*.).

A "native sequence" polypeptide comprises a polypeptide having the same amino acid sequence as a polypeptide derived from nature. Thus, a native sequence polypeptide can have the amino acid sequence of naturally-occurring polypeptide from any mammal. Such native sequence polypeptide can be isolated from nature or can be produced by recombinant or synthetic means. The term "native sequence" polypeptide specifically encompasses naturally-occurring truncated or secreted forms of the polypeptide (e.g., an extracellular domain sequence), naturally-occurring variant forms (e.g., alternatively spliced forms) and naturally-occurring allelic variants of the polypeptide.

A polypeptide "variant" means a biologically active polypeptide having at least about 80% amino acid sequence identity with the native sequence polypeptide. Such variants include, for instance, polypeptides wherein one or more amino acid residues are added, or deleted, at the N- or C-terminus of the polypeptide. Ordinarily, a variant will have at least about 80% amino acid sequence identity, more preferably at least about 90% amino acid sequence identity, and even more preferably at least about 95% amino acid sequence identity with the native sequence polypeptide.

"VEGF biological activity" includes binding to any VEGF receptor or any VEGF signaling activity such as regulation of both normal and abnormal angiogenesis and vasculogenesis (Ferrara and Davis-Smyth (1997) Endocrine Rev. 18:4-25; Ferrara (1999) J. Mol. Med. 77:527-543); promoting embryonic vasculogenesis and angiogenesis (Carmeliet et al. (1996) Nature 380:435-439; Ferrara et al. (1996) Nature 380:439-442); and modulating the cyclical blood vessel proliferation in the female reproductive tract and for bone growth and cartilage formation (Ferrara et al. (1998) Nature Med. 4:336-340; Gerber et al. (1999) Nature Med. 5:623-628). In addition to being an angiogenic factor in angiogenesis and vasculogenesis, VEGF, as a pleiotropic growth factor, exhibits multiple biological effects in other physiological processes, such as endothelial cell survival, vessel permeability and vasodilation, monocyte chemotaxis and calcium influx (Ferrara and Davis-Smyth (1997), *supra* and Cebe-Suarez et al. Cell. Mol. Life Sci. 63:601-615 (2006)). Moreover, recent studies have reported mitogenic effects of VEGF on a few non-endothelial cell types, such as retinal pigment epithelial cells, pancreatic duct cells, and Schwann cells. Guerrin et al. (1995) J. Cell Physiol. 164:385-394; Oberg-Welsh et al. (1997) Mol. Cell. Endocrinol. 126:125-132; Sondell et al. (1999) J. Neurosci. 19:5731-5740.

A "VEGF-specific antagonist" is described herein under Section III.

An "anti-VEGF antibody" is an antibody that binds to VEGF with sufficient affinity and specificity. The antibody selected will normally have a sufficiently strong binding affinity for VEGF, for example, the antibody may bind hVEGF with a K_{d} value of between 100 nM-1 pM. Antibody affinities may be determined by a surface plasmon resonance based assay (such as the BIAcore assay as described in PCT Application Publication No. WO2005/012359); enzyme-linked immunoabsorbent assay (ELISA); and competition assays (e.g. RIA's), for example. In certain embodiments, the anti-VEGF antibody of the invention can be used as a therapeutic agent in targeting and interfering with diseases or conditions wherein the VEGF activity is involved. Also, the antibody may be subjected to other biological activity assays, e.g., in order to evaluate its effectiveness as a therapeutic. Such assays are known in the art and depend on the target antigen and intended use for the antibody. Examples include the HUVEC inhibition assay (as described in the Examples below); tumor cell growth inhibition assays (as described in WO 89/06692, for example); antibody-dependent cellular cytotoxicity (ADCC) and complement-mediated cytotoxicity (CDC) assays (US Patent 5,500,362); and agonistic activity or hematopoiesis assays (see WO 95/27062). An anti-VEGF antibody will usually not bind to other VEGF homologues such as VEGF-B or VEGF-C, nor other growth factors such as P1GF, PDGF or bFGF. Additional information regarding anti-VEGF antibodies can be found under section III, A.

Throughout the present specification and claims, the numbering of the residues in an immunoglobulin heavy chain is that of the EU index as in Kabat et al., Sequences of Proteins of Immunological Interest, 5th Ed. Public Health Service, National Institutes of Health, Bethesda, Md. (1991), expressly incorporated herein by reference. The "EU index as in Kabat" refers to the residue numbering of the human IgG1 EU antibody.

The term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

The "Kd" or "Kd value" according to this invention is in one embodiment measured by a radiolabeled VEGF binding assay (RIA) performed with the Fab version of the antibody and a VEGF molecule as described by the following assay that measures solution binding affinity of Fabs for VEGF by equilibrating Fab with a minimal concentration of (¹²⁵I)-labeled VEGF(109) in the presence of a titration series of unlabeled VEGF, then capturing bound VEGF with an anti-Fab antibody-coated plate (Chen, et al., (1999) J. Mol Biol 293:865-881). To establish conditions for the assay, microtiter plates (Dynex) are coated overnight with 5 ug/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbant plate (Nunc #269620), 100 pM or 26 pM [¹²⁵I]VEGF(109) are mixed with serial dilutions of a Fab of interest, e.g., Fab-12 (Presta et al., (1997) Cancer Res. 57:4593-4599). The Fab of interest is then incubated overnight; however, the incubation may continue for 65 hours to insure that equilibrium is reached. Thereafter, the mixtures are transferred to the capture plate for incubation at room temperature for one hour. The solution is then removed and the plate washed eight times with 0.1% Tween-20 in PBS. When the plates had dried, 150 ul/well of scintillant (MicroScint-20; Packard) is added, and the plates are counted on a Topcount gamma counter (Packard) for ten minutes. Concentrations of each Fab that give less than or equal to 20% of maximal binding are chosen for use in competitive binding assays. According to another embodiment the Kd or Kd value is measured by using surface plasmon resonance assays using a BIAcore™-2000 or a BIAcore™-3000 (BIAcore, Inc., Piscataway, NJ) at 25°C with immobilized hVEGF (8-109) CM5 chips at ∼10 response units (RU). Briefly, carboxymethylated dextran biosensor chips (CM5, BIAcore Inc.) are activated with *N*-ethyl-*N*'- (3-dimethylaminopropyl)-carbodiimide hydrochloride (EDC) and *N*-hydroxysuccinimide (NHS) according to the supplier's instructions. Human VEGF is diluted with 10mM sodium acetate, pH 4.8, into 5ug/ml (∼0.2uM) before injection at a flow rate of 5ul/minute to achieve approximately 10 response units (RU) of coupled protein. Following the injection of human VEGF, 1M ethanolamine is injected to block unreacted groups. For kinetics measurements, two-fold serial dilutions of Fab (0.78 nM to 500 nM) are injected in PBS with 0.05% Tween 20 (PBST) at 25°C at a flow rate of approximately 25ul/min. Association rates (kₒₙ) and dissociation rates (k_{off}) are calculated using a simple one-to-one Langmuir binding model (BIAcore Evaluation Software version 3.2) by simultaneous fitting the association and dissociation sensorgram. The equilibrium dissociation constant (Kd) was calculated as the ratio k_{off}/kₒₙ. See, e.g., Chen, Y., et al., (1999) J. Mol Biol 293:865-881. If the on-rate exceeds 10⁶ M⁻¹ S⁻¹ by the surface plasmon resonance assay above, then the on-rate is can be determined by using a fluorescent quenching technique that measures the increase or decrease in fluorescence emission intensity (excitation = 295 nm; emission = 340 nm, 16 nm band-pass) at 25°C of a 20nM anti-VEGF antibody (Fab form) in PBS, pH 7.2, in the presence of increasing concentrations of human VEGF short form (8-109) or mouse VEGF as measured in a spectrometer, such as a stop-flow equipped spectrophometer (Aviv Instruments) or a 8000-series SLM-Aminco spectrophotometer (ThermoSpectronic) with a stirred cuvette.

A "blocking" antibody or an antibody "antagonist" is one which inhibits or reduces biological activity of the antigen it binds. For example, a VEGF-specific antagonist antibody binds VEGF and inhibits the ability of VEGF to induce vascular endothelial cell proliferation. Preferred blocking antibodies or antagonist antibodies completely inhibit the biological activity of the antigen.

Unless indicated otherwise, the expression "multivalent antibody" is used throughout this specification to denote an antibody comprising three or more antigen binding sites. For example, the multivalent antibody is engineered to have the three or more antigen binding sites and is generally not a native sequence IgM or IgA antibody.

An "Fv" fragment is an antibody fragment which contains a complete antigen recognition and binding site. This region consists of a dimer of one heavy and one light chain variable domain in tight association, which can be covalent in nature, for example in scFv. It is in this configuration that the three CDRs of each variable domain interact to define an antigen binding site on the surface of the V_{H}-V_{L} dimer. Collectively, the six CDRs or a subset thereof confer antigen binding specificity to the antibody. However, even a single variable domain (or half of an Fv comprising only three CDRs specific for an antigen) has the ability to recognize and bind antigen, although usually at a lower affinity than the entire binding site.

As used herein, "antibody variable domain" refers to the portions of the light and heavy chains of antibody molecules that include amino acid sequences of Complementarity Determining Regions (CDRs; ie., CDR1, CDR2, and CDR3), and Framework Regions (FRs). V_{H} refers to the variable domain of the heavy chain. V_{L} refers to the variable domain of the light chain. According to the methods used in this invention, the amino acid positions assigned to CDRs and FRs may be defined according to Kabat (Sequences of Proteins of Immunological Interest (National Institutes of Health, Bethesda, Md., 1987 and 1991)). Amino acid numbering of antibodies or antigen binding fragments is also according to that of Kabat.

As used herein, the term "Complementarity Determining Regions" (CDRs; i.e., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (i. e. about residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*i.e.* about residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). In some instances, a complementarity determining region can include amino acids from both a CDR region defined according to Kabat and a hypervariable loop. For example, the CDRH1 of the heavy chain of antibody 4D5 includes amino acids 26 to 35.

"Framework regions" (hereinafter FR) are those variable domain residues other than the CDR residues. Each variable domain typically has four FRs identified as FR1, FR2, FR3 and FR4. If the CDRs are defined according to Kabat, the light chain FR residues are positioned at about residues 1-23 (LCFR1), 35-49 (LCFR2), 57-88 (LCFR3), and 98-107 (LCFR4) and the heavy chain FR residues are positioned about at residues 1-30 (HCFR1), 36-49 (HCFR2), 66-94 (HCFR3), and 103-113 (HCFR4) in the heavy chain residues. If the CDRs comprise amino acid residues from hypervariable loops, the light chain FR residues are positioned about at residues 1-25 (LCFR1), 33-49 (LCFR2), 53-90 (LCFR3), and 97-107 (LCFR4) in the light chain and the heavy chain FR residues are positioned about at residues 1-25 (HCFR1), 33-52 (HCFR2), 56-95 (HCFR3), and 102-113 (HCFR4) in the heavy chain residues. In some instances, when the CDR comprises amino acids from both a CDR as defined by Kabat and those of a hypervariable loop, the FR residues will be adjusted accordingly. For example, when CDRH1 includes amino acids H26-H35, the heavy chain FR1 residues are at positions 1-25 and the FR2 residues are at positions 36-49.

The "Fab" fragment contains a variable and constant domain of the light chain and a variable domain and the first constant domain (CH1) of the heavy chain. F(ab')₂ antibody fragments comprise a pair of Fab fragments which are generally covalently linked near their carboxy termini by hinge cysteines between them. Other chemical couplings of antibody fragments are also known in the art.

"Single-chain Fv" or "scFv" antibody fragments comprise the V_{H} and V_{L} domains of antibody, wherein these domains are present in a single polypeptide chain. Generally the Fv polypeptide further comprises a polypeptide linker between the V_{H} and V_{L} domains, which enables the scFv to form the desired structure for antigen binding. For a review of scFv, see Pluckthun in The Pharmacology of Monoclonal Antibodies, Vol 113, Rosenburg and Moore eds. Springer-Verlag, New York, pp. 269-315(1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H} and V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Pro. Natl. Acad. Sci. USA, 90:6444-6448 (1993).

The expression "linear antibodies" refers to the antibodies described in Zapata et al., Protein Eng., 8(10):1057-1062 (1995). Briefly, these antibodies comprise a pair of tandem Fd segments (V_{H}-C_{H}1-V_{H}-C_{H}1) which, together with complementary light chain polypeptides, form a pair of antigen binding regions. Linear antibodies can be bispecific or monospecific.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the invention may be made by the hybridoma method first described by Kohler et al., Nature 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. Proc. Natal. Acad. Sci. 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized in vitro). See, e.g., Cole et al., Monoclonal Antibodies and Cancer Therapy, Alan R. Liss, p. 77 (1985); Boerner et al., J. Immunol., 147 (1):86-95 (1991); and U.S. Pat. No. 5,750,373.

An "affinity matured" antibody is one with one or more alterations in one or more CDRs thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al., J. Mol. Biol. 226:889-896 (1992).

A "functional antigen binding site" of an antibody is one which is capable of binding a target antigen. The antigen binding affinity of the antigen binding site is not necessarily as strong as the parent antibody from which the antigen binding site is derived, but the ability to bind antigen must be measurable using any one of a variety of methods known for evaluating antibody binding to an antigen. Moreover, the antigen binding affinity of each of the antigen binding sites of a multivalent antibody herein need not be quantitatively the same. For the multimeric antibodies herein, the number of functional antigen binding sites can be evaluated using ultracentrifugation analysis as described in Example 2 of U.S. Patent Application Publication No. 20050186208. According to this method of analysis, different ratios of target antigen to multimeric antibody are combined and the average molecular weight of the complexes is calculated assuming differing numbers of functional binding sites. These theoretical values are compared to the actual experimental values obtained in order to evaluate the number of functional binding sites.

An antibody having a "biological characteristic" of a designated antibody is one which possesses one or more of the biological characteristics of that antibody which distinguish it from other antibodies that bind to the same antigen.

In order to screen for antibodies which bind to an epitope on an antigen bound by an antibody of interest, a routine cross-blocking assay such as that described in Antibodies, A Laboratory Manual, Cold Spring Harbor Laboratory, Ed Harlow and David Lane (1988), can be performed.

A "species-dependent antibody" is one which has a stronger binding affinity for an antigen from a first mammalian species than it has for a homologue of that antigen from a second mammalian species. Normally, the species-dependent antibody "binds specifically" to a human antigen (*i.e.* has a binding affinity (K_{d}) value of no more than about 1 x 10⁻⁷ M, preferably no more than about 1 x 10⁻⁸ M and most preferably no more than about 1 x 10⁻⁹ M) but has a binding affinity for a homologue of the antigen from a second nonhuman mammalian species which is at least about 50 fold, or at least about 500 fold, or at least about 1000 fold, weaker than its binding affinity for the human antigen. The species-dependent antibody can be any of the various types of antibodies as defined above, but typically is a humanized or human antibody.

As used herein, "antibody mutant" or "antibody variant" refers to an amino acid sequence variant of the species-dependent antibody wherein one or more of the amino acid residues of the species-dependent antibody have been modified. Such mutants necessarily have less than 100% sequence identity or similarity with the species-dependent antibody. In one embodiment, the antibody mutant will have an amino acid sequence having at least 75% amino acid sequence identity or similarity with the amino acid sequence of either the heavy or light chain variable domain of the species-dependent antibody, more preferably at least 80%, more preferably at least 85%, more preferably at least 90%, and most preferably at least 95%. Identity or similarity with respect to this sequence is defined herein as the percentage of amino acid residues in the candidate sequence that are identical (i.e same residue) or similar (i.e. amino acid residue from the same group based on common side-chain properties, see below) with the species-dependent antibody residues, after aligning the sequences and introducing gaps, if necessary, to achieve the maximum percent sequence identity. None of N-terminal, C-terminal, or internal extensions, deletions, or insertions into the antibody sequence outside of the variable domain shall be construed as affecting sequence identity or similarity.

To increase the half-life of the antibodies or polypeptide containing the amino acid sequences of this invention, one can attach a salvage receptor binding epitope to the antibody (especially an antibody fragment), as described, e.g., in US Patent 5,739,277. For example, a nucleic acid molecule encoding the salvage receptor binding epitope can be linked in frame to a nucleic acid encoding a polypeptide sequence of this invention so that the fusion protein expressed by the engineered nucleic acid molecule comprises the salvage receptor binding epitope and a polypeptide sequence of this invention. As used herein, the term "salvage receptor binding epitope" refers to an epitope of the Fc region of an IgG molecule (*e.g*., IgG₁, IgG₂, IgG₃, or IgG₄) that is responsible for increasing the *in vivo* serum half-life of the IgG molecule (e.g., Ghetie et al., Ann. Rev. Immunol. 18:739-766 (2000), Table 1). Antibodies with substitutions in an Fc region thereof and increased serum half-lives are also described in WO00/42072, WO 02/060919; Shields et al., J. Biol. Chem. 276:6591-6604 (2001); Hinton, J. Biol. Chem. 279:6213-6216(2004)). In another embodiment, the serum half-life can also be increased, for example, by attaching other polypeptide sequences. For example, antibodies or other polypeptides useful in the methods of the invention can be attached to serum albumin or a portion of serum albumin that binds to the FcRn receptor or a serum albumin binding peptide so that serum albumin binds to the antibody or polypeptide, e.g., such polypeptide sequences are disclosed in WO01/45746. In one embodiment, the serum albumin peptide to be attached comprises an amino acid sequence of DICLPRWGCLW. In another embodiment, the half-life of a Fab is increased by these methods. *See also,* Dennis et al. J. Biol. Chem. 277:35035-35043 (2002) for serum albumin binding peptide sequences.

A "chimeric VEGF receptor protein" is a VEGF receptor molecule having amino acid sequences derived from at least two different proteins, at least one of which is as VEGF receptor protein. In certain embodiments, the chimeric VEGF receptor protein is capable of binding to and inhibiting the biological activity of VEGF.

An "isolated" polypeptide or "isolated" antibody is one that has been identified and separated and/or recovered from a component of its natural environment. Contaminant components of its natural environment are materials that would interfere with diagnostic or therapeutic uses for the polypeptide or antibody, and may include enzymes, hormones, and other proteinaceous or nonproteinaceous solutes. In certain embodiments, the polypeptide or antibody will be purified (1) to greater than 95% by weight of polypeptide or antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, silver stain. Isolated polypeptide or antibody includes the polypeptide or antibody in situ within recombinant cells since at least one component of the polypeptide's natural environment will not be present. Ordinarily, however, isolated polypeptide or antibody will be prepared by at least one purification step.

By "fragment" is meant a portion of a polypeptide or nucleic acid molecule that contains, preferably, at least 10%, 20%, 30%, 40%, 50%, 60%, 70%, 80%, 90%, 95%, or more of the entire length of the reference nucleic acid molecule or polypeptide. A fragment may contain 10, 20, 30, 40, 50, 60, 70, 80, 90, or 100, 200, 300, 400, 500, 600, or more nucleotides or 10, 20, 30, 40, 50, 60, 70, 80, 90, 100, 120, 140, 160, 180, 190, 200 amino acids or more.

An "anti-angiogenesis agent" or "angiogenesis inhibitor" refers to a small molecular weight substance, a polynucleotide, a polypeptide, an isolated protein, a recombinant protein, an antibody, or conjugates or fusion proteins thereof, that inhibits angiogenesis, vasculogenesis, or undesirable vascular permeability, either directly or indirectly. It should be understood that the anti-angiogenesis agent includes those agents that bind and block the angiogenic activity of the angiogenic factor or its receptor. For example, an anti-angiogenesis agent is an antibody or other antagonist to an angiogenic agent as defined above, e.g., antibodies to VEGF-A or to the VEGF-A receptor (e.g., KDR receptor or Flt-1 receptor), anti-PDGFR inhibitors such as Gleevec™ (Imatinib Mesylate). Anti-angiogensis agents also include native angiogenesis inhibitors, e.g., angiostatin, endostatin, etc. See, e.g., Klagsbrun and D'Amore, Annu. Rev. Physiol., 53:217-39 (1991); Streit and Detmar, Oncogene, 22:3172-3179 (2003) (e.g., Table 3 listing anti-angiogenic therapy in malignant melanoma); Ferrara & Alitalo, Nature Medicine 5:1359-1364 (1999); Tonini et al., Oncogene, 22:6549-6556 (2003) (e.g., Table 2 listing known antiangiogenic factors); and Sato. Int. J. Clin. Oncol., 8:200-206 (2003) (e.g., Table 1 lists anti-angiogenic agents used in clinical trials).

"Treatment" refers to both therapeutic treatment and prophylactic or preventative measures. Those in need of treatment include those already having a benign, pre-cancerous, or non-metastatic tumor as well as those in which the occurrence or recurrence of cancer is to be prevented.

The term "therapeutically effective amount" refers to an amount of a VEGF-specific antagonist to treat or prevent a disease or disorder in a mammal. In the case of pre-cancerous, benign, or early stage tumors, the therapeutically effective amount of the VEGF-specific antagonist may reduce the number of cancer cells; reduce the primary tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. For the treatment of tumor dormancy or micrometastases, the therapeutically effective amount of the VEGF-specific antagonist may reduce the number or proliferation of micrometastases; reduce or prevent the growth of a dormant tumor; or reduce or prevent the recurrence of a tumor after treatment or removal (e.g., using an anti-cancer therapy such as surgery, radiation therapy, or chemotherapy). To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), the response rates (RR), duration of response, time in remission, and/or quality of life. The effective amount may improve disease free survival (DFS), improve overall survival (OS), decrease likelihood of recurrence, extend time to recurrence, extend time to distant recurrence (*i.e.* recurrence outside of the primary site), cure cancer, improve symptoms of cancer (e.g. as gauged using a cancer specific survey), reduce appearance of second primary cancer, etc.

"Operable" cancer is cancer which is confined to the primary organ and suitable for surgery.

"Survival" refers to the patient remaining alive, and includes disease free survival (DFS), progression free survival (PFS) and overall survival (OS). Survival can be estimated by the Kaplan-Meier method, and any differences in survival are computed using the stratified log-rank test.

"Disease free survival (DFS)" refers to the patient remaining alive, without return of the cancer, for a defined period of time such as about 1 year, about 2 years, about 3 years, about 4 years, about 5 years, about 10 years, etc., from initiation of treatment or from initial diagnosis. In one aspect of the invention, DFS is analyzed according to the intent-to-treat principle, i.e., patients are evaluated on the basis of their assigned therapy. The events used in the analysis of DFS can include local, regional and distant recurrence of cancer, occurrence of secondary cancer, and death from any cause in patients without a prior event (e.g, breast cancer recurrence or second primary cancer).

"Overall survival" refers to the patient remaining alive for a defined period of time, such as about 1 year, about 2 years, about 3 years, about 4 years, about 5 years, about 10 years, etc., from initiation of treatment or from initial diagnosis. In the studies underlying the invention the event used for survival analysis was death from any cause.

By "extending survival" is meant increasing DFS and/or OS in a treated patient relative to an untreated patient (i.e. relative to a patient not treated with a VEGF-specific antagonist, e.g., a VEGF antibody), or relative to a control treatment protocol, such as treatment only with the chemotherapeutic agent, such as paclitaxel. Survival is monitored for at least about six months, or at least about 1 year, or at least about 2 years, or at least about 3 years, or at least about 4 years, or at least about 5 years, or at least about 10 years, etc., following the initiation of treatment or following the initial diagnosis.

"Hazard ratio" in survival analysis is a summary of the difference between two survival curves, representing the reduction in the risk of death on treatment compared to control, over a period of follow-up. Hazard ratio is a statistical definition for rates of events. For the purpose of the invention, hazard ratio is defined as representing the probability of an event in the experimental arm divided by the probability of an event in the control arm at any specific point in time.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

By "monotherapy" is meant a therapeutic regimen that includes only a single therapeutic agent for the treatment of the cancer or tumor during the course of the treatment period. Monotherapy using a VEGF-specific antagonist means that the VEGF-specific antagonist is administered in the absence of an additional anti-cancer therapy during that treatment period.

By "maintenance therapy" is meant a therapeutic regimen that is given to reduce the likelihood of disease recurrence or progression. Maintenance therapy can be provided for any length of time, including extended time periods up to the life-span of the subject. Maintenance therapy can be provided after initial therapy or in conjunction with initial or additional therapies. Dosages used for maintenance therapy can vary and can include diminished dosages as compared to dosages used for other types of therapy.

"Neoadjuvant therapy" or "preoperative therapy" herein refers to therapy given prior to surgery. The goal of neoadjuvant therapy is to provide immediate systemic treatment, potentially eradicating micrometastases that would otherwise proliferate if the standard sequence of surgery followed by systemic therapy were followed. Neoadjuvant therapy may also help to reduce tumor size thereby allowing complete resection of initially unresectable tumors or preserving portions of the organ and its functions. Furthermore, neoadjuvant therapy permits an in vivo assessment of drug efficacy, which may guide the choice of subsequent treatments.

"Adjuvant therapy" herein refers to therapy given after surgery, where no evidence of residual disease can be detected, so as to reduce the risk of disease recurrence. The goal of adjuvant therapy is to prevent recurrence of the cancer, and therefore to reduce the chance of cancer-related death.

Herein, "standard of care" chemotherapy refers to the chemotherapeutic agents routinely used to treat a particular cancer.

"Definitive surgery" is used as that term is used within the medical community. Definitive surgery includes, for example, procedures, surgical or otherwise, that result in removal or resection of the tumor, including those that result in the removal or resection of all grossly visible tumor. Definitive surgery includes, for example, complete or curative resection or complete gross resection of the tumor. Definitive surgery includes procedures that occurs in one or more stages, and includes, for example, multi-stage surgical procedures where one or more surgical or other procedures are performed prior to resection of the tumor. Definitive surgery includes procedures to remove or resect the tumor including involved organs, parts of organs and tissues, as well as surrounding organs, such as lymph nodes, parts of organs, or tissues.

The terms "cancer" and "cancerous" refer to or describe the physiological condition in mammals that is typically characterized by unregulated cell growth. Included in this definition are benign and malignant cancers as well as dormant tumors or micrometastatses. By "early stage cancer" or "early stage tumor" is meant a cancer that is not invasive or metastatic or is classified as a Stage 0, I, or II cancer.

The term "pre-cancerous" refers to a condition or a growth that typically precedes or develops into a cancer. A "pre-cancerous" growth will have cells that are characterized by abnormal cell cycle regulation, proliferation, or differentiation, which can be determined by markers of cell cycle regulation, cellular proliferation, or differentiation.

By "dysplasia" is meant any abnormal growth or development of tissue, organ, or cells. Preferably, the dysplasia is high grade or precancerous.

By "metastasis" is meant the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant.

By "micrometastasis" is meant a small number of cells that have spread from the primary tumor to other parts of the body. Micrometastasis may or may not be detected in a screening or diagnostic test.

By "non-metastatic" is meant a cancer that is benign or that remains at the primary site and has not penetrated into the lymphatic or blood vessel system or to tissues other than the primary site. Generally, a non-metastatic cancer is any cancer that is a Stage 0, I, or II cancer, and occasionally a Stage III cancer.

Reference to a tumor or cancer as a "Stage 0," "Stage I," "Stage II," "Stage III," or "Stage IV" indicates classification of the tumor or cancer using the Overall Stage Grouping or Roman Numeral Staging methods known in the art. Although the actual stage of the cancer is dependent on the type of cancer, in general, a Stage 0 cancer is an in situ lesion, a Stage I cancer is small localized tumor, a Stage II and III cancer is a local advanced tumor which exhibits involvement of the local lymph nodes, and a Stage IV cancer represents metastatic cancer. The specific stages for each type of tumor is known to the skilled clinician.

"Tumor", as used herein, refers to all neoplastic cell growth and proliferation, whether malignant or benign, and all pre-cancerous and cancerous cells and tissues.

By "primary tumor" or "primary cancer" is meant the original cancer and not a metastatic lesion located in another tissue, organ, or location in the subject's body.

By "benign tumor" or "benign cancer" is meant a tumor that remains localized at the site of origin and does not have the capacity to infiltrate, invade, or metastasize to a distant site.

"Cancer recurrence" herein refers to a return of cancer following treatment, and includes return of cancer in the primary organ, as well as distant recurrence, where the cancer returns outside of the primary organ.

By "tumor dormancy" is meant a prolonged quiescent state in which tumor cells are present but tumor progression is not clinically apparent. A dormant tumor may or may not be detected in a screening or diagnostic test.

By "tumor burden" is meant the number of cancer cells, the size of a tumor, or the amount of cancer in the body. Tumor burden is also referred to as tumor load.

By "tumor number" is meant the number of tumors.

By "subject" is meant a mammal, including, but not limited to, a human or non-human mammal, such as a bovine, equine, canine, ovine, or feline. Preferably, the subject is a human.

A "population" of subjects refers to a group of subjects with cancer, such as in a clinical trial, or as seen by oncologists following FDA approval for a particular indication, such as cancer neoadjuvant therapy. In one embodiment, the population comprises at least 3000 subjects.

The term "anti-cancer therapy" refers to a therapy useful in treating cancer. Examples of anti-cancer therapeutic agents include, but are limited to, e.g., surgery, chemotherapeutic agents, growth inhibitory agents, cytotoxic agents, agents used in radiation therapy, anti-angiogenesis agents, apoptotic agents, anti-tubulin agents, and other agents to treat cancer, such as anti-HER-2 antibodies, anti-CD20 antibodies, an epidermal growth factor receptor (EGFR) antagonist (e.g., a tyrosine kinase inhibitor), HER1/EGFR inhibitor (e.g., erlotinib (Tarceva™), platelet derived growth factor inhibitors (e.g., Gleevec™ (Imatinib Mesylate)), a COX-2 inhibitor (e.g., celecoxib), interferons, cytokines, antagonists (e.g., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also included in the invention.

The term "cytotoxic agent" as used herein refers to a substance that inhibits or prevents the function of cells and/or causes destruction of cells. The term is intended to include radioactive isotopes (e.g., I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2- ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol- Myers Squibb Oncology, Princeton, N.J.), ABRAXANE™ Cremophor-free, albumin-engineered nanoparticle formulation of paclitaxel (American Pharmaceutical Partners, Schaumberg, Illinois), and TAXOTERE® doxetaxel (Rhône- Poulenc Rorer, Antony, France); chloranbucil; GEMZAR® gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; NAVELBINE® vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluorometlhylornithine (DMFO); retinoids such as retinoic acid; capecitabine; combretastatin; leucovorin (LV); oxaliplatin, including the oxaliplatin treatment regimen (FOLFOX); inhibitors of PKC-alpha, Raf, H-Ras, EGFR (e.g.. erlotinib (Tarceva™)) and VEGF-A that reduce cell proliferation and pharmaceutically acceptable salts, acids or derivatives of any of the above.

Also included in this definition are anti-hormonal agents that act to regulate or inhibit hormone action on tumors such as anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON- toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole; and antiandrogens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Raf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELIX® rmRH; Vinorelbine and Esperamicins (see U.S. Pat. No. 4,675,187), and pharmaceutically acceptable salts, acids or derivatives of any of the above.

A "growth inhibitory agent" when used herein refers to a compound or composition which inhibits growth of a cell in vitro and/or in vivo. Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), TAXOL®, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); epidermal growth factor; hepatic growth factor; fibroblast growth factor; prolactin; placental lactogen; tumor necrosis factor-alpha and -beta; mullerian-inhibiting substance; mouse gonadotropin-associated peptide; inhibin; activin; vascular endothelial growth factor; integrin; thrombopoietin (TPO); nerve growth factors such as NGF-alpha; platelet-growth factor; transforming growth factors (TGFs) such as TGF-alpha and TGF-beta; insulin-like growth factor-I and -II; erythropoietin (EPO); osteoinductive factors; interferons such as interferon-alpha, -beta and -gamma colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; a tumor necrosis factor such as TNF-alpha or TNF-beta; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. See, e.g., Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one time administration and typical dosages range from 10 to 200 units (Grays) per day.

By "reduce or inhibit" is meant the ability to cause an overall decrease preferably of 20% or greater, more preferably of 50% or greater, and most preferably of 75%, 85%, 90%, 95%, or greater. Reduce or inhibit can refer to the symptoms of the disorder being treated, the presence or size of metastases or micrometastases, the size of the primary tumor, the presence or the size of the dormant tumor, or the size or number of the blood vessels in angiogenic disorders.

By "antisense nucleobase oligomer" is meant a nucleobase oligomer, regardless of length, that is complementary to at least a portion of the coding strand or mRNA of a gene.

By a "nucleobase oligomer" is meant a compound that includes a chain of at least eight nucleobases, preferably at least twelve, and most preferably at least sixteen bases, joined together by linkage groups. Included in this definition are natural and non-natural oligonucleotides, both modified and unmodified, as well as oligonucleotide mimetics such as Protein Nucleic Acids, locked nucleic acids, and arabinonucleic acids. Numerous nucleobases and linkage groups may be employed in the nucleobase oligomers of the invention, including those described in U.S. Patent Publication Nos. 20030114412 (see for example paragraphs 27-45 of the publication) and 20030114407 (see for example paragraphs 35-52 of the publication), incorporated herein by reference. The nucleobase oligomer can also be targeted to the translational start and stop sites. In certain embodiments, the antisense nucleobase oligomer comprises from about 8 to 30 nucleotides. The antisense nucleobase oligomer can also contain at least 40, 60, 85, 120, or more consecutive nucleotides that are complementary to VEGF mRNA or DNA, and may be as long as the full-length mRNA or gene.

By "small RNA" is meant any RNA molecule, either single-stranded or double-stranded, that is at least 15 nucleotides, preferably, 17, 18, 19, 20, 21, 22, 23, 24, 25, 26, 27, 28, 29, 30, 31, 32, 33, 34, or 35, nucleotides in length and even up to 50 or 100 nucleotides in length (inclusive of all integers in between). In certain embodiments, the small RNA is capable of mediating RNAi. As used herein the phrase "mediates RNAi" refers to the ability to distinguish which RNAs are to be degraded by the RNAi machinery or process. Included within the term small RNA are "small interfering RNAs" and "microRNA." In general, microRNAs (miRNAs) are small (e.g., 17-26 nucleotides), single-stranded noncoding RNAs that are processed from approximately 70 nucleotide hairpin precursor RNAs by Dicer. Small interfering RNAs (siRNAs) are of a similar size and are also non-coding; however, siRNAs are processed from long dsRNAs and are usually double stranded. siRNAs can also include short hairpin RNAs in which both strands of an siRNA duplex are included within a single RNA molecule. Small RNAs can be used to describe both types of RNA. These terms include double-stranded RNA, single-stranded RNA, isolated RNA (partially purified RNA, essentially pure RNA, synthetic RNA, recombinantly produced RNA), as well as altered RNA that differs from naturally occurring RNA by the addition, deletion, substitution and/or alteration of one or more nucleotides. Such alterations can include addition of non-nucleotide material, such as to the end(s) of the small RNA or internally (at one or more nucleotides of the RNA). Nucleotides in the RNA molecules of the invention can also comprise non-standard nucleotides, including non-naturally occurring nucleotides or deoxyribonucleotides. See "nucleobase oligomers" above for additional modifications to the nucleic acid molecule. In one embodiment, the RNA molecules contain a 3' hydroxyl group.

The term "intravenous infusion" refers to introduction of a drug into the vein of an animal or human patient over a period of time greater than approximately 5 minutes, preferably between approximately 30 to 90 minutes, although, according to the invention, intravenous infusion is alternatively administered for 10 hours or less.

The term "intravenous bolus" or "intravenous push" refers to drug administration into a vein of an animal or human such that the body receives the drug in approximately 15 minutes or less, preferably 5 minutes or less.

The term "subcutaneous administration" refers to introduction of a drug under the skin of an animal or human patient, preferable within a pocket between the skin and underlying tissue, by relatively slow, sustained delivery from a drug receptacle. The pocket may be created by pinching or drawing the skin up and away from underlying tissue.

The term "subcutaneous infusion" refers to introduction of a drug under the skin of an animal or human patient, preferably within a pocket between the skin and underlying tissue, by relatively slow, sustained delivery from a drug receptacle for a period of time including, but not limited to, 30 minutes or less, or 90 minutes or less. Optionally, the infusion may be made by subcutaneous implantation of a drug delivery pump implanted under the skin of the animal or human patient, wherein the pump delivers a predetermined amount of drug for a predetermined period of time, such as 30 minutes, 90 minutes, or a time period spanning the length of the treatment regimen.

The term "subcutaneous bolus" refers to drug administration beneath the skin of an animal or human patient, where bolus drug delivery is preferably less than approximately 15 minutes, more preferably less than 5 minutes, and most preferably less than 60 seconds. Administration is preferably within a pocket between the skin and underlying tissue, where the pocket is created, for example, by pinching or drawing the skin up and away from underlying tissue.

The word "label" when used herein refers to a detectable compound or composition which is conjugated directly or indirectly to the polypeptide. The label may be itself be detectable (e.g., radioisotope labels or fluorescent labels) or, in the case of an enzymatic label, may catalyze chemical alteration of a substrate compound or composition which is detectable.

### II. Using VEGF-specific antagonists for early stage tumor treatment and neoadjuvant and adjuvant therapy

The invention features the use of VEGF-specific antagonists to treat a subject having a benign, pre-cancerous, or non-metastatic tumor; to treat a subject having a dormant tumor or micrometastases; or to treat a subject having or to treat a subject at risk of developing cancer. For example, using two independent approaches to inhibit VEGF, namely, monotherapy with a monoclonal antibody (mAb) targeting VEGF-A and genetic deletion of VEGF-A, we have demonstrated, using the Apc^{min/+} mouse model of early intestinal adenoma formation, that inhibition of VEGF signaling is sufficient for tumor growth cessation and confers a long-term survival benefit in an intestinal adenoma model. We have also demonstrated, using a monoclonal antibody (mAb) targeting VEGF-A, that inhibition of VEGF-A was sufficient to inhibit pituitary adenoma growth and to lower excess hormonal secretion in a mouse model of multiple endocrine neoplasia type 1 (MEN1). Moreover, we have demonstrated, using a mouse pancreatic islet tumor model (RIP-TβAg) and a monoclonal antibody (mAb) targeting VEGF-A, that inhibition of VEGF causes a dramatic reduction of tumor angiogenesis and, when used as a therapy following cytoreduction using surgery or chemotherapeutic agents, suppresses re-growth of tumors. The invention also features the use of VEGF antagonists in the neoadjuvant and adjuvant setting.

### III. VEGF-specific antagonists

A VEGF-specific antagonist refers to a molecule (peptidyl or non-peptidyl) capable of binding to VEGF, reducing VEGF expression levels, or neutralizing, blocking, inhibiting, abrogating, reducing, or interfering with VEGF biological activities, including VEGF binding to one or more VEGF receptors and VEGF mediated angiogenesis and endothelial cell survival or proliferation. Preferably, the VEGF-specific antagonist reduces or inhibits, by at least 10%, 20%, 30%, 40%, 50%, 60%, 70%, 80%, 90% or more, the expression level or biological activity of VEGF. Preferably, the VEGF inhibited by the VEGF-specific antagonist is a VEGF isoform or multiple VEGF isoforms, e.g., VEGF (8-109), VEGF (1-109), VEGF₁₆₅. VEGF₁₂₁, VEGF₁₄₅, VEGF₁₈₉, or VEGF₂₀₆

VEGF-specific antagonists useful in the methods of the invention include peptidyl or non-peptidyl compounds that specifically bind VEGF, such as anti-VEGF antibodies and antigen-binding fragments thereof, antagonist variants of VEGF polypeptides, or fragments thereof that specifically bind to VEGF, and receptor molecules and derivatives that bind specifically to VEGF thereby sequestering its binding to one or more receptors (e.g., soluble VEGF receptor proteins, or VEGF binding fragments thereof, or chimeric VEGF receptor proteins), fusions proteins (e.g., VEGF-Trap (Regeneron)), and VEGF₁₂₁-gelonin (Peregrine). VEGF-specific antagonists also include antisense nucleobase oligomers complementary to at least a fragment of a nucleic acid molecule encoding a VEGF polypeptide; small RNAs complementary to at least a fragment of a nucleic acid molecule encoding a VEGF polypeptide; ribozymes that target VEGF; peptibodies to VEGF; and VEGF aptamers.

### A. Anti-VEGF atitibodies

Anti-VEGF antibodies that are useful in the methods of the invention include any antibody, or antigen binding fragment thereof, that bind with sufficient affinity and specificity to VEGF and can reduce or inhibit the biological activity of VEGF. An anti-VEGF antibody will usually not bind to other VEGF homologues such as VEGF-B or VEGF-C, nor other growth factors such as PIGF, PDGF, or bFGF.

In certain embodiments of the invention, the anti-VEGF antibodies include, but are not limited to, a monoclonal antibody that binds to the same epitope as the monoclonal anti-VEGF antibody A4.6.1 produced by hybridoma ATCC HB 10709; a recombinant humanized anti-VEGF monoclonal antibody generated according to Presta et al. (1997) Cancer Res. 57:4593-4599, including but not limited to the antibody known as bevacizumab (BV; Avastin®). Bevacizumab includes mutated human IgG1 framework regions and antigen-binding complementarity-determining regions from the murine anti-hVEGF monoclonal antibody A.4.6.1 that blocks binding of human VEGF to its receptors. Approximately 93% of the amino acid sequence of bevacizumab, including most of the framework regions, is derived from human IgG1, and about 7% of the sequence is derived from the murine antibody A4.6.1. Bevacizumab has a molecular mass of about 149,000 daltons and is glycosylated. Bevacizumab and other humanized anti-VEGF antibodies are further described in U.S. Pat. No. 6,884,879 issued Feb. 26, 2005. Additional examples of antibodies include, but are not limited to, the G6 or B20 series antibodies (e.g., G6-31, B20-4.1), as described in PCT Application Publication No. WO2005/012359. For additional antibodies see U.S. Pat. Nos. 7,060,269, 6,582,959, 6,703,020; 6, 342,219; 6,054,297; WO98/45332; WO 96/30046; WO94/10202; EP 0666868B1; U.S. Patent Application Publication Nos. 2006009360, 20050186208, 20030206899, 20030190317, 20030203409, and 20050112126; and Popkov et al., Journal of Immunological Methods 288:149-164 (2004). Other examples of antibodies that can be used in the invention include those that bind to a functional epitope on human VEGF comprising of residues F17, M18, D19, Y21, Y25, Q89, I91, K101, E103, and C104 or, alteratively, comprising residues F17, Y21, Q22, Y25, D63, I83 and Q89.

A G6 series antibody according to this invention is an anti-VEGF antibody that is derived from a sequence of a G6 antibody or G6-derived antibody according to any one of Figures 7, 24-26, and 34-35 of PCT Application Publication No. WO2005/012359. In one embodiment, the G6 series antibody binds to a functional epitope on human VEGF comprising residues F17, Y21, Q22, Y25, D63, I83 and Q89.

A B20 series antibody according to this invention is an anti-VEGF antibody that is derived from a sequence of the B20 antibody or a B20-derived antibody according to any one of Figures 27-29 of PCT Application Publication No. WO2005/012359. In one embodiment, the B20 series antibody binds to a functional epitope on human VEGF comprising residues F17, M18, D19, Y21, Y25, Q89, I91, K101, E103, and C104.

A "functional epitope" according to this invention refers to amino acid residues of an antigen that contribute energetically to the binding of an antibody. Mutation of any one of the energetically contributing residues of the antigen (for example, mutation of wild-type VEGF by alanine or homolog mutation) will disrupt the binding of the antibody such that the relative affinity ratio (IC50mutant VEGF/IC50wild-type VEGF) of the antibody will be greater than 5 (see Example 2 of WO2005/012359). In one embodiment, the relative affinity ratio is determined by a solution binding phage displaying ELISA. Briefly, 96-well Maxisorp immunoplates (NUNC) are coated overnight at 4°C with an Fab form of the antibody to be tested at a concentration of 2ug/ml in PBS, and blocked with PBS, 0.5% BSA, and 0.05% Tween20 (PBT) for 2h at room temperature. Serial dilutions of phage displaying hVEGF alanine point mutants (residues 8-109 form) or wild type hVEGF (8-109) in PBT are first incubated on the Fab-coated plates for 15 min at room temperature, and the plates are washed with PBS, 0.05% Tween20 (PBST). The bound phage is detected with an anti-M13 monoclonal antibody horseradish peroxidase (Amersham Pharmacia) conjugate diluted 1:5000 in PBT, developed with 3,3', 5,5'-tetramethylbenzidine (TMB, Kirkegaard & Perry Labs, Gaithersburg, MD) substrate for approximately 5 min, quenched with 1.0 M H3PO4, and read spectrophotometrically at 450 nm. The ratio of IC50 values (IC50,ala/IC50,wt) represents the fold of reduction in binding affinity (the relative binding affinity)

### B. VEGF receptor molecules

The two best characterized VEGF receptors are VEGFR1 (also known as Flt-1) and VEGFR2 (also known as KDR and FLK-1 for the murine homolog). The specificity of each receptor for each VEGF family member varies but VEGF-A binds to both Flt-1 and KDR. The full length Flt-1 receptor includes an extracellular domain that has seven Ig domains, a transmembrane domain, and an intracellular domain with tyrosine kinase activity. The extracellular domain is involved in the binding of VEGF and the intracellular domain is involved in signal transduction.

VEGF receptor molecules, or fragments thereof, that specifically bind to VEGF can be used in the methods of the invention to bind to and sequester the VEGF protein, thereby preventing it from signaling. In certain embodiments, the VEGF receptor molecule, or VEGF binding fragment thereof, is a soluble form, such as sFlt-1. A soluble form of the receptor exerts an inhibitory effect on the biological activity of the VEGF protein by binding to VEGF, thereby preventing it from binding to its natural receptors present on the surface of target cells. Also included are VEGF receptor fusion proteins, examples of which are described below.

A chimeric VEGF receptor protein is a receptor molecule having amino acid sequences derived from at least two different proteins, at least one of which is a VEGF receptor protein (e.g., the flt-1 or KDR receptor), that is capable of binding to and inhibiting the biological activity of VEGF. In certain embodiments, the chimeric VEGF receptor proteins of the invention consist of amino acid sequences derived from only two different VEGF receptor molecules; however, amino acid sequences comprising one, two, three, four, five, six, or all seven Ig-like domains from the extracellular ligand-binding region of the flt-1 and/or KDR receptor can be linked to amino acid sequences from other unrelated proteins, for example, immunoglobulin sequences. Other amino acid sequences to which Ig-like domains are combined will be readily apparent to those of ordinary skill in the art. Examples of chimeric VEGF receptor proteins include, e.g., soluble Flt-1/Fc, KDR/Fc, or FLt-1/KDR/Fc (also known as VEGF Trap). (See for example PCT Application Publication No. WO97/44453)

A soluble VEGF receptor protein or chimeric VEGF receptor proteins of the invention includes VEGF receptor proteins which are not fixed to the surface of cells via a transmembrane domain. As such, soluble forms of the VEGF receptor, including chimeric receptor proteins, while capable of binding to and inactivating VEGF, do not comprise a transmembrane domain and thus generally do not become associated with the cell membrane of cells in which the molecule is expressed.

### C. Ribozymes

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994) and PCT Application Publication No. WO 97/33551. One exemplary ribozyme that targets VEGF expression is Angiozyme™. (See, for example, U.S. Patent Application Publication No. 20060035278.)

### D. Aptamers

Aptamers are nucleic acid molecules that form tertiary structures that specifically bind to a target molecule, such as a VEGF polypeptide. The generation and therapeutic use of aptamers are well established in the art. See, e.g., U.S. Pat. No. 5,475,096. A VEGF aptamer is a pegylated modified oligonucleotide, which adopts a three-dimensional conformation that enables it to bind to extracellular VEGF. One example of a therapeutically effective aptamer that targets VEGF for treating age-related macular degeneration is pegaptanib (Macugen™, Eyetech, New York). Additional information on aptamers can be found in U.S. Patent Application Publication No. 20060148748.

### E. Peptibodies

A peptibody is a peptide sequence linked to an amino acid sequence encoding a fragment or portion of an immunoglobulin molecule. Polypeptides may be derived from randomized sequences selected by any method for specific binding, including but not limited to, phage display technology. In one embodiment, the selected polypeptide may be linked to an amino acid sequence encoding the Fc portion of an immunoglobulin. Peptibodies that specifically bind to and antagonize VEGF are also useful in the methods of the invention.

### F. VEGF-specific antagonistic nucleic acid molecules

The methods of the invention also feature the use of VEGF-specific antagonistic nucleic acid molecules including antisense nucleobase oliogmers and small RNAs.

In one embodiment, the invention features the use of antisense nucleobase oligomers directed to VEGF RNA. By binding to the complementary nucleic acid sequence (the sense or coding strand), antisense nucleobase oligomers are able to inhibit protein expression presumably through the enzymatic cleavage of the RNA strand by RNAse H.

One example of an antisense nucleobase oligomer particularly useful in the methods and compositions of the invention is a morpholino oligomer. Morpholinos are used to block access of other molecules to specific sequences within nucleic acid molecules. They can block access of other molecules to small (∼25 base) regions of ribonucleic acid (RNA). Morpholinos are sometimes referred to as PMO, an acronym for phosphorodiamidate morpholino oligo.

Morpholinos are used to knock down gene function by preventing cells from making a targeted protein or by modifying the splicing of pre-mRNA. Morpholinos are synthetic molecules that bind to complementary sequences of RNA by standard nucleic acid base-pairing. While morpholinos have standard nucleic acid bases, those bases are bound to morpholine rings instead of deoxyribose rings and linked through phosphorodiamidate groups instead of phosphates. Replacement of anionic phosphates with the uncharged phosphorodiamidate groups eliminates ionization in the usual physiological pH range, so morpholinos in organisms or cells are uncharged molecules.

Morpholinos act by "steric blocking" or binding to a target sequence within an RNA and blocking molecules which might otherwise interact with the RNA. Because of their completely unnatural backbones, morpholinos are not recognized by cellular proteins. Nucleases do not degrade morpholinos and morpholinos do not activate toll-like receptors and so they do not activate innate immune responses such as the interferon system or the NF-κB mediated inflammation response. Morpholinos are also not known to modify methylation of DNA. Therefore, morpholinos directed to any part of VEGF that can reduce or inhibit the expression levels or biological activity of VEGF are particularly useful in the methods and compositions of the invention.

The invention also features the use of RNA interference (RNAi) to inhibit expression of VEGF. RNAi is a form of post-transcriptional gene silencing initiated by the introduction of double-stranded RNA (dsRNA). Short 15 to 32 nucleotide double-stranded RNAs, known generally as "siRNAs," "small RNAs," or "microRNAs" are effective at down-regulating gene expression in nematodes (Zamore et al., Cell 101: 25-33) and in mammalian tissue culture cell lines (Elbashir et al., Nature 411:494-498, 2001, hereby incorporated by reference). The further therapeutic effectiveness of this approach in mammals was demonstrated *in vivo* by McCaffrey et al. (Nature 418:38-39. 2002). The small RNAs are at least 15 nucleotides, preferably, 17, 18, 19, 20, 21, 22, 23, 24, 25, 26, 27, 28, 29, 30, 31, 32, 33, 34, 35, nucleotides in length and even up to 50 or 100 nucleotides in length (inclusive of all integers in between). Such small RNAs that are substantially identical to or complementary to any region of VEGF, are included as VEGF-specific antagonists of the invention.

The specific requirements and modifications of small RNA are known in the art and are described, for example, in PCT Application Publication No. WO01/75164 and U.S. Application Publication Numbers 20060134787, 20050153918, 20050058982, 20050037988, and 20040203145, the relevant portions of which are herein incorporated by reference. In particular embodiments, siRNAs can be synthesized or generated by processing longer double-stranded RNAs, for example, in the presence of the enzyme dicer under conditions in which the dsRNA is processed to RNA molecules of about 17 to about 26 nucleotides. siRNAs can also be generated by expression of the corresponding DNA fragment (e.g., a hairpin DNA construct). Generally, the siRNA has a characteristic 2- to 3- nucleotide 3' overhanging ends, preferably these are (2'-deoxy) thymidine or uracil. The siRNAs typically comprise a 3' hydroxyl group. In some embodiments, single stranded siRNAs or blunt ended dsRNA are used. In order to further enhance the stability of the RNA, the 3' overhangs are stabilized against degradation. In one embodiment, the RNA is stabilized by including purine nucleotides, such as adenosine or guanosine. Alternatively, substitution of pyrimidine nucleotides by modified analogs e.g. substitution of uridine 2-nucleotide overhangs by (2'-deoxy)thymide is tolerated and does not affect the efficiency of RNAi. The absence of a 2' hydroxyl group significantly enhances the nuclease resistance of the overhang in tissue culture medium.

siRNA molecules can be obtained through a variety of protocols including chemical synthesis or recombinant production using a *Drosophila in vitro* system. They can be commercially obtained from companies such as Dharmacon Research Inc. or Xeragon Inc., or they can be synthesized using commercially available kits such as the *Silencer*™ siRNA Construction Kit from Ambion (catalog number 1620) or HiScribe™ RNAi Transcription Kit from New England BioLabs (catalog number E2000S).

Alternatively siRNA can be prepared using standard procedures for *in vitro* transcription of RNA and dsRNA annealing procedures such as those described in Elbashir et al. (Genes & Dev. 15:188-200, 2001), Girard et al. (Nature 442:199-202 (2006)), Aravin et al. (Nature 442:203-207 (2006)), Grivna et al. (Genes Dev. 20:1709-1714 (2006))), and Lau et al. (Science 313:363-367 (2006)).

Short hairpin RNAs (shRNAs), as described in Yu et al. (Proc. Natl. Acad. Sci USA, 99:6047-6052, 2002) or Paddison et al. (Genes & Dev, 16:948-958, 2002), can also be used in the methods of the invention. shRNAs are designed such that both the sense and antisense strands are included within a single RNA molecule and connected by a loop of nucleotides (3 or more). shRNAs can be synthesized and purified using standard *in vitro* T7 transcription synthesis as described above and in Yu et al., *supra*. shRNAs can also be subcloned into an expression vector that has the mouse U6 promoter sequences which can then be transfected into cells and used for *in vivo* expression of the shRNA.

A variety of methods are available for transfection, or introduction, of dsRNA into mammalian cells. For example, there are several commercially available transfection reagents useful for lipid-based transfection of siRNAs including, but not limited to, TransIT-TKO™ (Mirus, Cat. # MIR 2150), Transmessenger™ (Qiagen, Cat. # 301525), Oligofectamine™ and Lipofectamine™ (Invitrogen, Cat. # MIR 12252-011 and Cat. #13778-075), siPORT™ (Ambion, Cat. #1631), DharmaFECT™ (Fisher Scientific, Cat. # T-2001-01). Agents are also commercially available for electroporation-based methods for transfection of siRNA, such as siPORTer™ (Ambion Inc. Cat. # 1629). Microinjection techniques can also be used. The small RNA can also be transcribed from an expression construct introduced into the cells, where the expression construct includes a coding sequence for transcribing the small RNA operably linked to one or more transcriptional regulatory sequences. Where desired, plasmids, vectors, or viral vectors can also be used for the delivery of dsRNA or siRNA and such vectors are known in the art. Protocols for each transfection reagent are available from the manufacturer.

### IV. Therapeutic Uses

Despite the extensive information regarding the role of VEGF in angiogenesis, relatively little is known about the role of VEGF in benign, pre-cancerous, or non-metastatic cancers or in the adjuvant or neoadjuvant setting. We have discovered that VEGF-specific antagonists can be used for the treatment of benign, pre-cancerous, or non-metastatic cancers; for the treatment of dormant tumors or micrometases; for the prevention of tumor recurrence or re-growth; or for treatment or prevention of cancer in a subject at risk for developing cancer. VEGF-specific antagonists can also be used for adjuvant therapy for the treatment of a subject with nonmetastatic cancer, following definitive surgery or for neoadjuvant therapy for the treatment of a subject with an operable cancer where the therapy is provided prior to the surgical removal of operable cancer in the subject. While the therapeutic applications are separated below into therapy, prevention, neoadjuvant therapy, and adjuvant therapy, it will be appreciated by the skilled artisan that these categories are not necessarily mutually exclusive.

### A. Classification of Tumors

The term cancer embraces a collection of proliferative disorders, including but not limited to pre-cancerous growths, benign tumors, malignant tumors, and dormant tumors. Benign tumors remain localized at the site of origin and do not have the capacity to infiltrate, invade, or metastasize to distant sites. Malignant tumors will invade and damage other tissues around them. They can also gain the ability to break off from the original site and spread to other parts of the body (metastasize), usually through the bloodstream or through the lymphatic system where the lymph nodes are located. Dormant tumors are quiescent tumors in which tumor cells are present but tumor progression is not clinically apparent.

Primary tumors are classified by the type of tissue from which they arise; metastatic tumors are classified by the tissue type from which the cancer cells are derived. Over time, the cells of a malignant tumor become more abnormal and appear less like normal cells. This change in the appearance of cancer cells is called the tumor grade, and cancer cells are described as being well-differentiated (low grade), moderately-differentiated, poorly-differentiated, or undifferentiated (high grade). Well-differentiated cells are quite normal appearing and resemble the normal cells from which they originated. Undifferentiated cells are cells that have become so abnormal that it is no longer possible to determine the origin of the cells.

Cancer staging systems describe how far the cancer has spread anatomically and attempt to put patients with similar prognosis and treatment in the same staging group. Several tests may be performed to help stage cancer including biopsy and certain imaging tests such as a chest x-ray, mammogram, bone scan, CT scan, and MRI scan. Blood tests and a clinical evaluation are also used to evaluate a patient's overall health and detect whether the cancer has spread to certain organs.

To stage cancer, the American Joint Committee on Cancer first places the cancer, particularly solid tumors, in a letter category using the TNM classification system. Cancers are designated the letter T (tumor size), N (palpable nodes), and/or M (metastases). T1, T2, T3, and T4 describe the increasing size of the primary lesion; N0, N1, N2, N3 indicates progressively advancing node involvement; and M0 and M1 reflect the absence or presence of distant metastases.

In the second staging method, also known as the Overall Stage Grouping or Roman Numeral Staging, cancers are divided into stages 0 to IV, incorporating the size of primary lesions as well as the presence of nodal spread and of distant metastases. In this system, cases are grouped into four stages denoted by Roman numerals I through IV, or are classified as "recurrent." For some cancers, stage 0 is referred to as "in situ" or "Tis," such as ductal carcinoma in situ or lobular carcinoma in situ for breast cancers. High grade adenomas can also be classified as stage 0. In general, stage I cancers are small localized cancers that are usually curable, while stage IV usually represents inoperable or metastatic cancer. Stage II and III cancers are usually locally advanced and/or exhibit involvement of local lymph nodes. In general, the higher stage numbers indicate more extensive disease, including greater tumor size and/or spread of the cancer to nearby lymph nodes and/or organs adjacent to the primary tumor. These stages are defined precisely, but the definition is different for each kind of cancer and is known to the skilled artisan.

Many cancer registries, such as the NCI's Surveillance, Epidemiology, and End Results Program (SEER), use summary staging. This system is used for all types of cancer. It groups cancer cases into five main categories:
*In situ* is early cancer that is present only in the layer of cells in which it began.
*Localized* is cancer that is limited to the organ in which it began, without evidence of spread.
*Regional* is cancer that has spread beyond the original (primary) site to nearby lymph nodes or organs and tissues.
*Distant* is cancer that has spread from the primary site to distant organs or distant lymph nodes.
*Unknown* is used to describe cases for which there is not enough information to indicate a stage.

In addition, it is common for cancer to return months or years after the primary tumor has been removed. Cancer that recurs after all visible tumor has been eradicated, is called recurrent disease. Disease that recurs in the area of the primary tumor is locally recurrent, and disease that recurs as metastases is referred to as a distant recurrence. A dormant tumor is a tumor that exists in a quiescent state in which tumor cells are present but tumor progression is not clinically apparent. Micrometastases are a small metastases or a number of cells that have spread from the primary tumor to other parts of the body. Micrometastasis may or may not be detected in a screening or diagnostic test. The methods of the invention are useful for preventing the occurrence of dormant tumors or micrometastases or the recurrence of the tumor, for example, in a setting where a dormant tumor or micrometastases is present but may or may not be clinically detected.

The methods of the invention are also useful for the treatment of early cancers including but not limited to benign, pre-cancerous, or non-metastatic tumors. This includes any stage 0, I, or II tumor; any non-metastatic stage II tumor; any condition that typically precedes or develops into a cancer, including but not limited to, dysplasia; and any tumor that remains localized at the site of origin and has not infiltrated, invaded, or metastasized to distant sites. Examples of benign, pre-cancerous, or non-metastatic tumors include a polyp, adenoma, fibroma, lipoma, gastrinoma, insulinoma, chondroma, osteoma, hemangioma, lymphangioma, meningioma, leiomyoma, rhabdomyoma, squamous cell papilloma, acoustic neuromas, neurofibroma, bile duct cystanoma, leiomyomas, mesotheliomas, teratomas, myxomas, trachomas, granulomas, hamartoma, transitional cell papilloma, pleiomorphic adenoma of the salivary gland, desmoid tumor, dermoid cystpapilloma, cystadenoma, focal nodular hyperplasia, and nodular regenerative hyperplasia.

Because angiogenesis is involved in both primary tumor growth and metastasis, the antiangiogenic treatment provided by the invention is capable of inhibiting the neoplastic growth of tumor at the primary site as well as preventing metastasis of tumors at the secondary sites, therefore allowing attack of the tumors by other therapeutics. Examples of cancer to be treated herein include both solid and non-solid or soft tissue tumors. Examples include, but are not limited to, carcinoma, lymphoma, blastoma, sarcoma, and leukemia. More particular examples of such cancers include squamous cell cancer; lung cancer (including small-cell lung cancer, non-small cell lung cancer, adenocarcinoma of the lung, and squamous carcinoma of the lung); cancer of the peritoneum, hepatocellular cancer, gastric or stomach cancer (including gastrointestinal cancer); pancreatic cancer; glioblastoma; cervical cancer; ovarian cancer; liver cancer; bladder cancer; hepatoma; breast cancer; colon cancer; colorectal cancer; endometrial or uterine carcinoma; salivary gland carcinoma; kidney or renal cancer; liver cancer; prostate cancer; vulval cancer; thyroid cancer; hepatic carcinoma; and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome. More particularly, cancers that are amenable to treatment by the VEGF-specific antagonists of the invention include breast cancer, colorectal cancer, rectal cancer, non-small cell lung cancer, non-Hodgkins lymphoma (NHL), renal cell cancer, prostate cancer, liver cancer, pancreatic cancer, soft-tissue sarcoma, kaposi's sarcoma, carcinoid carcinoma, head and neck cancer, brain tumors, gliomas (e.g., anaplastic astrocytoma, anaplastic oligoastrocytoma, anaplastic oligodendroglioma, glioblastoma multiforme), melanoma, ovarian cancer, mesothelioma, and multiple myeloma. More preferably, the methods of the invention are used to treat colorectal cancer in a human patient.

### B. Adenomas

In one embodiment, the methods of the invention are used for the treatment of a benign epithelial cell tumor known as an adenoma. An adenoma is a benign tumor that has a glandular origin. Adenomas typically originate from epithelial cells used for secretion. Epithelial cells are located throughout the body but only a subset of such cells is used for secretion. Epithelial cells that are used for secretion make up specific parts of the body referred to as glands. Glands have the job of forming a number of substances in the body including, but not limited to, sweat, saliva, breast milk, mucous, and hormones. An adenoma can form from most glandular cells in the body.

An adenoma may form in a similar way to a malignant or cancerous tumor. Adenomas are benign and therefore do not metastasize or spread to other organs or tissues. Although most adenomas remain benign, some adenomas can develop into malignancies, and, if this occurs, the newly malignant adenoma is called an adenocarcinoma. For example, colon and rectal cancers may begin as adenomas or polyps and later develop into adenocarcinomas; bronchial adenomas can develop into lung cancer.

Frequently, adenomas have a noticeable effect on the organs or gland tissue in which they develop. Often, adenomas secrete excess levels of hormones. When this occurs, the effects can be quite uncomfortable for the affected individual. In certain situations, the effects can be life threatening. However, some adenomas develop without any demonstrable effects.

There are certain types of adenomas that are more common in women, such as adenomas of the liver. Others, such as colon adenomas, are most common in adults of advancing age, for example, over fifty. In addition, there are factors that predispose a patient to the development of an adenoma. For example, women who use oral contraceptives may be at increased risk of developing liver adenomas. Certain types of adenomas, for example, colon adenomas, may be inheritable.

Symptoms related to adenomas vary widely. For example, a breast adenoma, called a fibroadenoma, typically causes no symptoms and may be so small that the affected individual is unable to detect it. Other breast adenomas, however, may be large enough to be noticeable by touch. By contrast, a lung adenoma can cause fever, chills, shortness of breath, and a bloody cough.

Adenomas are diagnosed using a variety of techniques, including the collection of blood and urine samples, ultrasound imaging, computed tomography (CT) scanning, and magnetic resonance imaging (MRI). Biopsies are typically employed to determine whether the tumor is benign or malignant. The methods of the invention are particularly useful for the treatment of adenomas, the treatment or prevention of adenoma recurrence, for example in a subject having a dormant tumor or micrometastases, or for the prevention of adenomas in a subject having any of the risk factors associated with adenomas.

### C. Gastrointestinal tumors

In another embodiment, the methods of the invention are used for the treatment of a benign, pre-cancerous, non-metastatic, or dormant gastrointestinal tumor, or micrometastases from a gastrointestinal tumor. This includes any stage 0, I, or II tumor; any tumor or condition that typically precedes or develops into a gastrointestinal cancer; and any gastrointestinal tumor that remains localized at the site of origin and has not infiltrated, invaded, or metastasized to distant sites. Included in gastrointestinal tumors is any polyp, adenoma, tumor, or cancer of the digestive system, specifically the esophagus, stomach, liver, biliary tract (gallbladder, bile ducts, ampulla of vater), intestines, pancreas, colon, rectum, and anus. These are described in detail below.

### (i) Anal cancer

Anal cancer is a malignant tumor of either the anal canal or anal verge. Anal cancers frequently start as anal dysplasia. Anal dysplasia is made up of cells of the anus that have abnormal changes, but do not show evidence of invasion into the surrounding tissue. The most severe form of anal dysplasia is called carcinoma in situ where the cells appear like cancer cells, but have not invaded beyond where the normal cells lie. Over time, anal dysplasia eventually changes to the point where the cells become invasive and gain the ability to metastasize, or break way to other parts of the body. Anal dysplasia is sometimes referred to as anal intraepithelial neoplasia (AIN). When anal cancer does spread, it is usually through direct invasion into the surrounding tissue or through the lymphatic system. Spread of anal cancer through the blood is less common, although it can occur.

Several factors have been associated with anal cancer. Most importantly, infection with the human papilloma virus (HPV) has been shown to be related to anal cancers and has been associated with several other cancers including cervical cancer and cancers of the head and neck. Another sexually transmitted virus, the human immunodeficiency virus (HIV), has been linked to anal cancers, and individuals infected with HIV are at increased risk for infection with HPV. Because anal cancer appears to first start as anal dysplasia before progressing to anal cancer, patients with a history of AIN are at increased risk to develop anal cancer. In addition, there appears to be an increased rate of anal cancer in patients who have benign anal conditions such as anal fistulae, anal fissures, perianal abscesses, or hemorrhoids.

The methods of the invention are particularly useful for the treatment of early stage anal cancer, the treatment or prevention of anal cancer recurrence, for example in a subject having a dormant tumor or micrometastases, or for the prevention of anal cancer in a subject having any of the risk factors associated with anal cancer.

### (ii) Colorectal and rectal cancers

The colon is the longest portion of the large intestine, also known as the large bowel. Colon cancer is the third most common type of cancer, in both males and females, in the Western world. The incidence is highest in African Americans, who are also more likely to die of the disease. The risk of colon cancer rises substantially after age 50, but every year there are numerous cases reported in younger people. In general, colon and rectal cancers are grouped together and have the same risk factors associated with them. Individuals with a personal or family history of colon cancer, polyps, or inherited colon cancer syndromes (e.g., FAP and HNPCC), as well as patients with ulcerative colitis or Crohn's disease, are all at higher risk and may require screening at an earlier age than the general population. A person with one first degree relative (parent, sibling or child) with colon cancer is 2 to 3 times as likely to develop the cancer as someone who does not have an affected relative.

Colon cancers can be diagnosed using a variety of techniques known to the clinician. Screening tests are the most effective method of diagnosing colon cancer in the early stages (e.g., polyps or adenomas) as these stages often are not associated with any symptoms. It is generally as the polyp grows into a tumor that it may bleed or obstruct the colon causing symptoms. These symptoms include bleeding from the rectum, blood in the stool or toilet after a bowel movement, a change in the shape of the stool (i.e., thinning), cramping pain in the abdomen, and feeling the need to have a bowel movement at times when a bowel movement is not needed. For tumors and polyps that may bleed intermittently, the blood can be detected in stool samples by a test called fecal occult blood testing (FOBT). By itself, FOBT only finds about 24% of cancers. A flexible sigmoidoscopy or a colonoscopy can also be used to diagnose colorectal cancers.

Generally, colorectal cancer is staged as follows:
Stage 0 (also called carcinoma in situ) - the cancer is confined to the outermost portion of the colon wall.
Stage I - the cancer has spread to the second and third layer of the colon wall, but not to the outer colon wall or beyond. This is also called Dukes' A colon cancer.
Stage II - the cancer has spread through the colon wall, but has not invaded any lymph nodes (these are small structures that help in fighting infection and disease). This is also called Dukes' B colon cancer.
Stage III - the cancer has spread through the colon wall and into lymph nodes, but has not spread to other areas of the body. This is also called Dukes' C colon cancer.
Stage IV - the cancer has spread to other areas of the body (i.e. liver and lungs). This is also called Dukes' D colon cancer.

The methods of the invention are particularly useful for the treatment of early stage colorectal cancer, the treatment or prevention of colorectal cancer recurrence, for example in a subject having a dormant tumor or micrometastases, or for the prevention of colorectal cancer in a subject having any of the risk factors colorectal cancer, such as those described above.

### (iii) Esophageal cancer

The esophagus is a muscular tube that connects the throat to the stomach. The vast majority of esophageal cancers develop from the inner lining (mucosa) of the esophagus and not from the muscle or cartilage cells that make up the rest of the esophagus. The lining of the esophagus is somewhat unique in that it changes as it goes from the throat to the stomach. In the upper (proximal) esophagus, the lining of the esophagus resembles the lining of the throat, made up of squamous cells. Hence, when cancers develop in this region, they are usually squamous cell carcinomas. In the lower (distal) esophagus, the more common type of cancer is called adenocarcinoma.

In addition to invasive cancers, patients are sometimes diagnosed with precancerous lesions, called carcinoma in situ. These precancerous lesions can be seen prior to the development of either squamous cell carcinoma or adenocarcinoma. Carcinoma in situ occurs when the lining of the esophagus undergoes changes similar to cancerous changes without any invasion into the deeper tissues. Hence, while the cells themselves have cancer-like qualities, there is no risk of spread, as no invasion has occurred. Another type of lesion that is considered to be a precursor to cancer itself is called Barrett's esophagus, which is explained in depth below.

Esophageal cancer occurs in approximately 13,500 Americans per year, causing about 12,500 deaths. Most patients are diagnosed in their 50s or 60s, with approximately four times as many men diagnosed than women. In the past, the vast majority (∼85%) of the esophageal cancers diagnosed were squamous cell cancers that occurred in the upper esophagus. Risk factors for this type of cancer include smoking and alcohol use. Although both are thought to be independent risk factors (with smoking being the stronger), there seems to be a synergistic effect between the two for the development of esophageal cancer. Other potential carcinogens for the development of squamous cell carcinoma of the esophagus are nitrosamines, asbestos fibers, and petroleum products.

This is contrasted with the group of patients at risk for adenocarcinoma, usually of the lower esophagus. Adenocarcinoma was previously a less common disease when compared to squamous cell carcinoma. However, it has recently become even more prevalent than squamous cell carcinoma. Adenocarcinoma is thought generally to arise in the setting of Barrett's esophagus, which is a condition in which the normal lining of the esophagus is replaced by lining resembling the stomach. Barrett's esophagus is diagnosed by endoscopy, in which a fiberoptic camera is used to look down into the esophagus and to biopsy any suspicious areas. Barrett's esophagus is thought to be caused by the chronic exposure of the lower esophagus to gastric acid. This exposure happens in patients with gastro-esophageal reflux disease (GERD), which causes patients symptoms of heartburn, bloating, loss of appetite, or stomach pains with food or at night while sleeping. Patients with chronic GERD are at risk for developing Barrett's esophagus and hence are at higher risk for developing adenocarcinoma of the esophagus.

Although Barrett's esophagus, by definition, occurs when the lining of the esophagus is abnormal, there can be varied levels of the degree of the abnormalities. This is graded in terms of dysplasia, which is used to determine how likely the Barrett's esophagus is to progress to cancer. Patients with Barrett's esophagus with high grade dysplasia should be followed by endoscopy every 3 months or actually undergo treatment, as these are considered premalignant changes that have a high likelihood of progressing to cancer. The most sensitive test to document local esophageal cancer or dysplasia is endoscopy. With endoscopy, the area of concern in the esophagus can be viewed directly with the fiber-optic camera, and the location of the abnormality, the presence or absence of bleeding, and the amount of obstruction can be visualized. Performance of a laryngoscopy (looking at the throat) or a bronchoscopy (looking at the trachea and airways) may also be required depending on the location and extent of the esophageal cancer. The standard of care today also includes performing an ultrasound during the endoscopy, called an endoscopic ultrasound examination (EUS). A CT scan, a barium swallow test, an x-ray, and other, more routine tests, including blood screening tests, are typically performed to properly diagnose and stage the cancer.

The methods of the invention are particularly useful for the treatment of early stage esophageal cancer, the treatment or prevention of esophageal cancer recurrence, for example in a subject having a dormant tumor or micrometastases, or for the prevention of esophageal cancer in a subject having any of the risk factors associated with esophageal cancer, such as those described above.

### (iv) Gall bladder cancer

The gall bladder is a small pear-shaped organ that stores and concentrates bile. The gallbladder and liver are connected by the hepatic duct. Primary cancer of the gallbladder affects about 6000 adults in the US each year. The majority of these cancers are adenocarcinomas, with subtypes such as papillary, nodular, and tubular, depending on the appearance of the tumor cells under the microscope. Less common subtypes include squamous cell, signet ring cell, and adenosquamous (adenoacanthoma).

Gallbladder cancer is most often seen in older patients, with a median age at diagnosis of 62-66 years. It occurs more often in females, with a female-to-male ratio of about 3:1.

The cause of gallbladder cancer is unknown, although it has been associated with gallstones, high estrogen levels, cigarette smoking, alcohol, obesity, and the female gender. Also, patients with inflammatory bowel disease (ulcerative colitis and Crohn's disease) are 10 times more likely to develop cancer of the extrahepatic biliary tract.

In general, gall bladder cancer is diagnosed thorough history and physical examination and laboratory work that includes metabolic chemistry and liver function panels to look for abnormal levels of various substances in the blood that are suggestive of general hepatobiliary disease. A urinalysis is usually done to evaluate urinary levels of some of these substances as well. Additional techniques such as ultrasound, MRI, cholangiography, and CT scans can also be used.

The methods of the invention are particularly useful for the treatment of early stage gall bladder cancer, the treatment or prevention of gall bladder cancer recurrence, for example in a subject having a dormant tumor or micrometastases, or for the prevention of gall bladder cancer in a subject having any of the risk factors associated with gall bladder cancer, such as those described above.

### (v) Gastric Cancers

Gastric cancer is cancer of the stomach. In the United States, gastric cancer now ranks as the 14^{th} most common cancer. It is rare to see gastric cancer before the age of 40, and its incidence increases with age thereafter.

Over 90% of gastric cancers arise from the lining of the stomach. Since this lining has glands, the cancer that comes from it is called an adenoma or, for more advanced forms, an adenocarcinoma. Although there are other cancers that can arise in the stomach (lymphomas-from lymph tissue, leiomyosarcoma-from muscle tissue, squamous cell carcinoma-from lining without glands), the vast majority are adenocarcinomas.

Studies have also linked infection with *Helicobacter pylori* with gastric cancer. *H. pylori* is associated with gastric ulcers and chronic atrophic gastritis, which may explain the high incidence of gastric cancer in patients infected with *H. pylori.* However, the exact role of *H. pylori* in the development of gastric cancer remains unclear.

A variety of tests are used to accurately identify gastric cancers, including double-contrast barium radiographs (so-call "upper GIs" or "barium swallows") and upper endoscopies. Other procedures including CT scans, PET scans, and laparoscopy are used for the diagnosis of gastric cancer.

The methods of the invention are particularly useful for the treatment of early stage gastric cancer, the treatment or prevention of gastric cancer recurrence, for example in a subject having a dormant tumor or micrometastases, or for the prevention of gastric cancer in a subject having any of the risk factors associated with gastric cancer, such as those described above.

### (vi) Liver cancer

There are a number of benign liver tumors. Hemangiomas are the most common benign tumor of the liver and occur when a benign, blood-filled tumor forms within the liver. Other benign tumors include adenomas and focal nodular hyperplasia. Although these tumors do not invade surrounding tissues or metastasize, it is often difficult to tell the difference between benign and malignant tumors on radiographic imaging.

Hepatocellular carcinoma (HCC), a cancer arising from the hepatocytes, is the most common type of primary liver cancer and accounts for around 70% of all liver cancers. Cancers that arise from the bile ducts within the liver are known as cholangiocarcinomas and represent 10-20% of all liver cancers. These cancers can arise from the bile ducts within the liver (known as intrahepatic cholangiocarcinomas) or from within the bile ducts as they lead away from the liver (known as extrahepatic cholangiocarcinomas). Other types of rare cancers can occur within the liver. These include hemangiosarcomas (malignant blood-filled tumors) and hepatoblastoma (a rare cancer that develops in very young children).

There are a number of risk factors that are associated with liver cancer. In the United States, the most common risk factor for liver cancer is liver cirrhosis. Chronic infection with hepatitis C virus (HCV) is also a common cause of liver cancer in the United States. Worldwide, other risk factors, such as chronic infection with hepatitis B virus (HBV) and aflatoxin B1 food contamination are more common.

There are several screening tests that are used to detect liver cancer. One potential screening test involves detection of blood levels of alpha-fetoprotein (AFP). AFP is a protein that is found at high levels in fetal blood, but normally disappears after birth. AFP levels increase in the presence of HCC and can be a marker of the development of liver cancer. While some patients who are at high risk for developing liver cancer are routinely tested for AFP levels, not all liver cancers produce high levels of AFP in the blood, and by the time most patients are found to have high AFP levels, the tumor is already at an advanced stage. Other blood proteins may potentially be used as screening tools for liver cancer. Several studies have shown the use of proteins such as des-gamma-carboxy prothrombin (DCP) and Lens culinaris agalutinin-reactive fraction (AFP-L3) may also be used as markers of liver cancer formation; however, in practice, these are infrequently used.

In addition, when liver cancer is suspected, ultrasound, CT scans, MRI, angiography, fluorodexoyglucose-positron emission tomography (FDG-PET), biopsy, and exploratory laporatomy are performed to further diagnose and stage the liver cancer.

The methods of the invention are particularly useful for the treatment of early stage liver cancer, the treatment or prevention of liver cancer recurrence, for example in a subject having a dormant tumor or micrometastases, or for the prevention of liver cancer in a subject having any of the risk factors associated with liver cancer, such as those described above.

### (vii) Pancreatic cancer

The pancreas is a pear-shaped gland, about six inches in length, located deep within the abdomen, between the stomach and the spine. It is referred to in three parts: the widest part is called the head, the middle section is the body, and the thin end is called the tail. The pancreas is responsible for making hormones, including insulin, which help regulate blood sugar levels, and enzymes, which are used by the bowel for the digestion of food. These enzymes are transported through ducts within the pancreas, emptied into the common bile duct, which carries the enzymes into the bowel. The incidence of pancreatic cancer is highest between 60 and 80 years of age, and is only rarely seen in people under 40. It is seen about equally in men and women, although the rates in women have risen in recent years, which may be due to higher rates of smoking in women. Cigarette smokers are two to three times more likely to develop pancreatic cancer. A person's risk triples if their mother, father, or siblings have had the disease. A family history of breast or colon cancer also increases risk. This increased risk is due to inherited mutations in cancer causing genes. The actual cause of this disease is not known, but is thought to be a result of a combination of inherited genetic changes and changes caused by environmental exposures.

When a physician suspects that a patient may have pancreatic cancer, ultrasound, a CT scan, and endoscopy are used to diagnose and stage the cancer. Some patients with pancreatic cancer may have an elevated level of carbohydrate antigen 19-9 (CA 19-9). In patients who have an elevated level, it is useful in confirming a diagnosis in conjunction with other tests and for monitoring the disease during treatment. The level can be periodically checked during treatment to see if the cancer is stable or worsening.

The methods of the invention are particularly useful for the treatment of early stage pancreatic cancer, the treatment or prevention of pancreatic cancer recurrence, for example in a subject having a dormant tumor or micrometastases, or for the prevention of pancreatic cancer in a subject having any of the risk factors associated with pancreatic cancer, such as those described above.

### (viii) Small intestine cancer

The small bowel, also known as the small intestine, is the portion of the digestive tract that connects the stomach and the large bowel, also called the colon. There are three distinct parts of the small bowel: 1) the duodenum, 2) the jejunum and 3) the ileum. Surprisingly, despite the amazingly long length of the small bowel compared to the rest of the digestive tract, cancer of the small bowel is very rare. This includes either cancers starting in the bowel or cancers spreading there from another body site. Specifically, small bowel cancers represent less than 5% of all bowel cancers and about 0.5% of all cancers diagnosed in the U.S.

The cause of most small bowel cancers is unknown. There are, however, some possible risk factors that may increase the chance of developing small bowel cancer. Some examples include Crohn's disease, celiac sprue disease, Peutz-Jegher's syndrome, and intestinal polyposis.

There are four main types of small bowel cancer, depending on the appearance under the microscope and the cell of origin. Adenocarcinoma is the most common type. It typically starts in the lining or inside layer of the bowel, and usually occurs in the duodenum. Another type is sarcoma and the typical subtype is leiomyosarcoma, which starts in the muscle wall of the small bowel and usually occurs in the ileum. The third type is carcinoid, which starts in the special hormone-making cells of the small bowel and usually occurs in the ileum, sometimes in the appendix (which is the first part of the large bowel). The fourth type is lymphoma, which starts in the lymph tissue of the small bowel and usually occurs in the jejunum. The most typical subtype of lymphoma is non-Hodgkin's lymphoma. An uncommon subtype of small bowel cancer is gastrointestinal stromal tumor, which can occur in any of the three parts of the small bowel.

Cancer of the small intestine is usually diagnosed using a complete medical history, a physical examination and a stool sample, endoscopy or colonoscopy, barium C-rays, CT scans, ultrasound, or other x-rays.

The methods of the invention are particularly useful for the treatment of early stage cancer of the small intestine, the treatment or prevention of cancer of the small intestine recurrence, for example in a subject having a dormant tumor or micrometastases, or for the prevention of cancer of the small intestine in a subject having any of the risk factors associated with cancer of the small intestine, such as those described above.

### V. Prevention

In a further aspect of the invention, we have discovered that VEGF-specific antagonists can be used for the treatment of benign, pre-cancerous, or early stage cancers, or for the treatment or prevention of tumor recurrence. The methods can be used to treat the cancer itself or to prevent progression of the cancer to a metastatic or invasive stage or to a higher grade or stage. For example, the methods of the invention can be used to treat a subject with Stage 0 cancer or polyps in order to prevent progression to a Stage I or higher stage tumor. Similarly, in a patient having Stage II cancer, the methods can be used to prevent progression of the cancer to a Stage III or Stage IV cancer.

VEGF-specific antagonists can also be used to prevent the recurrence of a tumor. For example, if a tumor has been identified and treated (e.g., with chemotherapy or surgically removed), VEGF-specific antagonists can be used to prevent the recurrence of the colorectal tumor either locally or a metastasis of the colorectal tumor. For the prevention of the recurrence of the tumor, the VEGF-specific antagonists can be used, for example, to treat a dormant tumor or micrometastases, or to prevent the growth or re-growth of a dormant tumor or micrometastases, which may or may not be clinically detectable.

We have also discovered that VEGF-specific antagonists can be used for the prevention of cancer in a subject who has never had cancer or who is at risk for developing a cancer. There are a variety of risk factors known to be associated with cancer and many of them are described above. Exemplary risk factors include advancing age (i.e., over the age of fifty), a family history of cancer, viral infection with HPV, HIV, HBV, and HCV, oral contraceptive use, cirrhosis, ulcerative colitis, Barrett's esophagus, H. pylori infection, and the presence of polyps or dysplasia. In addition, a subject known to have an inherited cancer syndrome is considered to be at risk for developing a cancer. Non-limiting examples of such syndromes include APC, HNPCC, Gardner's syndrome, and MEN1. Additional risk factors for developing cancers can be determined upon clinical evaluation and include elevated levels of hormones or blood proteins such as PSA (prostate cancer) CA-125 (ovarian cancer), AFP (liver cancer), DCP (liver cancer), and CA 19-9 (pancreatic cancer).

### VI. Neoadjuvant Therapy

The invention provides a method of neoadjuvant therapy prior to the surgical removal of operable cancer in a subject, e.g., a human patient, comprising administering to the patient (e.g., where the patient has been diagnosed with a tumor and/or cancer) an effective amount of a VEGF-specific antagonist, e.g., a VEGF antibody. Optionally, the VEGF-specific antagonist is administered in combination with at least one chemotherapeutic agent. The additional step of administering to the subject an effective amount of a VEGF-specific antagonist after surgery to prevent recurrence of the cancer can also be employed with the neoadjuvant therapies described herein. For the methods that include the additional step of administering to the subject an effective amount of a VEGF-specific antagonist after surgery, any of the adjuvant methods described herein can be used.

For example, one method includes treating cancer in a subject comprising the following steps: a) a first stage comprising a plurality of treatment cycles wherein each cycle comprises administering to the subject an effective amount of a VEGF-specific antagonist, e.g., bevacizumab, and, optionally, at least one chemotherapeutic agent at a predetermined interval; b) a definitive surgery whereby the cancer is removed; and, optionally, c) a second stage comprising a plurality of maintenance cycles wherein each cycle comprises administering to the subject an effective amount of a VEGF-specific antagonist, e.g., bevacizumab, with or without any chemotherapeutic agent at a predetermined interval.

For neoadjuvant therapy, the VEGF-specific antagonist can be administered in an amount or for a time (e.g., for a particular therapeutic regimen over time) to reduce (e.g., by 20%, 30%, 40%, 50%, 60%, 70%. 80%, 90%, 100% or more) the number of cancer cells in the tumor; to reduce the size of the tumor (e.g., to allow resection); to reduce the tumor burden; to inhibit (i.e., to decrease to some extent and/or stop) cancer cell infiltration into peripheral organs; to reduce vessel density in the tumor; to inhibit tumor metastasis; to reduce or inhibit tumor growth or tumor cell proliferation; to reduce or prevent the growth of a dormant tumor; to reduce or prevent the growth or proliferation of a micrometastases; to increase or extend the DFS or OS of a subject susceptible to or diagnosed with a benign, precancerous, or non-metastatic tumor; and/or to relieve to some extent one or more of the symptoms associated with the cancer.

In one example, the neoadjuvant therapy is administered to extend DFS or OS, wherein the DFS or the OS is evaluated about 2 to 5 years after an initial administration of the antibody. In certain embodiments, the subject's DFS or OS is evaluated about 3-5 years, about 4-5 years, or at least about 4, or at least about 5 years after initiation of treatment or after initial diagnosis. Typically, the VEGF-specific antagonist is a VEGF antibody such as bevacizumab.

In another example, administration of the antibody and/or chemotherapy can decrease disease recurrence (cancer recurrence in the primary organ and/or distant recurrence), in a population of subjects by about 50% at 3 years (where "about 50%" herein, includes a range from about 45% to about 70%), for example decreases recurrence in the primary organ by about 52% at 3 years, and/or decreases distant recurrence by about 53% at 3 years, compared to subjects treated with chemotherapy (e.g. taxoid, such as paclitaxel) alone.

The VEGF-specific antagonist, e.g., a VEGF antibody, is administered to a subject, e.g., a human patient, in accord with known methods, such as intravenous administration, *e.g.,* as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred.

While the VEGF-specific antagonist, e.g., a VEGF antibody, may be administered as single a gent, the patient is optionally treated with a combination of the VEGF antibody, and one or more chemotherapeutic agent(s). In one embodiment, at least one of the chemotherapeutic agents is a taxoid. The combined administration includes coadministration or concurrent administration, using separate formulations or a single pharmaceutical formulation, and consecutive administration in either order, wherein preferably there is a time period while both (or all) active agents simultaneously exert their biological activities. Thus, the chemotherapeutic agent may be administered prior to, or following, administration of the VEGF-specific antagonist, e.g., VEGF antibody. In this embodiment, the timing between at least one administration of the chemotherapeutic agent and at least one administration of the VEGF-specific antagonist, e.g., a VEGF antibody, is preferably approximately 1 month or less and most preferably approximately 2 weeks or less. Alternatively, the chemotherapeutic agent and the VEGF-specific antagonist, e.g., a VEGF antibody are administered concurrently to the patient, in a single formulation or separate formulations. Treatment with the combination of the chemotherapeutic agent (e.g. taxoid) and the VEGF antibody (e.g. bevacizumab) may result in a synergistic, or greater than additive, therapeutic benefit to the patient.

The chemotherapeutic agent, if administered, is usually administered at dosages known therefor, or optionally lowered due to combined action of the drugs or negative side effects attributable to administration of the antimetabolite chemotherapeutic agent. Preparation and dosing schedules for such chemotherapeutic agents may be used according to manufacturers' instructions or as determined empirically by the skilled practitioner. Where the chemotherapeutic agent is paclitaxel, preferably, it is administered every week *(e.g.* at 80mg/m²) or every 3 weeks (for example at 175mg/m² or 135mg/m²). Suitable docetaxel dosages include 60mg/m², 70mg/m², 75mg/m², 100mg/m² (every 3 weeks); or 35mg/m² or 40mg/m² (every week).

Various chemotherapeutic agents that can be combined are disclosed above. In certain embodiments of the invention, the chemotherapeutic agents to be combined with the VEGF-specific antagonist, e.g., a VEGF antibody, include, but are not limited to, e.g., a taxoid (including docetaxel and paclitaxel), vinca (such as vinorelbine or vinblastine), platinum compound (such as carboplatin or cisplatin), aromatase inhibitor (such as letrozole, anastrazole, or exemestane), anti-estrogen (e.g. fulvestrant or tamoxifen), etoposide, thiotepa, cyclophosphamide, methotrexate, liposomal doxorubicin, pegylated liposomal doxorubicin, capecitabine, gemcitabine, COX-2 inhibitor (for instance, celecoxib), or proteosome inhibitor (e.g. PS342).

Where an anthracycline (e.g. doxorubicin or epirubicin) is administered to the subject, preferably this is given prior to and/or following administration of the VEGF-specific antagonist, e.g., bevacizumab. However, a modified anthracycline, such as liposomal doxorubicin (TLC D-99 (MYOCET®), pegylated liposomal doxorubicin (CAELYX®), or epirubicin, with reduced cardiac toxicity, may be combined with the VEGF-specific antagonist, e.g., bevacizumab.

In one embodiment of an administration schedule, the neoadjuvant therapy of the invention comprises a first step wherein a VEGF-specific antagonist, e.g*.,* bevacizumab, and one or more chemotherapeutic agents are administered to the patients in a plurality of neoadjuvant cycles, followed by a surgery to definitively remove the tumor. Each neoadjuvant cycle consists of one to three weeks, depending on the particular treatment plan. For example, a treatment cycle can be three weeks, which means patients receive one dose of chemotherapy and one dose of bevacizumab every three weeks. A treatment cycle can also be two weeks, which means patients receive one dose of chemotherapy and one dose of bevacizumab every other week. The entire first stage of neoadjuvant treatment can last for about 4-8 cycles. In certain embodiments of the invention, the neoadjuvant therapy lasts for less than one year, in one embodiment, less than six months prior to surgery. Depending on the type and severity of the disease, preferred dosages for the VEGF-specific antagonist, e.g., bevacizumab, are in the range from about 1 µg/kg to about 50mg/kg, most preferably from about 5mg/kg to about 15mg/kg, including but not limited to 7.5 mg/kg or 10 mg/kg. In some aspects, the chemotherapy regimen involves the traditional high-dose intermittent administration. In some other aspects, the chemotherapeutic agents are administered using smaller and more frequent doses without scheduled breaks ("metronomic chemotherapy"). The progress of the therapy of the invention is easily monitored by conventional techniques and assays.

Aside from the VEGF antibody and the chemotherapeutic agent, other therapeutic regimens may be combined therewith. For example, a second (third, fourth, etc) chemotherapeutic agent(s) may be administered, wherein the second chemotherapeutic agent is either another, different taxoid chemotherapeutic agent, or a chemotherapeutic agent that is not a taxoid. For example, the second chemotherapeutic agent may be a taxoid (such as paclitaxel or docetaxel), a vinca (such as vinorelbine), a platinum compound (such as cisplatin or carboplatin), an anti-hormonal agent (such as an aromatase inhibitor or antiestrogen), gemcitabine, capecitabine, etc. Exemplary combinations include taxoid/platinum compound, gemcitabine/taxoid, gemcitabine/vinorelbine, vinorelbine/taxoid, capecitabine/taxoid, etc. "Cocktails" of different chemotherapeutic agents may be administered.

Other therapeutic agents that may be combined with the VEGF antibody include any one or more of: another VEGF antagonist or a VEGF receptor antagonist such as a second anti-VEGF antibody, VEGF variants, soluble VEGF receptor fragments, aptamers capable of blocking VEGF or VEGFR, neutralizing anti-VEGFR antibodies, inhibitors of VEGFR tyrosine kinases and any combinations thereof. Other therapeutic agents useful for combination tumor therapy with the antibody of the invention include antagonist of other factors that are involved in tumor growth, such as EGFR, ErbB2 (also known as Her2) ErbB3, ErbB4, or TNF. In one exemplary embodiment, the composition for theVEGF-specific antagonist does not include an anti-ErbB2 antibody, or fragment or derivative thereof (e.g., the Herceptin® antibody). In certain embodiments of the invention, the anti-VEGF antibody can be used in combination with small molecule receptor tyrosine kinase inhibitors (RTKIs) that target one or more tyrosine kinase receptors such as VEGF receptors, FGF receptors, EGF receptors and PDGF receptors. Many therapeutic small molecule RTKIs are known in the art, including, but are not limited to, vatalanib (PTK787), erlotinib (TARCEVA®), OSI-7904, ZD6474 (ZACTIMA^{®}), ZD6126 (ANG453), ZD1839, sunitinib (SUTENT^{®}), semaxanib (SU5416), AMG706, AG013736, Imatinib (GLEEVEC^{®}), MLN-518, CEP-701, PKC- 412, Lapatinib (GSK572016), VELCADE^{®}, AZD2171, sorafenib (NEXAVAR^{®}), XL880, and CHIR-265.

Suitable dosages for any of the above coadministered agents are those presently used and may be lowered due to the combined action (synergy) of the agent and VEGF antibody.

In addition to the above therapeutic regimes, the patient may be subjected to radiation therapy.

In certain embodiments of the invention, the administered VEGF antibody is an intact, naked antibody. However, the VEGF antibody may be conjugated with a cytotoxic agent. In certain embodiments, the conjugated antibody and/or antigen to which it is bound is/are internalized by the cell, resulting in increased therapeutic efficacy of the conjugate in killing the cancer cell to which it binds. In one embodiment, the cytotoxic agent targets or interferes with nucleic acid in the cancer cell. Examples of such cytotoxic agents include maytansinoids, calicheamicins, ribonucleases and DNA endonucleases.

### VII. Adjuvant Therapy

The invention provides a method of adjuvant therapy comprising administering a VEGF-specific antagonist, e.g., a VEGF antibody, to a subject with nonmetastatic cancer, following definitive surgery.

For example, a method can include following steps: a) a first stage comprising a plurality of treatment cycles wherein each cycle comprises administering to the subject an effective amount of a VEGF-specific antagonist, e.g., bevacizumab, and optionally, at least one chemotherapeutic agent at a predetermined interval; and b) a second stage comprising a plurality of maintenance cycles wherein each cycle comprises administering to the subject an effective amount of a VEGF-specific antagonist, e.g., bevacizumab, without any chemotherapeutic agent at a predetermined interval; wherein the combined first and second stages last for at least one year after the initial postoperative treatment. In one embodiment, the first stage comprises a first plurality of treatment cycles wherein a VEGF-specific antagonist, e.g., bevacizumab, and a first chemotherapy regimen are administered, followed by a second plurality of treatment cycles wherein a VEGF-specific antagonist, *e.g.,* bevacizumab, and a second chemotherapy regimen are administered.

For adjuvant therapy, the VEGF-specific antagonist can be administered in an amount or for a time (e.g., for a particular therapeutic regimen over time) to reduce (e.g., by 20%, 30%, 40%, 50%, 60%, 70%, 80%, 90%, 100% or more) the number of cancer cells in the tumor; to reduce the size of the tumor; to reduce the tumor burden; to inhibit (i.e., to decrease to some extent and/or stop) cancer cell infiltration into peripheral organs; to reduce vessel density in the tumor; to inhibit tumor metastasis; to reduce or inhibit tumor growth or tumor cell proliferation; to reduce or prevent the growth of a dormant tumor; to reduce or prevent the growth or proliferation of a micrometastases; to reduce or prevent the re-growth of a tumor after treatment or removal; and/or to relieve to some extent one or more of the symptoms associated with the cancer. In some additional embodiments, the VEGF-specific antagonist can be used to prevent the occurrence or reccurrence of cancer in the subject. In one example, prevention of cancer recurrence is evaluated in a population of subjects after about four years to confirm no disease recurrence has occurred in at least about 80% of the population. In another example, prevention of disease recurrence is evaluated at about 3 years, wherein disease recurrence is decreased by at least about 50% compared to subjects treated with chemotherapy alone.

The VEGF-specific antagonist is generally administered after a period of time in which the subject has recovered from the surgery. This period of time can include the period required for wound healing or healing of the surgical incision, the time period required to reduce the risk of wound dehiscence, or the time period required for the subject to return to a level of health essentially similar to or better than the level of health prior to the surgery. The period between the completion of the definitive surgery and the first administration of the VEGF-specific antagonist can also include the period needed for a drug holiday, wherein the subject requires or requests a period of time between therapeutic regimes. Generally, the time period between completion of definitive surgery and the commencement of the VEGF-specific antagonist therapy can include less than one week, 1 week, 2 weeks, 3 weeks, 4 weeks (28 days), 5 weeks, 6 weeks, 7 weeks, 8 weeks, 3 months, 4 months, 5 months, 6 months, 7 months, 8 months, 9 months, 10 months, 11 months, 1 year, 2 years, 3 years, or more. In one embodiment, the period of time between definitive surgery and administering the VEGF-specific antagonist is greater than 2 weeks and less than 1 year.

In one example, the VEGF-specific antagonist, *e.g.,* a VEGF antibody, is administered in an amount effective to extend disease free survival (DFS) or overall survival (OS), wherein the DFS or the OS is evaluated about 2 to 5 years after an initial administration of the antibody. In certain embodiments, the subject's DFS or OS is evaluated about 3-5 years, about 4-5 years, or at least about 4, or at least about 5 years after initiation of treatment or after initial diagnosis.

The VEGF-specific antagonist, e.g., a VEGF antibody, is administered to a subject, e.g., a human patient, in accord with known methods, such as intravenous administration, e.g., as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred.

The VEGF-specific antagonist may be administered as single agent. In other embodiments the patient is treated with a combination of the VEGF-specific antagonist, and one or more chemotherapeutic agent(s). In some embodiments, at least one of the chemotherapeutic agents is a taxoid. The combined administration includes coadministration or concurrent administration, using separate formulations or a single pharmaceutical formulation, and consecutive administration in either order, wherein optionally there is a time period while both (or all) active agents simultaneously exert their biological activities. Thus, the chemotherapeutic agent may be administered prior to, or following, administration of the VEGF-specific antagonist, e.g., a VEGF antibody. In this embodiment, the timing between at least one administration of the chemotherapeutic agent and at least one administration of the VEGF-specific antagonist, e.g., a VEGF antibody, is preferably approximately 1 month or less, and most preferably approximately 2 weeks or less. Alternatively, the chemotherapeutic agent and the VEGF antibody are administered concurrently to the patient, in a single formulation or separate formulations. Treatment with the combination of the chemotherapeutic agent (e.g. taxoid) and the VEGF antibody (e.g. bevacizumab) may result in a synergistic, or greater than additive, therapeutic benefit to the patient.

The chemotherapeutic agent, if administered, is usually administered at dosages known therefor, or optionally lowered due to combined action of the drugs or negative side effects attributable to administration of the antimetabolite chemotherapeutic agent. Preparation and dosing schedules for such chemotherapeutic agents may be used according to manufacturers' instructions or as determined empirically by the skilled practitioner. Where the chemotherapeutic agent is paclitaxel, preferably, it is administered every week (*e.g.* at 80mg/m²) or every 3 weeks (for example at 175mg/m² or 135mg/m²). Suitable docetaxel dosages include 60mg/m², 70mg/m², 75mg/m², 100mg/m² (every 3 weeks); or 35ma/m² or 40mg/m² (every week).

Various chemotherapeutic agents that can be combined are disclosed above. Examples of chemotherapeutic agents to be combined with the VEGF antibody include, but are not limited to, e.g., a taxoid (including docetaxel and paclitaxel), vinca (such as vinorelbine or vinblastine), platinum compound (such as carboplatin or cisplatin), aromatase inhibitor (such as letrozole, anastrazole, or exemestane), anti-estrogen (e.g. fulvestrant or tamoxifen), etoposide, thiotepa, cyclophosphamide, methotrexate, liposomal doxorubicin, pegylated liposomal doxorubicin, capecitabine, gemcitabine, COX-2 inhibitor (for instance, celecoxib), or proteosome inhibitor (*e*.*g*. PS342).

Where an anthracycline (e.g. doxorubicin or epirubicin) is administered to the subject, preferably this is given prior to and/or following administration of the VEGF antibody, such as in the protocols disclosed in the Example below where an anthracycline/cyclophosphomide combination was administered to the subject following surgery, but prior to administration of the VEGF antibody and taxoid. However, a modified anthracycline, such as liposomal doxorubicin (TLC D-99 (MYOCET®), pegylated liposomal doxorubicin (CAELYX®), or epirubicin, with reduced cardiac toxicity, may be combined with the VEGF antibody.

In one administration schedule, the adjuvant therapy of the invention comprises a first stage wherein a VEGF-specific antagonist, e.g., a VEGF antibody, and one or more chemotherapeutic agents are administered to the patients in a plurality of treatment cycles; and a second stage wherein a VEGF-specific antagonist, e.g., a VEGF antibody, is used as a single agent in a plurality of maintenance cycles. Each treatment cycle consists of one to three weeks, depending on the particular treatment plan. For example, a treatment cycle can include bevacizumab as the VEGF-specific antagonist and can be three weeks, which means patients receive one dose of chemotherapy and one dose of bevacizumab every three weeks. A treatment cycle can also be two weeks, which means patients receive one dose of chemotherapy and one dose of bevacizumab, every other week. The entire first stage of treatment can last for about 4-8 cycles. During the second, maintenance stage, bevacizumab is given biweekly or triweekly, depending on the length of the particular cycle, and for a total about 30-50 cycles. In certain embodiments, the adjuvant therapy lasts for at least one year from the initiation of the treatment, and the subject's progress will be followed after that time. Depending on the type and severity of the disease, preferred dosages for the VEGF antibody are in the range from about 1ug/kg to about 50mg/kg, most preferably from about 5mg/kg to about 15mg/kg, including but not limited to 7.5 mg/kg or 10 mg/kg. In some aspects, the chemotherapy regimen involves the traditional high-dose intermittent administration. In some other aspects, the chemotherapeutic agents are administered using smaller and more frequent doses without scheduled breaks ("metronomic chemotherapy"). The progress of the therapy of the invention is easily monitored by conventional techniques and assays.

Administration of the antibody and chemotherapy can decrease the likelihood of disease recurrence (cancer recurrence in the primary organ and/or distant recurrence), in a population of subjects by about 50% at 3 years (where "about 50%" herein, includes a range from about 45% to about 70%), for example decreases recurrence in the primary organ by about 52% at 3 years, and/or decreases distant recurrence by about 53% at 3 years, compared to subjects treated with chemotherapy (e.g. taxoid, such as paclitaxel) alone.

The invention herein provides a method of curing nonmetastatic cancer in a population of human subjects with nonmetastatic cancer comprising administering an effective amount of a VEGF-specific antagonist, e.g., a VEGF antibody, and at least one chemotherapeutic agent to the subjects following definitive surgery, and evaluating the subjects after four or more years to confirm no disease recurrence has occurred after about 4 years in at least about 80% (preferably at least about 85%) of the subjects. The population may comprise 3000 or more human subjects.

The invention further concerns a method of decreasing the likelihood of disease recurrence in a population of human subjects with nonmetastatic cancer comprising administering an effective amount of bevacizumab and at least one chemotherapeutic agent to the subjects following definitive surgery, wherein the likelihood of disease recurrence is decreased by at least about 50% at 3 years compared to subjects treated with taxoid alone.

Aside from the VEGF antibody and the chemotherapeutic agent, other therapeutic regimens may be combined therewith. For example, a second (third, fourth, etc) chemotherapeutic agent(s) may be administered, wherein the second chemotherapeutic agent is either another, different taxoid chemotherapeutic agent, or a chemotherapeutic agent that is not a taxoid. For example, the second chemotherapeutic agent may be a taxoid (such as paclitaxel or docetaxel), a vinca (such as vinorelbine), a platinum compound (such as cisplatin or carboplatin), an anti-hormonal agent (such as an aromatase inhibitor or antiestrogen), gemcitabine, capecitabine, etc. Exemplary combinations include taxoid/platinum compound, gemcitabine/taxoid, gemcitabine/vinorelbine, vinorelbine/taxoid, capecitabine/taxoid, etc. "Cocktails" of different chemotherapeutic agents may be administered.

Other therapeutic agents that may be combined with the VEGF antibody include any one or more of: another VEGF antagonist or a VEGF receptor antagonist such as a second anti-VEGF antibody, VEGF variants, soluble VEGF receptor fragments, aptamers capable of blocking VEGF or VEGFR, neutralizing anti-VEGFR antibodies, inhibitors of VEGFR tyrosine kinases and any combinations thereof. Other therapeutic agents useful for combination tumor therapy with the antibody of the invention include antagonist of other factors that are involved in tumor growth, such as EGFR, ErbB2 (also known as Her2) ErbB3, ErbB4, or TNF. In one exemplary embodiment, the composition for theVEGF-specific antagonist does not include an anti-ErbB2 antibody, or fragment or derivative thereof (e.g., the Herceptin® antibody). In certain embodiments, the anti-VEGF antibody can be used in combination with small molecule receptor tyrosine kinase inhibitors (RTKIs) that target one or more tyrosine kinase receptors such as VEGF receptors, FGF receptors, EGF receptors and PDGF receptors. Many therapeutic small molecule RTKIs are known in the art, including, but are not limited to, vatalanib (PTK787), erlotinib (TARCEVA^{®}), OSI-7904, ZD6474 (ZACTIMA^{®}), ZD6126 (ANG453), ZD1839, sunitinib (SUTENT^{®}), semaxanib (SU5416), AMG706, AG013736, Imatinib (GLEEVEC^{®}), MLN-518, CEP-701, PKC- 412, Lapatinib (GSK572016), VELCADE^{®}, AZD2171, sorafenib (NEXAVAR^{®}), XL880, and CHIR-265.

Suitable dosages for any of the above coadministered agents are those presently used and may be lowered due to the combined action (synergy) of the agent and VEGF antibody.

In addition to the above therapeutic regimes, the patient may be subjected to radiation therapy.

In certain embodiments, the administered VEGF antibody is an intact, naked antibody. However, the VEGF antibody may be conjugated with a cytotoxic agent. In certain embodiments, the conjugated antibody and/or antigen to which it is bound is/are internalized by the cell, resulting in increased therapeutic efficacy of the conjugate in killing the cancer cell to which it binds. In one embodiment, the cytotoxic agent targets or interferes with nucleic acid in the cancer cell. Examples of such cytotoxic agents include maytansinoids, calicheamicins, ribonucleases and DNA endonucleases.

### VIII. Dosages, Formulations, and Duration

The VEGF-specific antagonist composition will be formulated, dosed, and administered in a fashion consistent with good medical practice. Factors for consideration in this context include the particular disorder being treated, the particular subject being treated, the clinical condition of the individual patient, the cause of the disorder, the site of delivery of the agent, the method of administration, the scheduling of administration, and other factors known to medical practitioners. The "therapeutically effective amount" of the VEGF-specific antagonist to be administered will be governed by such considerations, and is the minimum amount necessary to prevent, ameliorate, or treat, or stabilize, a benign, precancerous, or early stage cancer; or to treat or prevent the occurrence or recurrence of a tumor, a dormant tumor, or a micrometastases, for example, in the neoadjuvant or adjuvant setting. The VEGF-specific antagonist need not be, but is optionally, formulated with one or more agents currently used to prevent or treat cancer or a risk of developing a cancer. The effective amount of such other agents depends on the amount of VEGF-specific antagonist present in the formulation, the type of disorder or treatment, and other factors discussed above. These are generally used in the same dosages and with administration routes as used hereinbefore or about from 1 to 99% of the heretofore employed dosages.

Depending on the type and severity of the disease, about 1 µg/kg to 100 mg/kg (e.g., 0.1-20 mg/kg) of VEGF-specific antagonist is an initial candidate dosage for administration to the patient, whether, for example, by one or more separate administrations, or by continuous infusion. A typical daily dosage might range from about 1 µg/kg to about 100 mg/kg or more, depending on the factors mentioned above. Particularly desirable dosages include, for example, 7.5 mg/kg. 10 mg/kg. and 15 mg/kg. For repeated administrations over several days or longer, depending on the condition, the treatment is sustained until the cancer is treated, as measured by the methods described above or known in the art. However, other dosage regimens may be useful. In one example, if the VEGF-specific antagonist is an antibody, the antibody of the invention is administered once every week, every two weeks, or every three weeks, at a dose range from about 5 mg/kg to about 15 mg/kg, including but not limited to 7.5 mg/kg or 10 mg/kg. The progress of the therapy of the invention is easily monitored by conventional techniques and assays.

In one example, bevacizumab is the VEGF-specific antagonist. Bevacizumab is supplied for therapeutic uses in 100 mg and 400 mg preservative-free, single-use vials to deliver 4 ml or 16 ml of bevacizumab (25 mg/ml). The 100 mg product is formulated in 240 mg α, α-trehalose dehydrate, 23.2 mg sodium phosphate (monobasic, monohydrate), 4.8 mg sodium phosphate (dibasic, anhydrous), 1.6 mg polysorbate 20, and Water for Injection, USP. The 400 mg product is formulated in 960 mg α, α-trehalose dehydrate, 92.8 mg sodium phosphate (monobasic, monohydrate), 19.2 mg sodium phosphate (dibasic, anhydrous), 6.4 mg polysorbate 20, and Water for Injection, USP.

The duration of therapy will continue for as long as medically indicated or until a desired therapeutic effect (e.g., those described herein) is achieved. In certain embodiments, the VEGF-specific antagonist therapy is continued for 2 months, 4 months, 6 months, 8 months, 10 months, 1 year, 2 years, 3 years, 4 years, 5 years, or for a period of years up to the lifetime of the subject.

Generally, alleviation or treatment of a benign, precancerous, or early stage cancer or the adjuvant or neoadjuvant therapy of a cancer (benign or malignant) involves the lessening of one or more symptoms or medical problems associated with the cancer. The therapeutically effective amount of the drug can accomplish one or a combination of the following to reduce (e.g., by 20%, 30%, 40%, 50%, 60%, 70%, 80%, 90%, 100% or more) the number of cancer cells in the tumor; to reduce the size of the tumor; to reduce the tumor burden; to inhibit (i.e., to decrease to some extent and/or stop) cancer cell infiltration into peripheral organs; to reduce vessel density in the tumor; to inhibit tumor metastasis; to reduce or inhibit tumor growth or tumor cell proliferation; to reduce or prevent the growth of a dormant tumor; to reduce or prevent the growth or proliferation of a micrometastases; to reduce or prevent the re-growth of a tumor after treatment or removal (e.g., in adjuvant therapy); to increase or extend the DFS or OS of a subject susceptible to or diagnosed with a benign, precancerous, or non-metastatic tumor or a malignant tumor; to reduce the size of a tumor to allow for surgery (e.g., in neoadjuvant therapy); and/or to relieve to some extent one or more of the symptoms associated with the cancer. In some additional embodiments, the VEGF-specific antagonist can be used to prevent the occurrence or reccurrence of cancer in the subject. In one example, prevention of cancer recurrence is evaluated in a population of subjects after about four years to confirm no disease recurrence has occurred in at least about 80% of the population. In another example, prevention of disease recurrence is evaluated at about 3 years, wherein disease recurrence is decreased by at least about 50% compared to subjects treated with chemotherapy alone.

In one example, the VEGF-specific antagonist, e.g., a VEGF antibody, is administered in an amount effective to extend DFS or OS, wherein the DFS or the OS is evaluated about 2 to 5 years after an initial administration of the antibody. In certain embodiments, the subject's DFS or OS is evaluated about 3-5 years, about 4-5 years, or at least about 4, or at least about 5 years after initiation of treatment or after initial diagnosis.

In one embodiment, the invention can be used for increasing the duration of survival of a subject susceptible to or diagnosed with a benign, precancerous, or non-metastatic tumor. Duration of survival is defined as the time from first administration of the drug to death. Duration of survival can also be measured by stratified hazard ratio (HR) of the treatment group versus control group, which represents the risk of death for a patient during the treatment.

In yet another embodiment, the treatment of the invention significantly increases response rate in a group of subjects, e.g., human patients, susceptible to or diagnosed with a cancer who are treated with various anti-cancer therapies. Response rate is defined as the percentage of treated patients who responded to the treatment. In one aspect, the combination treatment of the invention using a VEGF-specific antagonist and surgery, radiation therapy, or one or more chemotherapeutic agents significantly increases response rate in the treated patient group compared to the group treated with surgery, radiation therapy, or chemotherapy alone, the increase having a Chi-square p-value of less than 0.005.

Treatment or prevention of the occurrence or recurrence of a tumor, a dormant tumor, or a micrometastases involves the prevention of tumor or metastases formation, generally after initial treatment or removal of a tumor (e.g., using an anti-cancer therapy such as surgery, chemotherapy, or radiation therapy). Surgery can leave behind residual tumor cells, or dormant micro-metastatic nodules, which have the potential to re-activate the "angiogenic program" and facilitate more exponential tumor growth. Although the presence of a dormant tumor or micrometastases is not necessarily detectable using clinical measurements or screens, a therapeutically effective amount is one that is sufficient to prevent or reduce detection of the dormant tumor, micrometastases, metastases, or tumor recurrence using techniques known to the clinician. In one example, a subject who is treated for a tumor by surgically removing the tumor is then treated with a VEGF-specific antagonist and monitored over time for the detection of a dormant tumor, micrometastases, or tumor recurrence. The VEGF-specific antagonist can be administered in combination with another anti-cancer therapy (e.g., prior to, with, or after the VEGF-specific antagonist) and one or both therapies can be continued as a maintenance therapy.

Additional measurements of therapeutic efficacy in the treatment of cancers are described in U.S. Patent Application Publication No. 20050186208.

Therapeutic formulations are prepared using standard methods known in the art by mixing the active ingredient having the desired degree of purity with optional physiologically acceptable carriers, excipients or stabilizers (Remington's Pharmaceutical Sciences (20th edition), ed. A. Gennaro, 2000, Lippincott, Williams & Wilkins, Philadelphia, PA). Acceptable carriers, include saline, or buffers such as phosphate, citrate and other organic acids; antioxidants including ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagines, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, PLURONICS™, or PEG.

Optionally, but preferably, the formulation contains a pharmaceutically acceptable salt, typically, e.g., sodium chloride, and preferably at about physiological concentrations. Optionally, the formulations of the invention can contain a pharmaceutically acceptable preservative. In some embodiments the preservative concentration ranges from 0.1 to 2.0%, typically v/v. Suitable preservatives include those known in the pharmaceutical arts. Benzyl alcohol, phenol, m-cresol, methylparaben, and propylparaben are examples of preservatives. Optionally, the formulations of the invention can include a pharmaceutically acceptable surfactant at a concentration of 0.005 to 0.02%.

The formulation herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, *supra.*

Sustained-release preparations may be prepared. Suitable examples of sustained-release preparations include semipermeable matrices of solid hydrophobic polymers containing the antibody, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

The VEGF-specific antagonists of the invention are administered to a subject, e.g., a human patient, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Local administration is particularly desired if extensive side effects or toxicity is associated with VEGF antagonism. An *ex vivo* strategy can also be used for therapeutic applications. Ex vivo strategies involve transfecting or transducing cells obtained from the subject with a polynucleotide encoding a VEGF antagonist. The transfected or transduced cells are then returned to the subject. The cells can be any of a wide range of types including, without limitation, hematopoietic cells (e.g., bone marrow cells, macrophages, monocytes, dendritic cells, T cells, or B cells), fibroblasts, epithelial cells, endothelial cells, keratinocytes, or muscle cells.

For example, if the VEGF-specific antagonist is an antibody, the antibody is administered by any suitable means, including parenteral, subcutaneous, intraperitoneal, intrapulmonary, and intranasal, and, if desired for local immunosuppressive treatment, intralesional administration. Parenteral infusions include intramuscular, intravenous, intraarterial, intraperitoneal, or subcutaneous administration. In addition, the antibody is suitably administered by pulse infusion, particularly with declining doses of the antibody. Preferably the dosing is given by injections, most preferably intravenous or subcutaneous injections, depending in part on whether the administration is brief or chronic.

In another example, the VEGF-specific antagonist compound is administered locally, e.g., by direct injections, when the disorder or location of the tumor permits, and the injections can be repeated periodically. The VEGF-specific antagonist can also be delivered systemically to the subject or directly to the tumor cells, e.g., to a tumor or a tumor bed following surgical excision of the tumor, in order to prevent or reduce local recurrence or metastasis, for example of a dormant tumor or micrometastases.

Alternatively, an inhibitory nucleic acid molecule or polynucleotide containing a nucleic acid sequence encoding a VEGF-specific antagonist can be delivered to the appropriate cells in the subject. In certain embodiments, the nucleic acid can be directed to the tumor itself.

The nucleic acid can be introduced into the cells by any means appropriate for the vector employed. Many such methods are well known in the art (Sambrook et al., *supra,* and Watson et al., Recombinant DNA, Chapter 12, 2d edition, Scientific American Books, 1992). Examples of methods of gene delivery include liposome mediated transfection, electroporation, calcium phosphate/DEAE dextran methods, gene gun, and microinjection.

### IX. Combination Therapies

The invention also features the use of a combination of two or more VEGF-specific antagonists of the invention or the combination of at least one VEGF-specific antagonist with one or more additional anti-cancer therapies. Examples of anti-cancer therapies include, without limitation, surgery, radiation therapy (radiotherapy), biotherapy, immunotherapy, chemotherapy, or a combination of these therapies. In addition, cytotoxic agents, anti-angiogenic and anti-proliferative agents can be used in combination with the VEGF-specific antagonist.

In one example, the VEGF-specific antagonist is used as adjuvant therapy for the treatment of a nonmetastatic cancer following definitive surgery. In this example, the VEGF-specific antagonist can be provided with or without at least one additional chemotherapeutic agent.

In another example, the VEGF-specific antagonist is used as neoadjuvant therapy for the treatment of an operable cancer prior to surgery. In this example, the VEGF-specific antagonist can be provided prior to surgery with or without at least one additional chemotherapeutic agent.

In one example, the invention features the use of a VEGF-specific antagonist with one or more chemotherapeutic agents (e.g., a cocktail). Non-limiting examples of chemotherapeutic agents include irinotecan, fluorouracil, leucovorin, or any combination thereof. The combined administration includes simultaneous administration, using separate formulations or a single pharmaceutical formulation, and consecutive administration in either order, wherein preferably there is a time period while both (or all) active agents simultaneously exert their biological activities. Preparation and dosing schedules for such chemotherapeutic agents may be used according to manufacturers' instructions or as determined empirically by the skilled practitioner. Preparation and dosing schedules for chemotherapy are also described in Chemotherapy Service Ed., M. C. Perry, Williams & Wilkins, Baltimore, Md. (1992). The chemotherapeutic agent may precede, or follow administration of the VEGF-specific antagonist or may be given simultaneously therewith.

The formulation herein may also contain more than one active compound as necessary for the particular indication being treated, preferably those with complementary activities that do not adversely affect each other. For example, it may be desirable to further provide antibodies which bind to EGFR, VEGF (e.g. an antibody which binds a different epitope on VEGF), VEGFR, or ErbB2 (e.g., Herceptin®) in the one formulation. In one exemplary embodiment, the composition for theVEGF-specific antagonist does not include an anti-ErbB2 antibody, or fragment or derivative thereof (e.g., the Herceptin® antibody). Alternatively, or in addition, the composition may comprise a cytotoxic agent, cytokine, growth inhibitory agent and/or small molecule VEGFR antagonist. Such molecules are suitably present in combination in amounts that are effective for the purpose intended.

For the prevention or treatment of disease, the appropriate dosage of VEGF-specific antagonist will depend on the type of disease to be treated, as defined above, the severity and course of the disease, whether the VEGF-specific antagonist is administered for preventive or therapeutic purposes, previous therapy, the patient's clinical history and response to the VEGF-specific antagonist, and the discretion of the attending physician. The VEGF-specific antagonist is suitably administered to the patient at one time or over a series of treatments. In a combination therapy regimen, the VEGF-specific antagonist and the one or more anti-cancer therapeutic agent of the invention are administered in a therapeutically effective or synergistic amount. As used herein, a therapeutically effective amount is such that co-administration of a VEGF-specific antagonist and one or more other therapeutic agents, or administration of a composition of the invention, results in reduction or inhibition of the cancer as described above. A therapeutically synergistic amount is that amount of a VEGF-specific antagonist and one or more other therapeutic agents necessary to synergistically or significantly reduce or eliminate conditions or symptoms associated with a particular disease.

The VEGF-specific antagonist and the one or more other therapeutic agents can be administered simultaneously or sequentially in an amount and for a time sufficient to reduce or eliminate the occurrence or recurrence of a tumor, a dormant tumor, or a micrometastases. The VEGF-specific antagonist and the one or more other therapeutic agents can be administered as maintenance therapy to prevent or reduce the likelihood of recurrence of the tumor.

The VEGF-specific antagonist can be packaged alone or in combination with other anti-cancer therapeutic compounds as a kit. The kit can include optional components that aid in the administration of the unit dose to patients, such as vials for reconstituting powder forms, syringes for injection, customized IV delivery systems, inhalers, etc. Additionally, the unit dose kit can contain instructions for preparation and administration of the compositions. The kit may be manufactured as a single use unit dose for one patient, multiple uses for a particular patient (at a constant dose or in which the individual compounds may vary in potency as therapy progresses); or the kit may contain multiple doses suitable for administration to multiple patients ("bulk packaging"). The kit components may be assembled in cartons, blister packs, bottles, tubes, and the like.

### X. Articles of Manufacture

In another embodiment of the invention, an article of manufacture containing materials useful for the treatment of the disorders described above is provided. The article of manufacture comprises a container, a label and a package insert. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is an anti-VEGF antibody. The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, and syringes. In addition, the article of manufacture comprises a package insert with instructions for use, including for example a warning that the composition is not to be used in combination with another composition, or instructing the user of the composition to administer the anti-VEGF antibody composition alone or in combination with an anti-cancer composition to a patient. The term "instructions for use" means providing directions for applicable therapy, medication, treatment, treatment regimens, and the like, by any means, e.g., in writing, such as in the form of package inserts or other written promotional material.

### X. Deposit of Materials

The following hybridoma cell line has been deposited under the provisions of the Budapest Treaty with the American Type Culture Collection (ATCC), Manassas, VA, USA:

| **Antibody Designation** **Deposit Date** | **ATCC No.** | |
|---|---|---|
| A4.6.1 | ATCC HB-10709 | March 29, |
| 1991 | | |

### EXAMPLES

### Example 1. Inhibition of VEGF-A Results in Arrest of Intestinal Adenoma Growth and Long-term Survival of Apc^{min/+} Mice

The syndrome of Familial Adenomatous Polyposis (FAP) and the majority of sporadic colorectal cancers are caused by mutations in the APC gene. FAP patients develop hundreds to thousands of adenomatous polyps in their lower gastrointestinal (GI) tract, in addition to extra-colonic tumors, which include desmoids and tumors of the upper GI tract. Apc^{min/+} mice with a heterozygous truncation allele at codon 850 mimic some features of the polyposis of FAP patients with germ line *APC* mutation (Moser et al., Science 247:322-324 (1990), Su et al., Science 256:668-670 (1992)). The onset of tumor formation in Apc^{min/+} mice is in early adulthood and the animals typically develop 60-150 intestinal polyps in a C57BL/6 genetic background. Tumor development results in a severely compromised longevity of the mice, usually resulting in death from anemia and/or hypoproteinemia (Moser et al., Science 247:322-324 (1990)) at around the age of five months. While humans with FAP typically develop colonic adenomas, Apc^{min/+} mice, for reasons that are not fully understood, develop the vast majority of polyps in the small intestine. These polyps reach a size of 1-2 mm in diameter, while larger polyps (up to 4 mm in diameter) arise at a lower frequency. Only occasional colonic adenomas are observed, commonly 0-3 per animal.

Apc has been reported to be involved in cellular processes including proliferation, apoptosis, cell migration, cell adhesion, microtubule assembly, signal transduction, and chromosome segregation (reviewed in Nathke, Annu. Rev. Cell. Dev. Biol. 20:337-366 (2004)). The best-studied function of Apc is its role as a regulator of beta-catenin on the Wnt signaling pathway (reviewed in Nathke, Mol. Pathol. 52:169-173 (1999)). Briefly, in the absence of Wnt signaling, Apc binds to axin and GSK-3beta kinase to form a destruction complex for cytoplasmic beta-catenin, thereby preventing its nuclear translocation and the subsequent activation of the T-cell factor/lymphoid enhancer factor (TCF/LEF) family of transcription factors. The transcriptional targets of TCF/LEF include molecules involved in cellular pathways mentioned above.

Investigating the mechanisms of tumor growth in xenografts has some limitations, since these models do not recapitulate tumor development in a natural setting. To examine the effects of anti-angiogenic therapy on a naturally occurring, genetically predisposed non-malignant tumor model, we have studied the Apc^{min/+} model of intestinal adenomatosis. In the following example, the tumor phenotype of Apc^{min/+} mice was analyzed after short- and long-term treatment with the exemplary VEGF-specific antagonist, anti-VEGF-A mAb, as well as after a genetic deletion of VEGF-A by Cre-LoxP technology in intestinal epithelial cells.

For the experiments described below, Apc^{min/+} mice (stock number 002020, 5) and 12.4KbVilCre mice (stock number 004586, hereafter VillinCre, Madison et al., J. Biol. Chem. 277:33275-33283 (2002)) were obtained from The Jackson Laboratory (Bar Harbor, ME). VEGF^{lox/lox} mice (hereafter VEGF^{lox}) have been previously published (Gerber et al., Development 126:1149-1159 (1999)). Mice were housed in micro isolator cages in a barrier facility and fed ad libitum. Maintenance of animals and experimental protocols were conducted following federal regulations and approved by Institutional Animal Care and Use Committee.

### Expression of VEGF-A in the Apc^{min/+} Intestinal Adenomas

To investigate the expression pattern of VEGF-A in intestinal tumors of Apc^{min/+} mouse, we performed *in situ* hybridization on adenomas from 14-week old mice. For these experiments, *in situ* hybridization was performed as previously described (Ferrara et al., Am. J. Pathol. 162:1881-1893 (2003)). Briefly, neutral buffered formalin fixed, dehydrated, and paraffin embedded intestinal tissue sections were deparaffinized and hydrated prior to deproteination in 20 µg/ml proteinase K for 15 minutes at 37°C. [³³P]UTP-labeled sense and antisense riboprobes were hybridized at 55°C overnight, followed by a high stringency wash at 55°C in 0.1X standard saline citrate for 2 hours. The dry glass slides were exposed for 3 days at room temperature to Kodak BioMax MR autoradiographic film (Eastman Kodak Co., Rochester, NY), followed by dipping in NTB2 nuclear track emulsion (Eastman Kodak Co.), exposure in sealed plastic slide boxes containing desiccant for 28 days at 4°C, developing, and counterstaining with (hematoxylin eosin) H&E. VEGF-A probe was prepared as previously described (Ferrara et al., Am. J. Pathol. 162:1881-1893 (2003)). VEGF-A probe length was 349 nucleotides corresponding to nucleotides 297-645 of NM_031836. The upper primer sequence was 5'- CAA CGT CAC TAT GCA GAT CAT GCG (SEQ ID NO: 1); the lower primer sequence was 5'- GGT CTA GTT CCC GAA ACC CTG AG (SEQ ID NO: 2).

In these in situ hybridization experiments, VEGF-A expression was observed in the epithelial cells with varying intensity compared to normal intestinal villus epithelium, while a focally prominent signal was observed in stromal cells of the adenomas, as well as in the stroma of the normal villi (Figs. 1A-F).

### Inhibition of VEGF-A Lowers Tumor Burden of Apc^{min/+} Mice

To determine whether anti-VEGF-A therapy would be effective at lowering the tumor burden of benign intestinal tumors in the mouse, we treated Apc^{min/+} mice with the anti-VEGF-A mAb G6-31 in a mouse-human chimeric format, to reduce the possibility of eliciting an immune response. The anti-VEGF-A mAb G6-31 was derived from human Fab phage libraries as described (Liang et al., J. Biol. Chem. 281:951-961 (2006)). To generate an antibody suitable for long-term administration in mice, the variable domains were grafted into murine IgG2a constant domain. mAb G6-31 (Liang et al., J. Biol. Chem. 281:951-961 (2006)) or isotype matched control murine IgG2a (anti-GP120), both at the dose of 5 mg/kg, was administered intraperitoneally once a week in a 90-140 µl volume in PBS. Treatments were continued for 3 weeks, 6 weeks, up to one year, or until mice were found moribund. Treatment of 5-14 mice per each group was started at 91±3 days of age.

We chose mAb G6-31 because of its ability to potently block both mouse and human VEGF-A (Liang et al., J. Biol. Chem. 281:951-961 (2006)). This is unlike the well-characterized anti-VEGF mAb A.4.6.1, which inhibits human but not mouse VEGF-A (Gerber et al., Cancer Res. 60:6253-6258 (2000), Liang et al., J. Biol. Chem. 281:951-961 (2006)). To assess the short-term effect of mAb G6-31 on tumor burden, treatment of ten mice per cohort was started at thirteen weeks of age and continued for 3 or 6 weeks. To determine the tumor phenotype at the age of treatment onset, an untreated control group of twelve mice was analyzed at thirteen weeks of age (day 0).

Treatment with anti-VEGF-A mAb for either 3 or 6 weeks significantly reduced overall tumor burden in the Apc^{min/+} mice. At day 0, the mean tumor burden of Apc^{min/+} mice was 39.3 mm³ (ranging from 12.3 mm³ to 97.0 mm³) (Fig. 2A). The mean tumor burden of mice treated with control IgG for three weeks was 96.8 mm³ (47.1-299.9 mm³), whereas the mean tumor burden of mice treated for 3 weeks with mAb G6-31 was 23.5 mm³ (4.5-58.2 mm³). This was a statistically significant 76%, or 4-fold, reduction in mean tumor burden upon mAb G6-31 treatment, with a p<0.008. After six weeks of administration with control IgG, the tumor burden reached a mean of 198.6 mm³ (40.5-315.7 mm³), while the tumor burden in mice treated with mAb G6-31 remained low at 28.4 mm³ (3.2-75.9 mm³), exhibiting a significant 86%, or 7 fold reduction in mean tumor burden with a p<5.3 x 10⁻⁵ (Fig. 2A).

The marked decrease in tumor burden after both three and six weeks of treatment with mAb G6-31 was due to a decreased adenoma size, as opposed to a decreased number of adenomas. After three weeks of treatment with control IgG, the mean tumor number was 116±9 (±SEM), while after mAb G6-31 administration the mean tumor number was 107±11 (p<0.28). After six weeks of treatment with control IgG, the mean tumor number was 120±11, while after mAb G6-31 administration it was 100±10 (p<0.09). At day 0, mice had an average of 100±9 tumors.

For the analysis of tumor size and number, the intestinal tract from glandular stomach to rectum was opened longitudinally, rinsed and spread flat on a filter paper. Following overnight fixation with Notox Histo Fixative (Scientific Design Laboratory Inc., Des Plaines, IL) and staining with methylene blue 0.1% aqueous solution, the number, location, and diameter of each intestinal adenoma of the small and large bowel was scored by a single observer, blinded to the treatment, through an ocular scale under 20x magnification on a Leica dissection microscope. By this method, polyps with a diameter 0.3 mm or greater were recorded reliably. Tumor volumes were calculated as hemispheres. Tumor burden for each mouse was calculated as a sum of its tumor volumes. P-values have been calculated with a two-tailed Student's t-test. A non-treated group of mice (day 0) were analyzed at the age of treatment onset (13 weeks) as a control to the antibody treated mice.

There was no evidence that adenoma growth escaped anti-VEGF-A treatment during 3 or 6 weeks of treatment: tumors in mice treated with mAb G6-31 had a more compact size distribution (Fig. 2B, middle and bottom graph) compared to the broader size distribution of tumors from mice treated with control IgG (Fig. 2B, graphs second and fourth from the top). The mean polyp diameter in mice administered for three weeks with control IgG was 1.28 mm, and with mAb G6-31 0.85 mm (p<9.2 x 10⁻¹¹⁷), while the mean polyp diameter in mice administered for six weeks with control IgG was 1.64 mm, and with mAb G6-31 0.86 mm (p<2.7 x 10⁻²¹⁴). Mean tumor diameter at day 0 was 0.97 mm.

Interestingly, anti-VEGF-A treatment appeared to inhibit the growth of tumors of all sizes. After a 3-week-treatment with mAb G6-31, the frequency of small tumors, 0.3-1.0 mm in diameter (for 6-week treatment 0.3-1.2 mm) was greater than in the control treated group, while the frequency of tumors with larger than 1.0 mm diameter (for 6 weeks >1.2 mm) was decreased (Fig. 2C, top and middle graphs). A comparison to the tumor size distribution at day 0 (Fig. 2C, bottom graph) suggested that the growth of the adenomas had essentially arrested upon the start of mAb G6-31 administration.

Moreover, anti-VEGF-A mAb G6-31 was effective at suppressing adenoma growth in all small-intestinal areas. A significantly lower mean tumor diameter was observed upon mAb G6-31 treatment compared to control IgG treatment after both three and six weeks of therapy (Fig. 2D). Furthermore, the mean adenoma diameter in the first intestinal quarter of mice treated with mAb G6-31 was significantly reduced from that observed in mice at day 0 (double asterisk in Fig. 2D). The reduction in mean tumor diameter of the colonic adenomas did not reach statistical significance (Fig. 2D). The mean diameter of the large bowel polyps in mice treated with mAb G6-31 for three weeks was 1.3±0.3 mm (±SEM), while the mean diameter in the control IgG-treated mice was 2.5±0.4 mm, with a p<0.064. The mean diameter of large bowel tumors after six weeks of treatment with mAb G6-31 was 2.2±0.3 mm and 2.6±0.3 mm after administration with control IgG, with a p<0.37.

### Deletion of VEGF-A in Intestinal Epithelial Cells Reduces Mean Tumor Diameter

We next sought to dissect the contribution of VEGF-A from intestinal epithelial sources to adenoma development in the Apc^{min/+} model. To this end, tumor diameter and number were assessed, as described above, in 13 week-old Apc^{min/+} mice that were crossed to mice in which VEGF-A was conditionally deleted in intestinal epithelial cells with Cre/loxP technology (VEGF^{lox};Villin-Cre mice). Apc^{min/+};Villin-Cre and Apc^{min/+};VEGF^{lox};Villin-Cre mice were analyzed at thirteen weeks of age.

The expression of Villin, an actin-binding protein and a major structural component of the brush border of specialized absorptive cells, begins during embryogenesis in the intestinal hindgut endoderm, and later extends throughout the small- and large-intestinal endoderm (Braunstein et al., Dev. Dyn. 224:90-102 (2002), Ezzell et al., Development 106:407-419 (1989), Maunoury et al., EMBO J. 7:3321-3329 (1988), and Maunoury et al., Development 115:717-728 (1992)). In the adult, Villin distribution becomes diffuse with moderate apical polarization in immature, proliferative cells of the crypts, and strong polarization in brush borders of fully differentiated cells lining the villi of the small intestine Robine et al., Proc. Natl. Acad. Sci. 82:8488-8492 (1985)). The expression of Cre recombinase driven by Villin promoter (Villin-Cre) has been previously characterized recapitulating the expression pattern of the Villin gene in every cell of the intestinal epithelium from crypt to villus tip and duodenum through colon Madison et al., J. Biol. Chem. 277:33275-33283 (2002).

Phenotypic analysis revealed that the mean tumor diameter of control Apc^{min/+};Villin-Cre mice was 1.02±0.3 mm (±SEM), whereas the mean tumor diameter of Apc^{min/+};VEGF^{lox};Villin-Cre mice was 0.82±0.3 mm (Fig. 2E), demonstrating a 19.8% reduction (p<7.2 x 10⁻⁵). Tumor number was not significantly different between the two groups. While Apc^{min/+};Villin-Cre mice had 137±11 intestinal adenomas, Apc^{min/+};VEGF^{lox};Villin-Cre mice had 150±17 adenomas (p<0.27).

These data indicate that deletion of VEGF-A from all intestinal epithelial cells from duodenum through colon, and crypt to villus tip results in a significant inhibition of tumor growth, albeit of a reduced degree compared to that resulting from systemic administration of anti-VEGF-A antibody. Thus, these data suggest that extra-epithelial sources of VEGF-A contribute to the growth of intestinal adenomas of Apc^{min/+} mice.

### Inhibition of VEGF-A Extends the Median Survival of Apc^{min/+} Mice

Given the effectiveness of anti-VEGF-A treatment in tumor growth inhibition, we wanted to investigate whether treatment with mAb G6-31 could yield long-term benefits for Apc^{min/+} mice. To this end, administration with mAb G6-31 or control IgG was continued up to 52 weeks or until the mice were observed to be moribund. Interestingly, mAb G6-31 treatment increased the median survival from 24.0 weeks with control IgG to 33.6 weeks with mAb G6-31 with log-rank p<2.4 x 10⁻³ (Fig. 2F).

Tumor phenotype of four mice treated with mAb G6-31 was analyzed upon euthanization at the age of 32, 51, 64, or 66 weeks (after 19, 38, 51, or 53 weeks of treatment with mAb G6-31, respectively). As is shown in Table 1, the mean tumor diameter remained at a level close to that seen in nineteen-week-old mice (1.64 mm in mice treated with control IgG for six weeks). Similarly, the tumor number remained comparable to that of thirteen-week-old mice (day 0 group mice had 59-161 intestinal adenomas with a mean of 100). Three (one from each mouse 2, 3, and 4 of Table 1) of fifteen colonic adenomas (total number identified in mice 1-4) that were caught at the plane of section in histologic analysis displayed no malignant transformation.

**Table 1. Tumor data of mice on extended G6-31 treatment.**

| mouse | age (weeks) | weeks on G6-31 | tumor number | mean tumor diameter (mm) | tumor burden (mm3) |
|---|---|---|---|---|---|
| 1 | 32 | 19 | 55 | 0.65 | 6.3 |
| 2 | 51 | 38 | 133 | 1.72 | 263.5 |
| 3* | 64 | 51 | 85 | 2.21 | 341.1 |
| 4* | 66 | 53 | 150 | 1.63 | 282.2 |

| | | | | | |
|---|---|---|---|---|---|
| *healthy animal euthanized at study end point | | | | | |

In summary, long-term anti-VEGF-A treatment was generally well tolerated and yielded in an increased survival of Apc^{min/+} mice. Moreover, tumor number and mean tumor diameter in mice treated long-term with mAb G6-31 remained strikingly low in view of the age of the mice, consistent with an inhibition of new adenoma formation and adenoma growth.

### Normal Serum Total Protein, Albumin and Triglycerides Level, and Reduced Splenic Extramedullary Hematopoiesis in Apc^{min/+} Mice Treated with Anti-VEGF-A

As a general observation, Apc^{min/+} mice treated with mAb G6-31 appeared considerably more alert and responsive than mice treated with control IgG. Moreover, pale paws, suggestive of the progressive anemia initially reported by Moser et al (Moser et al., Science 247:322-324 (1990)), were regularly observed in animals treated with control IgG, but not in animals treated with mAb G6-31. In line with this observation, the mean total serum protein and serum albumin of Apc^{min/+} mice administered with control IgG was decreased, while total protein and albumin levels were within normal range in mice treated with mAb G6-31 (Table 2).

**Table 2. Serum chemistry.**

| group | total protein (g/dl)* | albumin (g/dl)** | triglycerides (mg/dl)*** |
|---|---|---|---|
| control IgG 3 weeks (n=10) | 3.7±0.2 | 1.9±0.1 | 268.9±82.5 |
| G6-31 3 weeks (n=10) | 4.9±0.2 | 2.6±0.1 | 75.5±4.9 |
| control IgG 6 weeks (n=10) | 3.0±0.3 | 1.6±0.2 | 591.1±81.3 |
| G6-31 6 weeks (n=10) | 4.9±0.1 | 2.7±0.1 | 71.1±4.3 |

| | | | |
|---|---|---|---|
| *reference value 3.9-5.5 g/dl **reference value 2.3-3.2 g/dl ***reference value 35-244 mg/dl ± standard error of the mean (SEM) | | | |

As reported for Apc^{min/+} mice (Moser et al., Science 247:322-324 (1990)), and consistent with hypoproteinemia, mean triglyceride level was elevated in animals treated with control IgG, though it was lowered to a level comparable to a reference value upon treatment with mAb G6-31 (Table 2).

While there were no treatment-related differences in body masses after three or six weeks of treatment, the mean spleen masses were significantly (p<2.3 x 10⁻³) increased in mice treated with control IgG. After three weeks of administration with control IgG, the mice had a mean spleen mass of 0.26 g, or 1.17% of body mass, while the mean spleen mass was 0.11 g (0.49% of body mass) in mice treated with mAb G6-31 for three weeks. The increase in mean spleen mass in mice treated with control IgG is consistent with extramedullary hematopoiesis (EMH, compensatory erythropoiesis, in this case secondary to intestinal bleeding), which was confirmed by histologic examination of the spleens. Ten of ten mice treated for 6 weeks with control IgG showed marked EMH, while two mice treated with mAb G6-31 had moderate EMH, five had mild EMH, and three had no diagnostic changes in their spleens. Four of five mice treated with mAb G6-31 for 18-53 weeks were diagnosed with mild to extensive EMH in the spleen.

The lower degree of EMH in the spleens of mice treated with mAb G6-31 short-term suggests that anti-VEGF-A therapy has a beneficial effect on reducing intestinal bleeding.

### Kidney Changes After Long-term Treatment with mAb G6-31

To investigate potential toxicity related to administering high-affinity anti-VEGF-A mAb G6-31, pancreas, liver, and kidney were analyzed histologically after short- (3-6 weeks) and long-term (18-53 weeks) treatment. For the histological analysis, Notox fixed intestinal tissue was dehydrated and embedded in paraffin, sectioned, and stained with H&E for histological analysis following standard protocols.

No significant toxicity was noted in animals treated for 3-6 weeks. After long-term treatment with mAb G6-31, five of five mice showed variable (mild to severe) diffuse global glomerulosclerosis and moderate stromal edema of the pancreas (reflecting hypoproteinemia). These observations are consistent with previously observed toxicity resulting from long-term administration of mAb G6-31. Importantly, the adverse effects were outweighed by the overall improvement of health reflected by the increased median survival.

### Altered Tumor Morphology upon mAb G6-31 Treatment was not Accompanied by a Change in Proliferative Index

To further characterize intestinal polyps in Apc^{min/+} mice following treatment with anti-VEGF-A mAb G6-31, macroscopic and histologic analyses were performed as described above. The gross morphology of polyps treated with mAb G6-31 differed noticeably from that of the polyps treated with control IgG (Figs. 3A-B). While tumors from mice administered with control IgG typically had a relatively unbroken, smooth surface, tumors from animals treated with mAb G6-31 appeared with deep invaginations on their surface. Histologic analysis revealed tumors from mAb G6-31 and control IgG-treated mice to be tubular adenomas (Figs. 3C-F). Adenomas from mice treated with control IgG had marked intra-villous epithelial proliferation, with vertical and lateral expansion, and were typically widened more than 2-fold from their base to luminal surface. There was minimal fibrous stroma. Adenomas from mice treated with mAb G6-31 characteristically had fewer intra-villous epithelial cells, were less broad at the luminal surface, shallower, and involved fewer adjacent villi. Histologic analysis of the colonic polyps in both treatment groups showed pendunculated tubular adenomas with abundant fibrovascular stroma and a variable amount (up to 100%) of dysplastic epithelium.

To assess the extent of proliferation in the tumor tissue and in normal mucosa, an indirect immunohistochemical staining with Ki-67 antibody was performed (Figs. 3G-J). For these experiments, Notox fixed, dehydrated, and paraffin embedded intestinal tissue sections were deparaffinized and hydrated prior to incubation in Target Retrieval (DAKO, Glostrup, Denmark) at 99°C followed by quenching of endogenous peroxidase activity and blocking of avidin and biotin (Vector, Burlingame, CA). Sections were further blocked for 30 minutes with 10% blocking serum in PBS with 3% bovine serum albumin. Tissue sections were incubated with primary antibodies diluted in the blocking serum for 60 minutes, washed with TBST Buffer (DAKO) and incubated with secondary antibodies for 30 minutes, washed with TBST, and incubated in ABC Elite Reagent (Vector) for 30 minutes followed by an incubation in Metal Enhanced DAB (Pierce, Rockford, IL) and counterstaining with Mayer's hematoxylin. The primary antibody used was rabbit polyclonal against Ki-67 (SP6, 1:200, Lab Vision, Fremont, CA). Secondary antibody used was biotinylated goat anti-rabbit (7.5 µg/ml, Vector). All steps were performed at room temperature.

Quantitative analysis revealed similar amounts of Ki-67 positive cells in tumors from mice treated with either control IgG or with mAb G6-31. Likewise, the proliferative index of the normal adjacent mucosa was comparable between both treatments (Fig. 3K). The proliferative index was quantified from images of 5 µm paraffin sections of tumor tissue and normal mucosa acquired with an Ariol SL50 slide scanning microscope system (Applied Imaging, Inc., San Jose, CA) utilizing the Kisight assay (Ariol Review (v2.6)). Regions of tumor tissue and normal mucosa were manually identified and circumscribed by a blinded examiner. Proliferative index, measured as the percent of Ki-67 positive nuclei relative to total nuclei, was quantified in a semi-automated fashion based on nucleus color following definition of a positive threshold. Proliferative index measurements included analysis of 29 tumors in the mAb G6-31 treatment group (n=3), and 44 tumors in the control IgG group (n=3), all treated for six weeks.

To test whether the growth inhibition by mAb G6-31 was accompanied by changes in the expression level of molecules part of major signal transduction pathways, a Western blot analysis was performed. Jejunal adenomas and normal adjacent mucosa from mAb G6-31 and control antibody treated mice were harvested with a scalpel and mechanically homogenized with OMNI TH115 homogenizer in RIPA lysis buffer. Primary antibodies used were rabbit polyclonals against p38 MAPK, phosphor-p38 MAPK, p42/p44 MAPK, phosphor-p42/p44 MAPK, PTEN, Akt, phosphor-Akt, and phosphor-GSK3alpha/beta (all 1:1000, Cell Signaling, Danvers, MA). The secondary antibody used was horseradish peroxidase conjugated anti-rabbit (1:5000, Chemicon).

While the expression level of many of the molecules tested remained unchanged upon treatment with mAb G6-31, a modest restoration of phospho-p38 MAPK levels in three of four tumor samples towards those found in normal mucosa was observed (Fig. 3L; p-p38, compare T5-T8 with N5-N8).

### Reduced Vascular Density in mAb G6-31 Treated Tumors

Given that VEGF-A is known to be a mitogen for vascular endothelial cells through VEGFR-2 signaling, we examined the tumor vascular networks in mice treated with Mab G6-31 and control IgG by immunohistochemical staining of thick tissue sections with anti-CD31 antibody (Figs. 4A-B). For confocal microscope imaging purposes, mice were perfusion fixed with 1% paraformaldehyde (PFA) in PBS under isofluorane anesthesia, intestinal tract was spread flat on a filter paper, post fixed with 4% PFA, and submerged in 30% sucrose in PBS overnight at 4°C prior to embedding in O.C.T. and freezing on dry ice. Cryosections were cut at 80 µm, fixed in 4% PFA for 10 minutes, permeabilized with 0.2% Triton-X-100 in PBS, and blocked for 30 min with 5% normal goat serum in PBS with 0.2% Triton-X-100. Primary antibodies in blocking buffer were incubated overnight, secondary antibodies for 5-6 hours, washed with PBS and counterstained with Hoechst 33342 (0.5 mg/ml; Sigma, St. Louis, MO). Primary antibodies used were hamster monoclonal against CD31 (1:500, Chemicon, Temecula, CA), rat monoclonal against E-cadherin (1:2500, Zymed, South San Francisco, CA), and Cy3 conjugated mouse monoclonal against smooth muscle actin (1:1000, Sigma). Secondary antibodies used were Cy5 conjugated anti-Armenian hamster (1:500, Jackson Immunoresearch, Cambridgeshire, UK), and ALEXA 488 conjugated anti-rat (1:500, Molecular Probes, Eugene, OR).

Vessel density in tumors from Apc^{min/+} mice was quantified from digital images acquired with a CCD camera on a Zeiss Axioplan2 fluorescence microscope (Thornwood, NY). Each of the four groups (mice treated for 3 or 6 weeks with control IgG or mAb G6-31) consisted of two mice, and 11-22 tumors from each group were analyzed. Vessel area in 80-µm tumor sections was calculated via a threshold-based segmentation of CD31 positive fluorescence using ImageJ v.1.36 (http://rsb.info.nih.gov/ij/). Vessel density was then calculated as the ratio of CD31 positive pixels to total tumor area. Values of all tumors from each group were averaged to yield a mean value for the group.

Quantification of the vessel density indicated that the vascular component of tumors from mAb G6-31-treated mice was reduced compared to that seen in control IgG treated mice (Fig. 4C). After three weeks of administration with control IgG the mean tumor vessel area density was 23.2%, while it was reduced to 18.6% after administration with mAb G6-31. The mean vessel area of tumors treated with control IgG for six weeks was 25.5%, whereas the mean vessel area density of tumors from mice treated with mAb G6-31 was 19.7%.

### Discussion

We used Apc^{min/+} mice to investigate the role of VEGF-A in benign intestinal tumorigenesis. In the first part, the experiments were designed to measure the effects of short- and long-term anti-VEGF-A treatment on established intestinal adenomas undergoing robust growth. We have shown that treatment with anti-VEGF-A mAb G6-31 significantly lowered the tumor burden and extended the survival of the Apc^{min/+} mice.

Several studies have been conducted on the effect of dietary and chemopreventive agents, including non-steroidal anti-inflammatory drugs (NSAID), on tumor burden of Apc^{min/+} mice (reviewed in Corpet et al., Cancer Epidemiol. Biomarkers Prev. 12:391-400 (2003)), of which an updated list exists at http://corpet.net/min. Many of these studies report a significant decrease in tumor number. NSAIDs such as piroxicam and sulindac, which target both COX-1 and COX-2, have been among the most potent agents in suppressing tumor formation in Apc^{min/+} mice (Boolbol et al., Cancer Res. 56:2556-2560 (1996), Chiu et al., Cancer Res. 57:4267-4273 (1997), Hansen-Petrik et al., Cancer Lett. 175:157-163 (2002), Ritland et al., Carcinogenesis 20:51-58 (1999)), in addition to selective COX-2 inhibitors such as celecoxib (Jacoby et al., Cancer Res. 60:5040-5044 (2000)) and A-285969 (Wagenaar-Miller et al., Br. J. Cancer 88:1445-1452 (2003)). Combination therapies have also been used successfully to lower tumor number. Torrance et al. utilized a specific epidermal growth factor receptor inhibitor, EKI-785, in combination with sulindac, and showed near to a total elimination of tumor number (Torrance et al., Nat. Med. 6:1024-1028 (2000)). Similarly, the combined effect of chemotherapy agents raltitrexed (RTX) and 5-fluorouracil (FU) resulted in a significant (37%) reduction in Apc^{min/+} tumor numbers (Murphy et al., Cancer Biol. Ther. 3:1169-1176 (2004)). Recently, short-term administration of the receptor tyrosine kinase (RTK) inhibitor AZD2171 demonstrated a reduction of tumor burden in the Apc^{min/+} model (Goodlad et al., Carcinogenesis 27:2133-2139 (2006)). They noted that earlier treatment onset (at 6 weeks) with AZD2171 was able to reduce tumor number, whereas later intervention (at 10 weeks) only reduced tumor size (Goodlad et al., *supra*)*.* However, the treatment had no effect on vascular density (Goodlad et al., *supra*). AZD2171 inhibits several RTKs including, but not limited to, VEGFR-1, -2, and -3 (Wedge et al., Cancer Res. 65:4389-4400 (2005)).

We observed that anti-VEGF-A Mab G6-31 administered at 13 weeks did not reduce the number of existing tumors, although it decreased tumor size and appeared to inhibit new adenoma formation. These results correlated with an observed increase in survival. However, we believe that it is possible that an anti-VEGF-specific tumor prevention approach (with an earlier treatment onset) could potentially be more effective in reducing tumor number, than a tumor intervention approach (with a later treatment onset), that was used in our study. Regardless, anti-VEGF specific inhibition was effective at all stages of tumor growth.

Our study conclusively shows that targeting VEGF-A is sufficient to achieve profound therapeutic effects in the Apc^{min/+} model. Comparison of the systemic VEGF-A inhibition with Mab G6-31 to a genetic deletion of VEGF-A in the intestinal epithelial compartment alone in Apc^{min/+};VEGF^{lox};Villin-Cre mice suggests that, in addition to epithelial cells, other cellular sources of VEGF-A play an important role in Apc^{min/+} adenoma growth. These additional sources of VEGF-A potentially include mononuclear cells (Sunayama et al., Carcinogenesis 23:1351-1359 (2002)) and stromal fibroblasts (Seno et al., Cancer Res. 62:506-511 (2002), (Williams et al., J. Clin. Invest. 105:1589-1594 (2000)). Our *in situ* analysis indicates extra-epithelial VEGF-A expression within the adenomas and normal villi, supporting the observation.

Based on an extensive body of data, it is conceivable that much of the observed anti-tumor effects of mAb G6-31 is mediated by suppression of angiogenesis (Wise et al., Proc. Natl. Acad. Sci USA 96:3071-3076 (1999), Zachary et al., Cardiovasc Res. 49:568-581 (2001)). Indeed, a reduced vascular supply in response to anti-VEGF-A monoclonal antibody has been observed in tumor xenograft studies (Borgstrom et al., Prostate 35:1-10 (1998)). In agreement with this, a reduction in vessel area density of the Apc^{min/+} intestinal adenomas was observed after three and six weeks of administration with mAb G6-31, compared to tumors from mice treated with control IgG.

The observed significant accumulation of adenomas *smaller* than 1 mm upon inhibition of VEGF-A suggests that in the intestinal adenomas of the Apc^{min/+} mice, angiogenic switch may happen earlier than generally believed for tumor development, as has been seen in the Apc^{delta716} model (Seno et al., Cancer Res. 62:506-511 2002)).

An important and unexpected conclusion of our study is that anti-angiogenic monotherapy, targeting a single angiogenic factor, can be highly effective at suppressing tumor growth and can yield a survival benefit. This seems to be in contrast to a view, gathered primarily from investigation of malignant tumors, that the main benefit of such a therapy is to "normalize" tumor blood vessels in order to facilitate delivery of chemotherapy (Jain et al., Nat. Med. 7:987-989 (2001)). It is conceivable that a reduced propensity of benign tumors to acquire mutations, potentially leading to treatment resistance, may account, at least in part, for the difference. Therefore, our data suggest the possibility of a non-surgical treatment for benign tumors without the need for chemotherapeutic agents.

### Example 2. Anti-VEGF-A Monoclonal Antibody Inhibits the Growth of Pituitary Adenomas and Lowers Serum Prolactin and Growth Hormone Level in a Mouse Model of Multiple Endocrine Neoplasia

Multiple endocrine neoplasia (MEN) is a disorder characterized by the incidence of tumors involving two or more endocrine glands. A patient is classified with MEN type 1 (MEN1) when a combined occurrence of tumors in the parathyroid glands, the pancreatic islet cells, and the anterior pituitary is identified. Mutations in the *MEN1* gene were discovered to underlie the disorder, which commonly result in a truncation or absence of the protein menin (reviewed in Pannett et al., Endocr. Relat. Cancer 6:449-473 (1999)). With the added finding of a frequent loss of the remaining allele in the tumors, (Bystrom et al., Proc. Natl. Acad. Sci USA 87:1968-1972 (1990)*,* Debelenko et al., Cancer Res. 57:2238-2243 (1997), Larsson et al., Nature 332:85-87 (1988)) *MEN1* has been classified as a tumor suppressor gene. While MEN1 is largely inherited as an autosomal dominant disorder, *de novo* mutations of *MEN1* gene have been identified as the cause of sporadic cases of MEN1.

The function of menin remains largely unknown. The ubiquitously expressed, predominantly nuclear 610-amino acid protein has been suggested to be involved in transcriptional regulation, DNA processing and repair, and cytoskeletal organization through its *in vitro* interactions with proteins part of the above mentioned pathways (reviewed in Agarwal et al., Horm. Metab. Res. 37:369-374 (2005)). None of the protein interactions identified so far however provide an explanation to the tumorigenicity in MEN1.

Current standard of treatment for pancreatic tumors - more than 50% of which are gastrinomas and 10-30% insulinomas - is reduction of basal acid output in the case of gastrinomas, while surgery is seen as the optimal treatment for insulinomas. The treatment for pituitary tumors consists of selective surgery with varying medical therapy depending on the hormonal profile, while the definitive treatment for parathyroid tumors is a surgical removal of the overactive gland. However, there is variability to the degree and timing of the parathyroidectomy (reviewed in Brandi et al., J. Clin. Endocrinol. Metab. 86:5658-5671 (2001)). The latest developments on the generation of new approaches to the diagnosis and treatment of MEN 1 have recently been reviewed (Viola et al., Curr. Opin. Oncol. 17:24-27 (2005)).

Through homologous recombination, exons 3-8 of the mouse gene Men1 have been targeted for deletion (Crabtree et al., Proc. Natl. Acad. Sci. USA 98:1118-1123 (2001)). By nine months of age, heterozygous Men1 mice were reported to develop pancreatic islet lesions with additional frequent observations of parathyroid adenomas. Larger, more numerous tumors in pancreatic islets, parathyroids, thyroid, adrenal cortex, and pituitary were seen by 16 months of age (Crabtree et al., Proc. Natl. Acad. Sci USA 98:1118-1123 (2001)), features remarkably similar to the human disorder.

Ample evidence exists indicating that blocking of VEGF-A-mediated angiogenesis results in tumor suppression (Gerber et al., Cancer Res. 60:6253-6258 (2000), Holash et al., Proc. Natl. Acad. Sci. USA 99:11393-11398 (2002), Millauer et al., Nature 367:576-579 (1994), Prewett et al., Cancer Res. 59:5209-5218 (1999), Wood et al., Cancer Res. 60:2178-2189 (2000)) as anti-VEGF-A approaches have been used in treatment of various preclinical models derived from human malignant cancer cell lines (reviewed in Geber et al., Cancer Res. 60:2178-2189 (2000)). Tumor xenografts however poorly recapitulate tumor development in a natural setting. Furthermore, anti-VEGF-A antibody therapy has thus far not been attempted in inhibiting the growth of benign tumors, or tumors of endocrine origin. To investigate the role of VEGF-A in the development of endocrine tissue-specific adenomas, we examined the effects of anti-angiogenic therapy on a naturally occurring non-malignant tumor model, the Men1^{+/-} mouse model of MEN1. Tumor volume of pituitary adenomas in Men1^{+/-} mice, as well as subcutaneous pituitary tumor transplants in Balb/c Nude mice were analyzed after a short-term treatment with the exemplary VEGF-specific antagonist, anti-VEGF-A monoclonal antibody (mAb). In addition, the possibility of lowering the elevated hormone levels associated with MEN1 with anti-VEGF-A mAb was investigated.

For the experiments described below, Men1^{+/-} mice (stock number 004066) were obtained from The Jackson Laboratory (Bar Harbor, ME), and Balb/c Nude mice from Charles River Laboratories Inc. (Wilmington, MA). Experimental Men1^{+/-} female mice of mixed 129-FVB background were obtained by intercrossing Men1^{+/-} males and females. Mice were housed in micro-isolator cages in a barrier facility and fed *ad libitum.* Maintenance of animals and experimental protocols were conducted following federal regulations and approved by Institutional Animal Care and Use Committee.

### Treatment with mAb G6-31 Inhibits the Growth of Men1^{+/-} Pituitary Adenomas

To investigate whether anti-VEGF-A therapy would be effective in inhibiting the growth of pituitary adenomas, 125 eleven to thirteen month-old female Men1^{+/-} mice were subjected to MRI to identify mice with pituitary tumors. Tumor-bearing mice were subjected to imaging again 14 and 28 days later to establish the growth rate of the adenomas. A cohort of nine mice with 12.4% mean tumor growth per day and 15.58±4.0 mm³ (±SEM) mean tumor volume at study onset received control IgG, and a cohort of eight mice with 10.2% mean tumor growth per day and 16.70±5.7 mm³ mean tumor volume at study onset received an anti-VEGF-A mAb G6-31 for 67 days or until mice were found moribund. For treatment with mAb G6-31 and control IgG antibodies, intraperitoneal injection at 5 mg/kg of anti-VEGF mAb G6-31 (Liang et al., J. Biol. Chem. 281:951-961 (2005)) or isotype matched control IgG (anti-GP120) was given once a week in a 100-200 µl volume in PBS. Administration of eight (mAb G6-31) or nine (control IgG) Men1^{+/-} mice with pituitary adenoma *in situ* was started at 13.5-14.5 months of age and continued for 67 days or until mice were found moribund. Treatment of 23 (control IgG) or 35 (mAb G6-31) Balb/c Nude mice with a subcutaneous pituitary adenoma transplant was started four months after grafting, and continued for 35 days or until mice were found moribund or tumor volume had reached the volume of 3000 mm³.

Animals were imaged with MRI every two weeks to follow up pituitary adenoma growth *in vivo*. MRI images were acquired on a 9.4T horizontal bore magnet (Oxford Instruments Ltd., Oxford, UK) and controlled by a Varian Inova console (Varian, Inc., Palo Alto, CA) using a 3 cm volume coil for transmission and reception (Varian, Inc.). A fast spin echo imaging sequence was employed with a repetition time of 4 seconds, echo train length of 8, echo spacing 12 ms, effective echo time of 48 ms, and six averages. The image matrix was 128², with a field of view (20 mm)² and slice thickness of 0.5 mm. Mice were restrained in the prone position with 2% isoflurane in medical air, and body temperature was monitored with a rectal probe and maintained at 37°C with warm air for the duration of the 15-minute image acquisition. After imaging, animals were allowed to recover on a heated surface followed by returning them to the housing facility. Primary pituitary tumor volumes were calculated from MRI data using three-dimensional regions of interest drawn in Analyze software (AnalyzeDirect, Inc., Lenexa, KS).

At thirty-nine days of treatment, a statistically significant decrease of the mean pituitary tumor volume was observed in the mAb G6-31-treated group compared to the control IgG-treated group (Fig. 5A). At the study end-point (67 days), there was a statistically significant 72%, or 3.7-fold-reduction in mean tumor volume upon mAb G6-31 treatment, with a p-value less than 0.016. While most (6 out of 9) control IgG treated Men1^{+/-} tumors continued to grow robustly throughout the treatment period, the growth of 7 out of 8 mAb G6-31 treated pituitary adenomas slowed down considerably (Fig. 5B). Four control IgG, and three mAb G6-31 treated mice were euthanized before study's end-point due to ill health, including one control IgG treated mouse before imaging on treatment day 25. Tumor doubling-free survival was significantly increased in the mAb G6-31 treated group with a log rank p<0.019 (Fig. 5C), suggesting that the inhibition of pituitary tumor growth improved the health of those mice, compared to the mice treated with control IgG. The tumor volume of two mice in mAb G6-31 treatment group had not doubled by day 67 of treatment.

### Anti-VEGF-A Antibody Inhibits the Growth of Subcutaneous Pituitary Adenoma Transplants

To test the efficacy of anti-VEGF-A antibody treatment on a Men1^{+/-} pituitary adenoma transplant model, subcutaneous tumors were established in the flank of 6-8-week-old female Balb/c Nude mice according to the following procedure. For these experiments, a single *in situ* pituitary adenoma from a Men1^{+/-} mouse was extracted and minced to approximately 1 mm³ pieces, mixed with BD Matrigel Matrix Basement Membrane (BD, Bedford, MA), and inoculated subcutaneously in 200 µl volume to the dorsal flank of Balb/c Nude mice. Four months later, a single subcutaneous tumor (approximate volume 900 mm³) was extracted, minced, mixed with Matrigel and inoculated as described above to establish a cohort of mice with pituitary adenoma transplants.

Tumor size of subcutaneous pituitary adenoma transplants was measured with a caliper tool (Fred V. Fowler Co. Inc., Newton, MA) by collecting the largest tumor diameter and the diameter perpendicular to that. Tumor volume was calculated using the following formula: V=πab²/6 (a=largest tumor diameter, b=perpendicular diameter).

A cohort of 35 mice with a 515±42 mm³ mean tumor volume at treatment onset received mAb G6-31, and a cohort of 23 mice with a 527±64 mm³ mean tumor volume at study onset received control IgG for 35 days using the methods described above. At study end-point, control IgG treated tumors had nearly quadrupled their volumes, to a mean of 2071±152 mm³, while tumor growth in mice treated with mAb G6-31 had essentially stopped; the mean tumor volume was 556±89 mm³ at day 35 (Fig. 5D). There was a statistically significant 73%, or 3.7-fold reduction in mean tumor volume upon mAb G6-31 treatment, with a p<1.9 x 10⁻¹².

These data establish anti-VEGF-A mAb G6-31 effective in inhibiting the growth of pituitary adenomas and subcutaneous pituitary adenoma transplants alike, predisposed by heterozygosity of Men1.

### Expression of VEGF-A, VEGFR-1, and VEGFR-2 in Normal Pituitary Gland and in Pituitary Tumor Tissue

To investigate the expression level of VEGF-A, VEGFR-1, and VEGFR-2 in the pituitary tissue, and to examine whether their expression level was affected by mAb G6-31 treatment, we compared the mean relative expression of VEGF-A, VEGFR-1, and VEGFR-2 in five control IgG treated (mean volume 96.2±8.7 mm³) and five mAb G6-31 treated (35.2±4.0 mm³) *in situ* pituitary adenomas, together with five age-matched small non-treated (9.7±2.9 mm³) pituitary adenomas, four age-matched normal pituitary glands from Men1^{+/-} mice, and eight age-matched wild type pituitary gland samples. VEGF-A, VEGFR-1, and VEGFR-2 expression was also investigated from five control IgG-treated (mean volume 2063±205 mm³) and five mAb G6-31-treated (577±45 mm³) pituitary adenoma transplants.

For these experiments, total DNA-free RNA was prepared from flash frozen pituitary adenomas or normal pituitary glands with RNeasy kit (Qiagen, Hilden, Germany) according to the manufacturer's protocol. One-step quantitative RT-PCR was performed in a total volume of 50 µl with SuperScript III Platinum One-Step qRT-PCR Kit (Invitrogen, Carlsbad, CA), 100 ng of total RNA, 45 nM of each PCR primer, and 12.5 nM Taqman probe. To detect expression of the genes of interest, the following TaqMan Gene Expression Assay primers and probe mixes (Applied Biosystems, Foster City, CA) were used: *VEGF-A* (Assay ID: Mm00437304_m1), *VEGFR-1* (Assay ID: Mm00438980_m1), and *VEGFR-2* (Assay ID: Mm00440099_m1). *GAPDH* expression was detected using primers and Taqman probe synthesized in in-house facility (Forward primer sequence: ATGTTCCAGT ATGACTCCAC TCACG (SEQ ID NO: 3); Reverse primer sequence: GAAGACACCA GTAGACTCCA CGACA (SEQ ID NO: 4); Taqman probe sequence: AAGCCCATCA CCATCTTCCA GGAGCGAGA (SEQ ID NO: 5)).

Reactions were carried out using Applied Biosystems 7500 Real-Time PCR System, with the following conditions: a reverse-transcription step (15 minutes at 48°C), followed by denaturation step (2 minutes 95°C), and 40 cycles of 15 seconds at 95°C and 1 minute at 60°C. Levels of gene expression in each sample were determined with the relative quantification method (ddCt method), using *GAPDH* gene as an endogenous control, and mouse placenta total RNA (Clontech, Mountain View, CA) as a reference.

Notably, the mean relative expression of VEGF-A was significantly elevated in mAb G6-31 treated adenomas *in situ* compared to control IgG treated pituitary tumors, small non-treated tumors, and normal pituitary glands from wild type or Men1^{+/-} mice (Fig. 6). VEGF-A expression was comparably high in both subcutaneous tumor transplant samples. The observed high level of VEGF-A transcript in mAb G6-31 treated tumors is potentially a result of a compensatory mechanism to the systemic sequestering of VEGF-A by mAb G6-31. However, it appeared that elevated VEGF-A was not sufficient to drive the tumor growth.

While the mean relative expression of VEGFR-1 appeared reasonably unchanged within the different tissue samples, the mean relative expression of VEGFR-2 seemed lower in the *in situ* tumor samples treated with control IgG or mAb G6-31 compared to that found in tumor transplants, small non-treated tumors, or normal pituitary glands from wild type and Men1^{+/-} mice (Fig. 6).

### MRI Allows for In Vivo Follow-up of Tumor Growth

MRI was used as described above to follow up the growth of the *in situ* pituitary adenomas throughout the treatment period by subjecting animals to imaging every other week. Graphs of a coronal section of the brain with a representative adenoma from one control IgG and one mAb G6-31 treated Men1^{+/-} mouse are shown in Figure 7, taken 9, 39, and 67 days after treatment onset.

### Histology of Pituitary and Pancreatic Tumors

Men1^{+/-} pituitary gland adenomas were histologically similar in mAb G6-31 and control IgG treated mice (Figs. 8A-B). For these experiments, formalin fixed tissue was dehydrated and embedded in paraffin, sectioned, and stained with hematoxylin-eosin (H&E) for histological analysis following standard protocols. Typically, tumor cells were small (∼10 microns in diameter), with a high nuclear/cytoplasmic ratio, often mitotically active, with up to 40 mitotic figures per ten 625-micron diameter fields. Tumors were variably solid or cystic, with multiple endothelial cell-lined vessels, acutely hemorrhagic areas (intact red blood cells in non-endothelial-lined spaces, absence of fibrin or cellular organization), and scattered hemosiderin-laden macrophages, consistent with previous hemorrhage. There was variable single-cell necrosis, and minimal fibrosis. Tumor vessels were irregularly spaced, typically 5-10 microns in diameter though, rarely, as large as 50 microns, with few non-tumor perivascular stromal cells.

Vascular pattern was observed between control IgG and mAb G6-31 treated tumors by indirect immunohistochemical staining with panendothelial cell marker antibody MECA-32 (Figs. 8C-D). For these experiments, formalin fixed, paraffin embedded tissue sections were deparaffinized prior to quenching of endogenous peroxidase activity and blocking of avidin and biotin (Vector, Burlingame, CA). Sections were blocked for 30 minutes with 10% normal rabbit serum in PBS with 3% BSA. Tissue sections were then incubated with primary antibodies for 60 minutes, biotinylated secondary antibodies for 30 minutes, and incubated in ABC reagent (Vector, Burlingame, CA) for 30 minutes, followed by a 5-minute incubation in Metal Enhanced DAB (Pierce, Rockford, IL). Sections were then counterstained with Mayer's hematoxylin. Primary antibodies used were goat anti-mouse Prolactin at 0.10 µg/ml (R&D systems, Minneapolis, MN) and rat anti-mouse panendothelial cell antigen, clone MECA32, at 2 µg/ml (BD Biosciences, San Jose, CA). Secondary antibodies used were biotinylated rabbit anti-goat at 7.5 µg/ml (Vector, Burlingame, CA) and biotinylated rabbit anti-rat at 2.5 µg/ml (Vector, Burlingame, CA). Panendothelial cell antigen staining required pre-treatment with Target Retrieval (Dako, Carpenteria, CA) at 99°C for 20 minutes. All other steps were performed at room temperature.

Vessel density was significantly reduced by mab G6-31 treatment to 46% that of control IgG-treated tumors (p-value =0.009; Fig. 8I). In both treatment groups, tumor vascularity was less than in normal adjacent anterior pituitary. For quantitation of vascular density, MECA-32-stained sections were analyzed with an Ariol SL-50 slide scanning platform (Applied Imaging; San Jose, CA), using a 10x objective. Pituitary tumor regions were identified and outlined manually. Pixel colors corresponding to MECA-32 staining were defined, and the vascular area measured accordingly. Tumor cell nuclei were identified by pixel color and object shape. Vascular area was then normalized to pituitary tumor cell number. Pancreatic islets were analyzed similarly, except that MECA-32 staining area was normalized to islet tumor area.

In addition to the pituitary tumors, pancreatic islet tumors were frequently identified in the treated Men1^{+/-} mice and were histologically analyzed at study end-point. Islet tumors (defined as being larger than 10⁵ µm² in the plane of section) were typically solid, without significant hemorrhage or necrosis. Tumors from six animals treated with anti-VEGF-A Mab G6-31 (n=32 tumors) averaged only 39% the area of those from the five animals treated with control IgG (n=45 tumors; p-value = 0.026). Pancreatic adenomas (Figs. 8E-F) treated with control IgG appeared generally larger (up to 7.0 mm in plane of section) and more vascular than mAb G6-31 treated adenomas (tumor diameter up to 5.0 mm in plane of section). In four out of seven mice treated with control IgG, the pancreatic tumors contained dilated, blood filled, thin-walled, endothelial-lined spaces up to 300 µm in diameter, whereas the pancreatic adenomas in mAb G6-31 treated mice frequently lacked prominent vascularity (Figs. 8G-H). Pancreatic tumors from one out of eight mice treated with mAb G6-31 manifested dilated vessels. Hemosiderin-laden macrophages were intermittently found from pancreatic tumors with either treatment, suggestive of islet hemorrhage.

Vascular density in islet tumors was significantly reduced by G6-31 treatment to 56% that of control IgG-treated islet tumors (p-value = 2*10⁻⁷). Also, "normal"' islets (less then 10⁵ µm² in the plane of section) from Mab G6-31-treated mice had a vascular density reduced, by a smaller magnitude, to 76% that of control IgG-treated animals (p-value=4* 10⁻⁷; see Figure 8J). A single male animal, treated with control IgG, had a solitary 12 mm diameter adrenal cortical tumor, a recognized tumor type in the MEN1 syndrome glands.

Histology of the subcutaneous pituitary adenoma transplants was comparable to that of the pituitary tumors *in situ,* indicating a successful recapitulation of endocrine tumor growth at distant loci.

### Men1 Pituitary Adenomas are Prolactinomas

Approximately sixty percent of pituitary adenomas in MEN1 patients secrete prolactin (PRL), fewer than 25% growth hormone (GH), and 5% adrenocorticotropic hormone (ACTH, Trump et al., QJM 89:653-669 (1996)). To investigate whether the pituitary adenomas of Men1^{+/-} mice secrete PRL, immunohistochemical staining with anti-PRL antibodies was performed on control IgG and mAb G6-31 treated *in situ* pituitary adenomas, as well as pituitary tumor transplants. Six out of six control IgG and five out of five mAb G6-31 treated pituitary adenomas showed a specific, positive staining for prolactin in approximately 50-95% of the cells establishing them as prolactinomas (Figs. 9A-9B). In line with this result, Crabtree et al. reported that Men1^{TSM/+} pituitary tumors were positive for PRL in an immunohistochemical staining (Crabtree et al., Proc. Natl. Sci USA 98:1118-1123 (2001)). Prolactin staining was positive also in the pituitary adenoma transplants with either treatment (Figs. 9C-9D). Consistent with functional prolactin secretion from these tumors, mammary tissue in female mice bearing transplanted pituitary adenomas invariably showed moderate to marked lactational change in both control IgG and anti-VEGF-A-treated animals (Fig. 9E, F).

As assessed by immunohistochemical staining, 6 of 11 non-treated primary pituitary tumors were focally and weakly positive for growth hormone (Fig. 14A), whereas only one of four transplanted pituitary tumors showed focal weak staining (Fig. 14B). Growth hormone expression was present in both G6-31 and control IgG treated *in situ* pituitary tumors (Fig. 6C, D). Normal anterior pituitary showed strong reactivity in ∼20-30% of cells. (Fig. 14A).

### Serum Prolactin Level Correlates with Pituitary Tumor Volume in Untreated and Control IgG Treated Mice and is Decreased by mAb G6-31 Treatment

Given that all control IgG and all mAb G6-31 treated pituitary adenomas examined from Men1^{+/-} mice were positive for prolactin by immunohistochemical analysis, we investigated whether serum PRL levels were elevated in Men1^{+/-} pituitary adenoma-bearing mice. To this end, we initially analyzed 46 non-treated female Men1^{+/-} mice for their pituitary tumor status and serum PRL level, and five female wild-type littermate controls for serum PRL level. Serum prolactin amounts were analyzed by National Hormone & Peptide Program at Harbor UCLA (Torrance, CA).

The age range of these mice was 15.6 to 10.5 months, with an average age of 13.3 months. The mean serum PRL level in the wild-type mice was 43.8±25.3 (±SEM) ng/ml. Twenty-seven of the 46 Men1^{+/-} mice did not have a detectable pituitary tumor in MRI analysis. The mean serum PRL level in these mice was 69.0±24.6 ng/ml. A group of ten mice had a small pituitary tumor with a mean volume 1.7±0.6 mm³. Serum PRL level in these mice was elevated to a mean of 188.7±61.9 ng/ml. Nine mice that had a large pituitary tumor (mean volume 83.1±23.8 mm³), had a mean 13239.8±3466.5 ng/ml serum PRL. These data establish that there is a positive correlation between serum PRL levels and pituitary tumor volume in the Men1^{+/-} mice (Fig. 10A) with Pearson's correlation coefficient R=0.94, suggesting that serum PRL level could be useful as a diagnostic tool in establishing an estimate of pituitary tumor status.

To examine whether anti-VEGF-A treatment had an effect on serum PRL levels, we analyzed the serum of seven control IgG and seven mAb G6-31 treated Men1^{+/-} mice with a pituitary adenoma *in situ* at study end-point (day 67). In control IgG-treated mice, the mean serum PRL level was elevated to 12566.7±3047.4 ng/ml and was generally increased with an increasing tumor volume (mean of 116.2±18.5 mm³) with R=0.80 (p<0.03). In mAb G6-31-treated Men1^{+/-} mice analyzed, the serum PRL level remained lower, at 5163.7±1608.9 ng/ml, however, no statistical correlation to tumor volume was apparent (mean tumor volume 35.3±6.5 mm³), R=-0.12 with p<0.80 (Fig. 10B). Nonetheless, these data indicate that while mAb G6-31 inhibits the pituitary adenoma growth, it also leads to a decreased mean serum PRL, compared to control IgG treated mice with p<0.053.

### Anti-VEGF-A Treatment Lowers Serum PRL Levels in Mice with Subcutaneous Pituitary Tumor Transplants

While the above data indicate that anti-VEGF-A antibody treatment lowers the serum level of PRL in tumor-bearing Men1^{+/-} mice, we further investigated this in the context of the subcutaneous pituitary adenoma transplants in Balb/c Nude mice. Serum PRL was measured from samples originating from 23 control IgG and 35 mAb G6-31 treated mice, harvested at treatment onset (day 1), and at study end-point (day 35). While the mean serum PRL at day 1 was comparable between the two treatments, at day 35 mAb G6-31 treatment had significantly reduced the serum PRL levels (Fig. 10C and D, respectively).

As the current treatment of MEN 1 prolactinomas includes medical therapy or selective hypophysectomy followed by radiotherapy, our data indicating that mAb G6-31 treatment leads to lower PRL serum level with a prominent inhibition of tumor growth provides a potential new therapeutic approach to MEN 1 patients.

### Serum Insulin Levels are Elevated in Men1^{+/-} Mice

To examine whether the serum insulin levels were elevated in the Men1^{+/-} mice, serum samples were analyzed from six non-fasted mice treated with control IgG, and six non-fasted mice treated with mAb G6-31, which were all identified with pancreatic lesions in histologic analysis. Serum insulin levels were also analyzed from five non-fasted, age-matched wild type mice using an Ultrasensitive Mouse Insulin ELISA kit according to manufacturer's instructions (Mercodia, Uppsala, Sweden). No correlation with treatment was observed, however, the mean serum insulin was notably elevated in Men1^{+/-} mice (control IgG, 3.8±2.6 ng/ml; mAb G6-31, 3.7±2.4 ng/ml) compared to wild type mice (1.4±0.7 ng/ml (±SEM)).

### Discussion

Our data indicates that VEGF-A is required for the growth of benign pituitary gland adenomas in the mouse model of MEN 1, as therapy with a monoclonal antibody against VEGF-A was shown to be sufficient for tumor growth inhibition.

Based on the available literature, it is possible that much of the observed anti-tumor effects of mAb G6-31 are mediated by suppression of VEGFR-2-dependent angiogenesis (Wise et al., Proc. Natl. Acad. Sci. 96:3071-3076 (1999) and Zachary et al., Cardiovasc. Res. 49:568-581 (2001)). While the relative mean expression of VEGFR-2 mRNA in large pituitary adenomas was not significantly affected by anti-VEGF-A treatment (Fig. 6), immunohistochemical staining for MECA-32 showed highly significant decreases in vascularity (approximately 50% reduction) in both pituitary and pancreatic tumors treated with Mab G6-31. A corresponding reduction in anti-VEGF-A-treated tumor growth was noted in both pituitary and pancreatic adenomas. Vascular density in normal pancreatic islets was also significantly decreased by anti-VEGF-A treatment, though the magnitude of the change (25% reduction from control IgG treated) was less than that seen in pancreatic adenomas.

The observed high level of VEGF-A transcript in Mab G6-31 treated tumors is potentially a result of a compensatory mechanism to the systemic sequestering of VEGF-A by Mab G6-31. However, it appeared that such elevated VEGF-A was not sufficient to drive the tumor growth.

In addition to showing that a monotherapy with an anti-VEGF-A mAb G6-31 treatment significantly lowered the tumor burden of the Men1^{+/-} mice by effectively inhibiting adenoma growth, we showed that the serum prolactin level was also lowered. Thus, our data suggest the possibility of a non-surgical treatment for benign tumors of the endocrine, with a cessation of disease progression without the use of chemotherapeutic agents. Alternatively, such VEGF-A blockade may be combined with a pharmacological agent. For example, for prolactin-secreting adenomas, a VEGF-A antagonist may be combined with a dopamine agonist.

### Example 3. Anti-VEGF Intervention Efficacy and Regression/Survival Efficacy in the RIP-TßA_{g} Model of Multi-Stage Carcinogenesis

In order to better understand the role of anti-VEGF therapies in various stages of tumor growth, we turned to a number of preclinical tumor models, including the RIP-TβAg. RIP-TβAg (Exelixis, Inc.) is a conditional version of a mouse pancreatic islet tumor model driven by transgenic expression of the SV40 Large T antigen (TAg) (targeted to the pancreatic β-cell, where TAg functions as a potent oncogene by binding both p53 and Rb). The RIP-TβAg is phenotypically similar to the RIP-TAg model that has been previously described (Hanahan, Nature 315:115-122 (1985); Bergers et al., Science 284 (808-811), 1999). We have found that this model progresses through a series of increasingly aggressive stages, including the activation of VEGF signaling and an "angiogenic switch" (i.e., initiation of the process of forming new blood vessels) at approximately 5 weeks. Small tumors form by 10 weeks, coinciding with the start of its malignant conversion. Large, invasive carcinomas form by 12 weeks. Thus, prior to 10 weeks of age, cell growths in the mice are not considered malignant or metastatic. The period between 10-12 weeks of age generally includes the formation of an invasive cancer.

For these experiments, RIP-TβAg mice were housed and treated according to standard IACUC recommendations, the mice were provided with high-sugar chow and 5% sucrose water to alleviate symptoms of hyperinsulinemia caused by the increase in insulin-secreting beta-cells in the pancreas. At 9-9.5 or 11-12 weeks of age, the mice were treated twice-weekly with an intra-peritoneal injection of 5 5 mg/kg anti-VEGF antibody or isotype-matched anti-ragweed control monoclonal antibody in sterile phosphate-buffered saline. In the "intervention trial," the 9-9.5 week aged mice were treated for 14 days and then examined. In the "regression trial," the 11-12 week aged mice were examined after 7, 14, and 21 days of treatment. To examine survival, another cohort of mice was treated until the mice exhibited morbidity or mortality. At each defined time point in the intervention and regression trials, the pancreas and spleen of each mouse was removed and photographed. Tumor number within the pancreas was determined by dissecting out each spherical tumor and counting. Tumor burden was determined and measuring the two largest diameters of each tumor and calculating the volume using the spheroid volume calculation (x2 multiply by y) multiply by 0.52. The volume of all tumors within the pancreas of a mouse was summed to determine the total tumor burden. Means and standard deviations were calculated, and data was graphed using Microsoft Excel v11.3.3 (Microsoft, Inc.). Statistical comparisons between tumor number and burden from different groups were carried out using a Student's t-test. Kaplan-Meier curves for survival analysis were generated using JMP 6.0 (SAS Institute, Inc.) and statistical comparisons carried out using a log-rank analysis.

The treatment of the 9-week-old animals (the "intervention" trial) with anti-VEGF antibodies resulted in a dramatic reduction in tumor angiogenesis (FIGURE 11). The treatment of the 11-week-old animals (the "regression trial") resulted in a decrease in tumor vascularity and proliferation (FIGURE 12A). However, in contrast to the intervention trial, only a transient reduction in tumor growth was detected and there was no impact on survival (FIGURE 12B). These studies demonstrated that these early tumors are more sensitive to anti-VEGF targeted therapeutics as compared to advanced tumors.

### Example 4. Anti-VEGF and Chemotherapy are Effective in Pre-clinical Models

Surgery can leave behind residual tumor cells, or dormant micro-metastatic nodules, which have the potential to re-activate the "angiogenic program" and facilitate more exponential tumor growth.

We have used the mouse genetic tumors and human xenografts to model tumor dormancy, by using potent chemotherapy regimens to "cytoreduce" the tumor concurrently or sequentially with anti-VEGF therapies, followed by maintenance therapy with anti-VEGF monoclonal antibodies. Thus, we are treating the mouse to prevent the recurrence of a tumor, rather than chasing a tumor after it is formed or reformed, including, in some cases, into a tumor that is refractory, relapsed or resistant to more treatments including targeted anti-VEGF therapies. Figure 13 shows the observation that docetaxel is quite effective at reducing tumor burden, yet the dormant cells re-grow after approximately 2 months. However concurrent treatment with anti-VEGF suppresses tumor re-growth, even though it has little impact on its own on the growth of larger, more established tumors. Additional data demonstrates that prolonged anti-VEGF therapy also suppresses re-growth of tumors following cytoreduction with taxanes or gemcitabine. These results demonstrate that anti-VEGF can be used to effectively block growth or re-growth of dormant tumors or micrometastases. These findings are consistent with the fact that neovascularization is a prerequisite to the rapid clonal expansion associated with the formation of macroscopic tumors and support the use of VEGF-specific antagonists (e.g., anti-VEGF antibodies including but not limited to the G6 or B20 series antibodies) in the maintenance of tumor dormancy and the suppression of tumor re-growth after initial treatment of the tumor and before the formation of new tumors, reactivation of dormant tumors, malignant tumors or micrometastases.

### Example 5. Neoadjuvant therapy using bevacizumab

This example illustrates the use of bevacizumab in a neoadjuvant therapy of patients with palpable and operable breast cancer.

Neoadjuvant chemotherapy has been widely used in the treatment of locally advanced or potentially operable large breast cancers. Randomized trials have demonstrated that neoadjuvant chemotherapy reduces the need for mastectomy (thereby preserving the breast), with similar overall survival rates to adjuvant chemotherapy. Powles et al., J. Clin. Oncol. 13:547-52 (1995); Fisher et al., J. Clin. Oncol. 16:2672-85 (1998).

The primary goals of the present therapy are to provide improved clinical benefits by adding bevacizumab to chemotherapies in patients with palpable and operable breast cancer. Specifically, one of the primary measurements of clinical efficacy of neoadjuvant therapy can be the pathologic complete response (pCR) rates. Other measurements include overall response rates (OR), clinical complete response rates (cCR), disease-free survival (DFS) and overall survival (OS). Furthermore, side effects of the treatment can be monitored by, for example, surgical complication rates, toxicity, and adverse effects on cardiac function. Certain gene expression or other biomarker activities can also be used as markers of response to treatment.

Pathological complete response (pCR) has been used as a prognostic marker of treatment efficacy. pCR refers to a lack of residual histological evidence of tumor after neoadjuvant therapy at the time of surgery. Studies have suggested that patients achieving pCR have a significantly improved survival. However, there is no standard method for grading pathological response, and whether pCR can be used as a surrogate marker of efficacy remains controversial.

Patients suitable for the neoadjuvant therapy with VEGF-specific antagonists are selected based on predetermined criteria and guidelines, which vary depending on the particular cancer type under the treatment. For example, patients can be selected based on their life expectancy, age, histology of the cancer, hematologic/hepatic and cardiac functions, treatment history, reproductive status and plans, and psychiatric or addictive states. More importantly, the primary tumor should be measurable and or operable.

The treatment regimens include chemotherapy plus VEGF-specific antagonist, e.g., bevacizumab. Optionally, additional therapeutic agent(s) can be used in the regimen as well. Typically, the chemotherapy regimen commonly used for the particular cancer type (e.g., standard therapy regimen) is used in combination with bevacizumab. For example, for neoadjuvantly treating breast cancer, docetaxel, paclitaxel or doxorubicin/cyclophosphamide (AC) regimen can be used in combination with bevacizumab. Alternatively, docetaxel-based or paclitaxel-based regimen and AC regimen can be used sequentially as the chemotherapy combined with bevacizumab. For treating non-small cell lung cancer, cisplatin (either alone or in combination with other chemo agents such as gemcitabine, docetaxel, or vinorelbine) can be used as the primary chemo agent in combination with bevacizumab. For treating colorectal cancer, on the other hand, 5-FU-based regimens (such as FOLFOX) can be used in combination with bevacizumab.

The chemotherapeutic agents and VEGF-specific antagonist, e.g., bevacizumab, are administered to patients at given dosages and intervals, for a number of cycles. For example, bevacizumab can be given at 15 5 mg/kg every 3 weeks for 4 cycles, or at 10 mg/kg every two weeks for 6 cycles. In another example, the VEGF-specific antagonist, e.g., bevacizumab, is administered at 7.5 5 mg/kg once every two or three weeks. Patients are monitored for response during the treatment. Upon the completion of neoadjuvant therapy, patients undergo surgery to remove the primary tumor, which was either operable prior to the neoadjuvant therapy, or becomes operable in response to the neoadjuvant therapy. After the surgery, patients are continued on VEGF-specific antagonist therapy, e.g., bevacizumab, with or without chemotherapy, depending on the patient's particular status. Optionally, patients can undergo radiation therapy as well. Patient's progress is monitored throughout the treatment and post-treatment.

Adverse events (AEs) associated with anti-VEGF treatment should also be closely monitored and managed. Main AEs known from previous studies include hypertension, proteinuria, hemorrhage, thromboembolic events, gastrointestinal perforation/fistula and wound healing complications. Certain AEs such as hypertension can be managed with medicine. If AEs are severe and unmanageable, the treatment should be discontinued.

### Example 6. Adjuvant therapy using bevacizumab

This example illustrates the use of bevacizumab combined with chemotherapy in adjuvant treatment of patients with resected cancer.

Patients suitable for the adjuvant therapy with bevacizumab are selected based on predetermined criteria and guidelines, which vary depending on the particular cancer type under the treatment. For example, patients can be selected based on their life expectancy, age, histology of the cancer, hematologic/hepatic and cardiac functions, history of life style, treatment history, reproductive status and plans, and psychiatric or addictive states. More importantly, patients must have undergone complete resection of their cancer prior to the adjuvant treatment. Accepted types of resection depend on the particular tumor types. Also, sufficient pathology material representative of patient's cancer should be available for analysis of the initial stage and for efficacy determination. At the time of the treatment, the surgery should be fairly recent, and yet the patient must be fully recovered from the surgery. For example, patients must begin adjuvant treatment no less than 3-6 weeks and no more than 8-12 weeks after surgery.

The treatment regimens include chemotherapy plus bevacizumab. Optionally, additional therapeutic agent(s) can be used in the regimen as well. Typically, the chemotherapeutic agents in combination with bevacizumab are used during the first stage of treatment, followed by bevacizumab as single agent maintenance treatment for the remaining phase. For example, patients are treated with chemotherapeutic agents and bevacizumab for about 4-12 cycles, then with bevacizumab alone for up to 1 to 2 years. Duration of each cycle of chemotherapeutic + bevacizumab treatment depends on the specific agents and dosages used. For example, bevacizumab can be given at 15mg/kg every 3 weeks as one cycle, or at 5mg/kg every two weeks as one cycle. In another example, bevacizumab can be given at 7.5 5 mg/kg or 10mg/kg every two or every three weeks.

The chemotherapy regimen commonly used for the particular cancer type (e.g., standard therapy regimen) is used in combination with bevacizumab. For example, for adjuvant therapy of breast cancer, docetaxel, paclitaxel or doxorubicin/cyclophosphamide (AC) regimen can be used in combination with bevacizumab. Alternatively, docetaxel-based or paclitaxel-based regimen and AC regimen can be used sequentially as the chemotherapy combined with bevacizumab. For treating non-small cell lung cancer, cisplatin (either alone or in combination with other chemo agents such as gemcitabine, docetaxel, or vinorelbine) can be used as the primary chemo agent in combination with bevacizumab. For treating colorectal cancer, on the other hand, 5-FU-based regimens (such as FOLFOX) can be used in combination with bevacizumab.

The goal of adjuvant treatment with bevacizumab is to improve patient's survival, preferably disease free survival. Meanwhile, any adverse events associated with bevacizumab should be closely monitored, especially because the adjuvant treatment is long term. Survival can be estimated by the Kaplan-Meier method, and any differences in survival are computed using the stratified log-rank test. Mutlivariable analyses using the Cox proportional hazard model are used to estimate the simultaneous effects of prognostic factors on survival. The interactions with prognostic factors are examined with the Cox proportional hazard model. The SAS statistical software package is used for all calculations. The data is considered to be statistically significant when the P value is 0.05 or less. All statistical tests are two-sided.

Adverse events (AEs) associated with anti-VEGF treatment should be closely monitored and managed. Main AEs known from previous studies include hypertension, proteinuria, hemorrhage, thromboembolic events, gastrointestinal perforation/fistula and wound healing complications. Certain AEs such as hypertension can be managed with medicine. If AEs are severe and unmanageable, the treatment should be discontinued.

### Other Embodiments

From the foregoing description, it will be apparent that variations and modifications may be made to the invention described herein to adopt it to various usages and conditions. Such embodiments are also within the scope of the following claims.

All publications, patent applications, and patents mentioned in this specification including U.S. provisional application numbers 60/870,741, filed December 19, 2006; 60/870,745, filed December 19, 2006; 60/877,267, filed December 27, 2006; 60/919,638, filed March 22, 2007; 60/958,384, filed July 5, 2007; and 60/989,397, filed November 20, 2007, are herein incorporated by reference to the same extent as if each independent publication, patent, or patent application was specifically and individually indicated to be incorporated by reference.

## Claims

1. A method of treating a benign, pre-cancerous, or non-metastatic cancer in a subject, comprising administering to said subject an effective amount of a VEGF-specific antagonist.

2. The method of claim 1, wherein said administering of the VEGF-specific antagonist prevents said benign, pre-cancerous, or non-metastatic cancer from becoming an invasive or metastatic cancer.

3. The method of claim 1, wherein said benign, pre-cancerous, or non-metastatic cancer is a stage 0, stage I, or stage II cancer.

4. The method of claim 3, wherein said administering of the VEGF-specific antagonist prevents said benign, pre-cancerous or non-metastatic cancer from progressing to a stage III or stage IV cancer.

5. The method of claim 1, wherein said administering of the VEGF-specific antagonist reduces tumor size.

6. A method of treating a subject with a family history of cancer, polyps, or an inherited cancer syndrome, comprising administering to said subject an effective amount of a VEGF-specific antagonist to prevent occurrence or recurrence of a benign, pre-cancerous, or non-metastatic cancer in said subject.

7. The method of claim 6, wherein said method prevents occurrence or recurrence of said benign, pre-cancerous or non-metastatic cancer in a subject who has never had clinically detectable cancer or a subject who has only had a benign cancer.

8. A method of reducing tumor size in a subject having an unresectable tumor, comprising administering to said subject an effective amount of a VEGF-specific antagonist, wherein said administering of the VEGF-specific antagonist reduces the tumor size thereby allowing complete resection of the tumor.

9. The method of claim 8, further comprising the step of administering to said subject an effective amount of a VEGF-specific antagonist after complete resection of the tumor.

10. A method of treating a subject with operable cancer, comprising administering to said subject an effective amount of a VEGF-specific antagonist prior to surgery and performing surgery whereby the cancer is resected.

11. The method of claim 10, further comprising the step of administering to said subject an effective amount of a VEGF-specific antagonist after surgery to prevent recurrence of the cancer.

12. The method of claim 9 or 11, wherein said administering of the VEGF-specific antagonist prevents proliferation of micrometastases.

13. A method of neoadjuvant therapy in a subject with operable cancer, comprising administering to said subject a VEGF-specific antagonist.

14. A method of preventing recurrence of cancer in a subject, comprising administering to said subject a VEGF-specific antagonist, wherein said administering prevents cancer recurrence in said subject.

15. A method of reducing the likelihood of cancer recurrence in a subject, comprising administering to said subject a VEGF-specific antagonist, wherein said administering reduces the likelihood of cancer recurrence in said subject.

16. The method of claim 14 or 15, wherein said administering of the VEGF-specific antagonist prevents or reduces the likelihood of occurrence of a clinically detectable tumor, or metastasis thereof.

17. The method of any one of claims 14 or 15, wherein the subject has had definitive surgery prior to said step of administering a VEGF-specific antagonist.

18. The method of any one of claims 1, 6, 8, 10, 13, 14, 15, or 17, wherein the VEGF-specific antagonist is a monotherapy.

19. The method of any one of claims 1, 6, 8, 10, 13, 14, 15, 17, or 18, wherein the subject has been previously treated with an anti-cancer therapy.

20. The method of claim 19, wherein said anti-cancer therapy comprises anti-angiogenic therapy.

21. A method of preventing the regrowth of a tumor in a subject comprising the steps of removing the tumor and thereafter administering to the subject a VEGF-specific antagonist.

22. A method of preventing the recurrence of cancer in a subject having a tumor comprising the steps of removing the tumor and thereafter administering to the subject a VEGF-specific antagonist.

23. The method of claim 21 or 22, further comprising a period of time between removal of the tumor and administering the VEGF-specific antagonist wherein the period of time is greater than 2 weeks.

24. The method of claim 23, wherein the period of time is greater than two weeks and less than 1 year.

25. The method of claim 21 or 22, further comprising a period of time between removal of the tumor and administering the VEGF-specific antagonist wherein the period of time is 28 days.

26. The method of claim 21 or 22, further comprising a period of time between removal of the tumor and administering the VEGF-specific antagonist wherein the period of time is sufficient for the surgical incision to be fully healed or to reduce the risk of wound dehiscence.

27. The method of claim 21 or 22, wherein the VEGF-specific antagonist is a monotherapy.

28. The method of any one of claims 1, 6, 8, 10, 13, 14, 15, 18, or 22, further comprising monitoring the subject for recurrence of said cancer.

29. The method of any one of claims 1, 6, 8, 10, 13, 14, 15, 18, 21, or 22, wherein the cancer or tumor is gastrointestinal, colorectal, breast, ovarian, lung or renal.

30. The method of any one of claims 1, 6, 8, 10, 13, 14, 15, 18, 21, or 22, further comprising administering an additional anti-cancer therapy.

31. The method of claim 30, wherein said additional anti-cancer therapy is chemotherapy.

32. The method of any one of claims 1, 6, 8, 10, 13, 14, 15, 18, 21, or 22 wherein said VEGF-specific antagonist is selected from the group consisting of a polypeptide that specifically binds to VEGF, a ribozyme, a peptibody, an antisense nucleobase oligomer, a small RNA molecule and an aptamer.

33. The method of claim 32, wherein said polypeptide that specifically binds to VEGF is a soluble VEGF receptor protein, or VEGF-binding fragment thereof, or a chimeric VEGF receptor protein.

34. The method of claim 33, wherein said chimeric VEGF receptor protein is Flt-1/Fc, KDR/Fc or Flt/KDR/Fc.

35. The method of claim 32, wherein said polypeptide that specifically binds to VEGF is an anti-VEGF antibody or antigen-binding fragment thereof.

36. The method of claim 35, wherein said anti-VEGF antibody is a monoclonal antibody.

37. The method of claim 36, wherein said monoclonal antibody is chimeric, humanized, or fully human antibody.

38. The method of claim 37, wherein said monoclonal antibody is bevacizumab.


SEQUENCE LISTING

<110>  Genentech, Inc. et al.

<120>  VEGF-Specific Antagonists For Adjuvant and Neoadjuvant Therapy
       and the Treatment of Early Stage Tumors

<130>  CMD/FP7161193

<150>  EP 07869456.9
<151>  2007-12-18

<150>  PCT/US2007/088000
<151>  2007-12-18

<150>  US 60/989,397
<151>  2007-11-20

<150>  US 60/958,384
<151>  2007-07-05

<150>  US 60/919,638
<151>  2007-03-22

<150>  US 60/877,267
<151>  2006-12-27

<150>  US 60/870,745
<151>  2006-12-19

<150>  US 60/870,741
<151>  2006-12-19

<160>  5

<170>  PatentIn version 3.3

<210>  1
<211>  24
<212>  DNA
<213>  Artificial Sequence

<220>
<223>  synthetic

<400>  1
caacgtcact atgcagatca tgcg                                              24


<210>  2
<211>  23
<212>  DNA
<213>  Artificial Sequence

<220>
<223>  synthetic

<400>  2
ggtctagttc ccgaaaccct gag                                               23


<210>  3
<211>  25

<212>  DNA
<213>  Artificial Sequence

<220>
<223>  synthetic

<400>  3
atgttccagt atgactccac tcacg                                              25


<210>  4
<211>  25
<212>  DNA
<213>  Artificial Sequence

<220>
<223>  synthetic

<400>  4
gaagacacca gtagactcca cgaca                                              25


<210>  5
<211>  29
<212>  DNA
<213>  Artificial Sequence

<220>
<223>  synthetic

<400>  5
aagcccatca ccatcttcca ggagcgaga                                          29